(19) Europäisches Patentamt
European Patent Office
Office européen des brevets

(11) EP 1 792 613 A2

(12) EUROPEAN PATENT APPLICATION

(43) Date of publication:
**06.06.2007 Bulletin 2007/23**

(21) Application number: **06118366.1**

(22) Date of filing: **03.08.2006**

(51) Int Cl.:
***A61K 31/33*** *(2006.01)* ***A61K 31/341*** *(2006.01)*
***A61K 31/381*** *(2006.01)* ***A61K 31/4164*** *(2006.01)*
***A61K 31/4184*** *(2006.01)* ***A61K 31/443*** *(2006.01)*
***A61P 33/02*** *(2006.01)* ***A61P 35/00*** *(2006.01)*
***C07D 307/68*** *(2006.01)* ***C07D 329/00*** *(2006.01)*
***C07D 405/14*** *(2006.01)* ***C07D 421/14*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **02.12.2005 US 741689 P**

(71) Applicants:
- **UNIVERSITY OF NORTH CAROLINA AT CHAPEL HILL**
  **Chapel Hill, NC 27599-4105 (US)**
- **GEORGIA STATE UNIVERSITY RESEARCH FOUNDATION, INC.**
  **Atlanta, GA 30303 (US)**

(72) Inventors:
- **Tidwell, Richard R.**
  **Pittsboro, GA 27312 (US)**
- **Boykin, David W.**
  **Atlanta, GA 30306 (US)**
- **Ismail, Mohamed A.**
  **Atlanta, GA 30303 (US)**
- **Wilson, W. David**
  **Atlanta, GA 30307 (US)**
- **White, Elizabeth W.**
  **Atlanta, GA 30345 (US)**
- **Kumar, Arvind**
  **Lilburn, GA 30047 (US)**
- **Nanjunda, Rupesh**
  **Atlanta, GA 30039 (US)**

(74) Representative: **Behnisch, Werner**
**Reinhard-Skuhra-Weise & Partner GbR**
**Friedrichstrasse 31**
**80801 München (DE)**

(54) **Dicationic compounds which selectively recognize G-quadruplex DNA**

(57) Dicationic compounds that are highly selective for binding G-quadruplex DNA are described. Several compounds exhibit groove binding toward G-quadruplex DNA and *in vitro* and *in vivo* activity versus *Trypanosoma brucei rhodesiense*. The compounds represent novel drugs for the treatment of cancer, malaria, leishmania, and trypanosomiasis.



Printed by Jouve, 75001 PARIS (FR)

## Description

TECHNICAL FIELD

The presently disclosed subject matter relates to methods and compounds for binding G-quadruplex DNA. More particularly, the presently disclosed subject matter relates to dicationic polyaryl compounds that bind to quadruplex forms of telomeres and oncogene promoters, methods of disrupting telomerase activity, and the use of dicationic polyaryl compounds to treat cancer.

ABBREVIATIONS

| | |
|---|---|
| δ | = chemical shift |
| A | = adenine |
| Ac | = acetyl |
| $Ac_2O$ | = acetic anhydride |
| C | = cytosine |
| ˚C | = degrees Celsius |
| calcd | = calculated |
| CD | = circular dichroism |
| $C_f$ | = unbound concentration |
| cm | = centimeters |
| dec | = decomposition point |
| DMF | = dimethylformamide |
| DMSO | = dimethylsulfoxide |
| DNA | = deoxyribonucleic acid |
| $D_2O$ | = deuterium oxide |
| DODC | = 3,3'-diethyloxadicarbocyanine |
| DSS | = 3-(trimethyl silyl) propane sulfonic acid sodium salt |
| DTC | = *N,N'*-diethylthiacarbocyanine iodide |
| EDTA | = ethylenediaminetetraacetic acid |
| EIMS | = electrospray-ionization mass spectrometry |
| EtOAc | = ethyl acetate |
| EtOH | = ethanol |
| g | = grams |
| G | = guanine |
| h | = hours |
| HCl | = hydrogen chloride |
| HEPES | = N-(2-Hydroxyethyl)piperazine-N'-(2-ethanesulfonic acid) |
| HPLC | = high-pressure liquid chromatography |
| hTERT | = human Telomerase Reverse Transcriptase unit |
| hTR | = human Telomerase RNA unit |
| Hz | = hertz |
| ip | = intraperitoneal |
| KCl | = potassium chloride |
| kg | = kilograms |
| KO-*t*-Bu | = potassium *tert*-butoxide |
| *L. d.* | = *Leishmania donovani* |
| LiOH | = lithium hydroxide |
| MeOH | = methanol |
| MHz | = megahertz |
| min. | = minutes |

(continued)

| | |
|---|---|
| mL | = milliliters |
| mm | = millimeters |
| mM | = millimolar |
| mmol | = millimole |
| m.p. | = melting point |
| ms | = millisecond |
| MS | = mass spectroscopy |
| MTT | = 3-(4,5-dimethylthiazol-2-yl)-2,5-diphenyltetrazolium bromide |
| $Na_2CO_3$ | = sodium carbonate |
| $Na_2SO_4$ | = sodium sulfate |
| NBS | = *N*-bromosuccinimide |
| $NH_2OH$•HCl | = hydroxylamine hydrochloride |
| nM | = nanomolar |
| NMR | = nuclear magnetic resonance |
| NOESY | = nuclear overhauser effect spectroscopy |
| Pd | = palladium |
| Pd-C | = 10% palladium on carbon |
| $Pd(PPh_3)_4$ | = tetrakis(triphenylphosphine)palladium |
| *P. f.* | = *Plasmodium falciparum* |
| PIPER | = *N,N'*-bis(2-piperdinoethyl)-3,4,9,10-perylenetetracarboxylic acid diimide |
| po | = oral |
| ppm | = parts per million |
| psi | = pounds per square inch |
| RU | = response units |
| s | = seconds |
| spp. | = species |
| SPR | = surface plasmon resonance |
| T | = thymine |
| *T. b. r.* | = *Trypanosoma brucei rhodesiense* |
| *T. cruzi* | = *Trypanosoma cruzi* |
| THF | = tetrahydrofuran |
| TLC | = thin-layer chromatography |
| TMS | = tetramethylsilane |
| TOCSY | = total correlation spectroscopy |
| U | = uracil |
| UV | = ultraviolet |

BACKGROUND

G-quadruplex deoxyribonucleic acid (DNA) is a four-stranded DNA structure formed by single-stranded DNA containing runs of consecutive guanine residues. See Cech, T.R., Nature, 332, 777-778 (1988). Guanine-rich sequences believed to be capable of forming quadruplex structures are present in biologically significant regions of the genome, including immunoglobulin switch regions (Sen, D., and Gilbert, W., Nature, 334, 364-366 (1988)); the transcriptional regulatory regions of a number of genes, such as the insulin gene (Castasi, P., et al., J. Mol. Biol., 264, 534-545 (1996) ); and also the promoter regions of certain oncogenes, including c-MYC (Siddiqui-Jain, A., et al., Proc. Natl. Acad. Sci. USA, 99, 11593-11598 (2002); Simonsson, T. et al., Nuc. Acids Res., 26, 1167-1172 (1998).

Telomeric DNA sequences also have been shown to form quadruplex structures *in vitro*. See Wang, Y., and Patel, D. J., Structure, 2, 1141-1156 (1994); Wang, Y., and Patel, D. J., J. Mol. Biol., 251, 76-94 (1995); and Smith, F. W., et al., Structure, 3, 997-1008 (1995). Telomeres are regions of non-coding DNA located at the ends of eukaryotic chromosomes in organisms as diverse as trypanosomes and humans. Their function is to protect the ends of the chromosomes from erosion and end-end fusion and to aid in chromosomal alignment during recombination.

Human telomeres have a 5-15 kilobase double-stranded region with one purine-rich strand and one pyrimidine-rich strand. At the extreme 3' end, the purine-rich strand exists as a single-stranded overhang about 200 bases long. The telomeric sequence varies depending on the organism. In humans and other vertebrates, telomeres have tandem $T_2AG_3$ repeats.

The human telomere sequence has been shown to adopt a G-quadruplex conformation *in vitro* under physiological conditions. See Parkinson, G. N., et al., Nature, 417, 876-880 (2002) and Wang, Y., and Patel, D., Structure, 1, 263-282 (1993). The discovery of proteins such as transcription factors, nucleases, and helicases that can bind to and even promote the formation of telomeric quadruplexes suggests that these structures exist *in vivo* under certain conditions. See Giraldo, T., et al., Proc. Natl. Acad. Sci. USA, 91, 7658-7662 (1994); Fang, G., and Cech, T. R., Biochemistry, 32, 11646-11657 (1993); and Fang, G., and Cech, T, R., Cell, 74, 875-885 (1993). A very recent finding by Boussin and coworkers is that a radiolabeled G-quadruplex binding ligand accumulated in nuclei of cultured cells and preferentially bound to the terminal regions of the chromosomes, suggesting that G-quadruplexes exist *in vivo* and are accessible to drugs. See Granotier, C., et al., Nuc. Acids. Res., 33, 4182-4190 (2005).

Each time a cell divides, DNA polymerase is unable to replicate the extreme end of the 3' strand of the chromosome, since the 3' strand is the lagging strand during replication. This "end replication problem" results in a shortening of the telomere by about 30 - 200 bases per cell doubling. After 60-70 rounds of cell replication, the telomeres reach a critical length, and the cells enter a non-dividing state called senescence, which leads to apoptosis and eventually cell death. See Harley, C. B., et al., Nature, 345, 458-460 (1990). In 85-90 % of cancer cells, however, the reverse transcription enzyme telomerase is activated. See Kim, N. W., et al., Science, 266, 2011-2015 (1994). The enzyme also is active in eukaryotic parasites such as trypanosomes and leishmania. This enzyme is inactive in most normal somatic cells, providing a potentially very specific target for the treatment of cancer and parasitic disease. In humans, telomerase adds $T_2AG_3$ repeats to the telomere ends, which balances telomere shortening during cell division. The result is that telomere length is maintained, contributing to immortality of the cancer cells.

Telomerase comprises an 11-base RNA template and a human Telomerase Reverse Transcriptase (hTERT) catalytic subunit with reverse transcriptase activity. Telomerase inhibition has received recent interest as an anti-cancer strategy. Targets for telomerase inhibition include the hTERT catalytic subunit, targeted by reverse transcriptase inhibitors and dominant negative hTERT constructs. See Zhang, X., et al., Gene Dev., 13, 2388-2399 (1999); Hahn, W. C., et al., Nat. Med., 5, 1164-1170 (1999); Strahl, C., and Blackburn, E. H., Mol. Cell. Biol., 16, 53-56 (1996); Melana, S. M., et al., Clin. Cancer Res., 4, 693-696 (1997); Murakami, J., et al., Eur. J. Cancer, 35, 1027-1034 (1998); and Gomez, D. E., et al., Biochem. Biophys. Res. Commun., 246, 107-110 (1998). The RNA template also has been targeted using antisense oligonucleotides (Schindler, A., et al., Int J. Oncol., 19, 25-30 (2001) and Fu, W. M., et al., J. Mol. Neurosci., 14, 3-15 (2000)), as well as hammerhead ribozymes (Yokoyama, Y., et al., Biochem. Biophys. Res. Commun., 273, 316-321 (2000) and Ludwig, A., et al., Cancer Res., 61, 3053-3061 (2001)).

Another method of interfering with telomerase activity is to interact with the telomere, rather than with the actual telomerase enzyme. Telomerase requires the telomere primer to be single stranded. The formation of higher ordered structures, such as G-quadruplexes, prevents hybridization of the telomerase RNA template onto the telomere primer and thus inhibits telomerase activity. See Zahler, A. M., et al., Nature, 350, 718-720 (1991). Stabilization of the quadruplex conformation of telomeres, such as by binding with a small molecule, has been shown to inhibit telomerase activity. The development of small molecules that can selectively bind to and stabilize the G-quadruplex conformation of the telomere is therefore a current area of interest in anti-cancer drug design.

In eukaryotic parasites, the telomere and telomerase are essential for all of the functions described above and also for additional gene control mechanisms. For example, the enzyme telomerase is found in disease-causing unicellular protozoan parasites, such as *Plasmodium* spp., *Trypanosoma* spp., and *Leishmania* spp. See Bottius, E. N., et al., Mol. Cell. Biol. 18, 919-925 (1998) and Cano, M. I. N., et al., Proc. Natl. Acad. Sci. USA, 96, 3616-3121 (1999). The telomeres of these organisms have the same sequence of tandem $T_2AG_3$ repeats as in human telomeres. These protozoa undergo very rapid cell division, but because telomere shortening is compensated for by telomerase, these cells, much like cancer cells, can undergo an unlimited number of cell divisions. The protozoan pathogens cause diseases, such as malaria and African sleeping sickness, and are responsible for millions of deaths each year. The few available antiprotozoan therapies suffer from problems such as drug resistance and severe toxicity to the host. Telomerase inhibition through stabilization of the quadruplex conformation of the telomere offers an attractive, selective target for the design of agents which can impair the proliferation of these protozoa with decreased cytotoxicity and drug resistance.

The majority of compounds that have been shown to bind to quadruplex DNA are planar, aromatic compounds that bind via external end-stacking to the G-quartet on either one end or both ends of the quadruplex. These compounds, which include anthraquinones, cationic porphyrins, acridines, perylenes, macrocycles and related molecules, have large aromatic planar chromophores that mimic the planar surface of the G-quartets. See Han, F. X., et al., J. Am. Chem. Soc., 121, 3561-3570 (1999); Teulade-Fichou, M-P., et al., J. Am. Chem. Soc., 125, 4732-4740 (2003); Fedoroff O. Y., et al., Biochemistry, 37, 12367-12374 (1998); Read, M., et al., Proc. Natl. Acad. Sci. USA, 98, 4844-4849 (2001); and Clark, G. R., et al., J. Am. Chem. Soc., 125, 4066-4067 (2003). These molecules bind non-covalently through electrostatic, van der Waals, and $\pi$-orbital overlap interactions, and generally also interact with the loop bases. Since essentially all

known quadruplex DNA binders are based on or derived from prototype DNA duplex intercalators, many quadruplex DNA binders exhibit little selectivity for quadruplex over duplex structures. These types of molecules exhibit non-selective binding to many sites, leading to the cytotoxic side effects generally associated with chemotherapy, such as nausea, vomiting, hair loss, and heart and kidney damage. Thus, there is a need in the art for new classes of compounds that can bind to quadruplex DNA via novel binding modes, with high affinity and with selectivity over duplex DNA, thereby providing enhanced telomerase inhibition and improved therapeutics for treating cancer and microbial (*e.g.*, protozoal) infections with fewer side effects.

SUMMARY

In some embodiments the presently disclosed subject matter provides a method of binding quadruplex DNA, the method comprising contacting the quadruplex DNA with a compound of Formula (I):

$$Am_1\text{-}Ar_1\text{-}Ar_2\text{-}Ar_3\text{-}(Ar_4)_m\text{-}Am_2 \qquad (I)$$

wherein:

m is an integer from 0 to 1;
$Ar_1$, $Ar_2$, $Ar_3$, and $Ar_4$ are independently selected from the group consisting of:

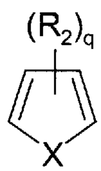 , 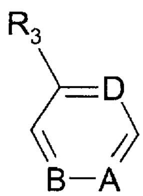 , 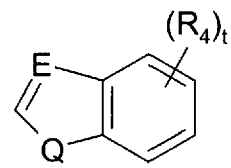 ,

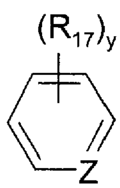 , and 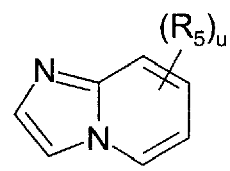 ,

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;
each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;
each E is independently selected from the group consisting of $CR_{18}$ and N; wherein $R_{18}$ is selected from the group consisting of H, halo, hydroxyl, alkyl, alkoxyl, substituted alkyl, cycloalkyl, aryl, aryloxyl, and substituted aryl;
each Z is independently selected from the group consisting of $CR_{19}$ and N; wherein $R_{19}$ is selected from the group consisting of H, halo, hydroxyl, alkyl, alkoxyl, substituted alkyl, cycloalkyl, aryl, aryloxyl, and substituted aryl;
each q is independently an integer from 0 to 2;
each y is independently an integer from 0 to 3;
each t is independently an integer from 0 to 3;
each u is independently an integer from 0 to 3;
each $R_2$, $R_3$, $R_4$, $R_5$ and $R_{17}$ is independently selected from the group consisting of H, alkyl, substituted alkyl, aryl, substituted aryl, halo, hydroxyl, alkoxyl, aryloxyl, and aralkyloxyl;

$Am_1$ and $Am_2$ are each independently selected from the group consisting of:

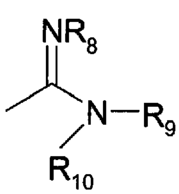 ; 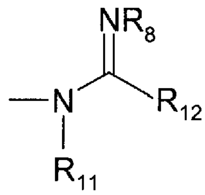 ; and 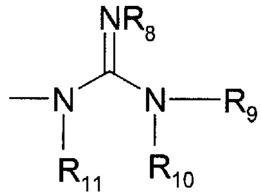 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

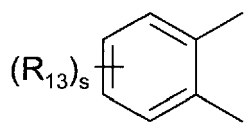 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl.

In some embodiments, at least one Argroup of the compound of Formula (I) is

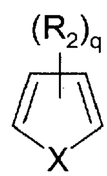 ,

wherein:

X is selected from the group consisting of O, S, CH, Se, Te, and $NR_1$, wherein $R_1$ is selected from the group consisting of H, alkyl, substituted alkyl, cycloalkyl, aryl, and substituted aryl;
q is an integer from 0 to 2; and
each $R_2$ is independently selected from the group consisting of H, alkyl, substituted alkyl, cycloalkyl, halo, hydroxyl, alkoxyl, aryl, aryloxyl, substituted aryl, and aralkyloxyl.

In some embodiments, $Ar_2$ and $Ar_3$ are each five-membered rings and the compound of Formula (I) has the following formula:

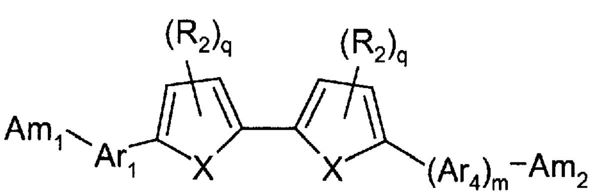 (II)

wherein:

m is an integer from 0 to 1;

$Ar_1$ and $Ar_4$ are independently selected from the group consisting of:

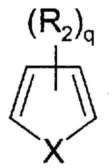 , 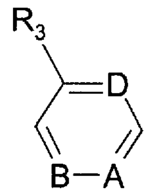 , 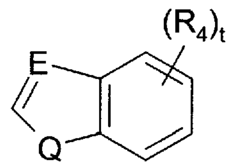 ,

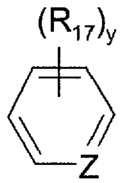 , and 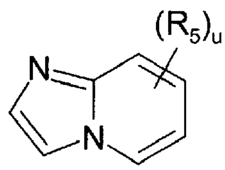 ,

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;
each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;
each E is independently selected from the group consisting of $CR_{18}$ and N; wherein $R_{18}$ is selected from the group consisting of H, halo, hydroxyl, alkyl, alkoxyl, substituted alkyl, cycloalkyl, aryl, aryloxyl, and substituted aryl;
each Z is independently selected from the group consisting of $CR_{19}$ and N; wherein $R_{19}$ is selected from the group consisting of H, halo, hydroxyl, alkyl, alkoxyl, substituted alkyl, cycloalkyl, aryl, aryloxyl, and substituted aryl;
each q is independently an integer from 0 to 2;
each y is independently an integer from 0 to 3;
each t is independently an integer from 0 to 3;
each u is independently an integer from 0 to 3;
each $R_2$, $R_3$, $R_4$, $R_5$ and $R_{17}$ is independently selected from the group consisting of H, alkyl, substituted alkyl, aryl, substituted aryl, halo, hydroxyl, alkoxyl, aryloxyl, and aralkyloxyl;

$Am_1$ and $Am_2$ are each independently selected from the group consisting of:

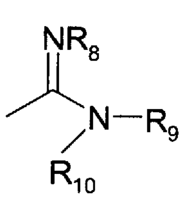 ; 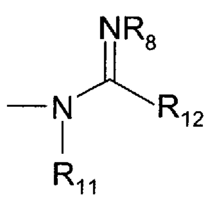 ; and 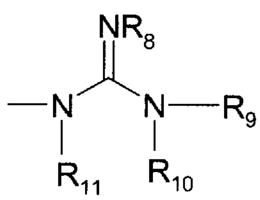 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or

$R_8$ and $R_9$ or $R_8$ and $R_{12}$ together are:

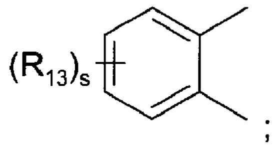

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl.

In some embodiments, $Ar_2$ and $Ar_3$ are each five-membered rings, $Ar_1$ is a six-membered aromatic ring, $Ar_4$, if present, is a five-membered or a six-membered ring, and the compound of Formula (I) has the following formula:

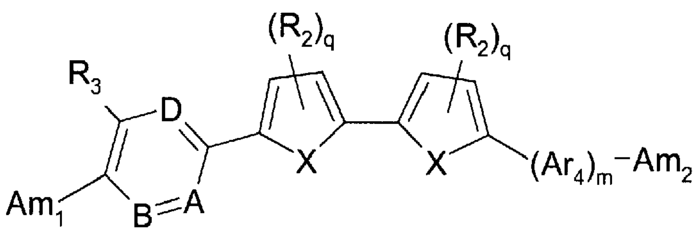

(III)

wherein:

m is selected from 0 and 1;

$Ar_4$ is selected from the group consisting of:

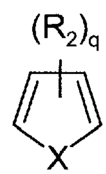

and

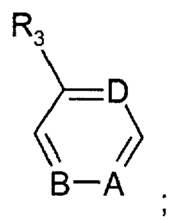

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl;

each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;

each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;

each q is independently an integer from 0 to 2;

each $R_2$ and $R_3$ is independently selected from the group consisting of H, alkyl, substituted alkyl, cycloalkyl, aryl, substituted aryl, halo, hydroxyl, alkoxyl, aryloxyl, and aralkyloxyl;

$Am_1$ and $Am_2$ are each independently selected from the group consisting of:

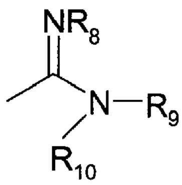 ; 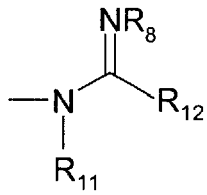 ; and 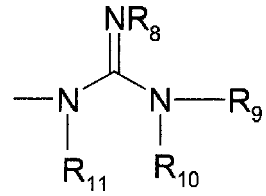 ;

wherein:

each $R_8$ is independently selected from the group consisting of H, hydroxyl, alkyl, substituted alkyl, aryl, substituted aryl, acyloxyl, and alkoxyl;

each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or

$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or

$R_8$ and $R_9$ or $R_8$ and $R_{12}$ together are:

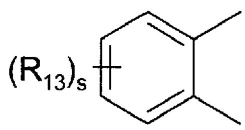 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl.

In some embodiments, the compound of Formula (I) has one of the following structures:

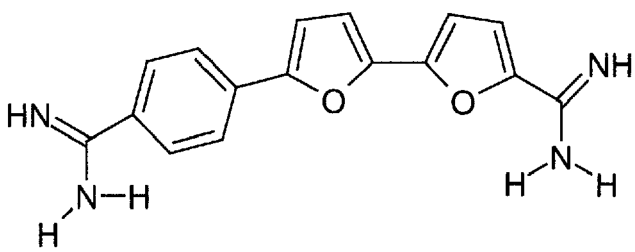 ,

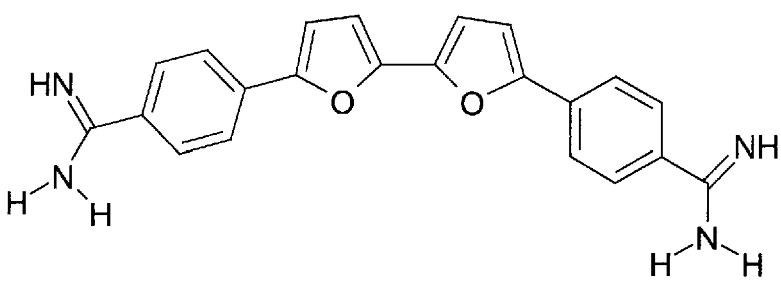 ,

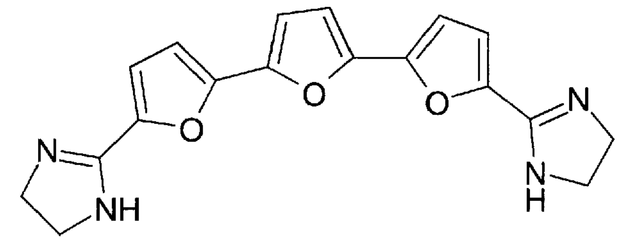

,

and

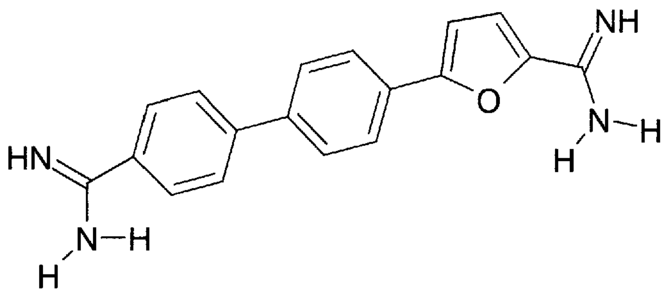

.

In some embodiments, two molecules of a compound of Formula (I-III) bind in a groove of the quadruplex DNA.
In some embodiments, the quadruplex DNA is a telomeric DNA sequence. In some embodiments, the telomeric DNA sequence is a human telomeric sequence. In some embodiments, the telomeric DNA is a nematodal DNA sequence. In some embodiments, the telomeric DNA sequence is a protozoal DNA sequence. In some embodiments, the telomeric DNA sequence is a sequence from a *Plasmodium* spp., *Trypanosoma* spp., or *Leishmania* spp.
In some embodiments, the presently disclosed subject matter provides a method for reducing telomeric extension, the method comprising providing a compound of Formula (I-III) and contacting the compound with telomeric DNA in the presence of telomerase, wherein the compound of Formula (I-III) is in an amount effective to stabilize and maintain the telomeric DNA in a quadruplex form, reducing the ability of the telomerase to bind to the telomeric DNA and thereby reducing telomeric extension.
In some embodiments, the presently disclosed subject matter provides a method for reducing the proliferative capacity in a cell by contacting the cell with an effective amount of a compound of Formula (I-III). In some embodiments, the cell is a cancer cell. Thus, in some embodiments, the presently disclosed subject matter provides a method for treating a cancer in a subject in need of treatment thereof, by administering to the subject an effective amount of a compound of Formula (I-III).
In some embodiments, the presently disclosed subject matter provides novel compounds of Formula (I) as described hereinabove, wherein m = 0, $Ar_1$, $Ar_2$, and $Ar_3$ are five-membered rings, and the compound has the following structure:

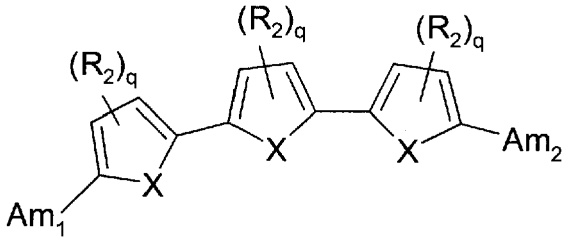

(IV)

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl, provided that at least one X is selected from Se, Te, and $NR_1$, wherein $R_1$ is selected from aryl and substituted aryl;
each q is independently an integer from 0 to 2;
each $R_2$ is independently selected from the group consisting of H, alkyl, substituted alkyl, cycloalkyl, aryl, substituted aryl, halo, hydroxyl, alkoxyl, aryloxyl, and aralkyloxyl;

$Am_1$ and $Am_2$ are each independently selected from the group consisting of:

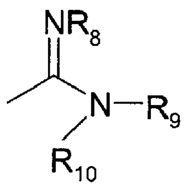 ; 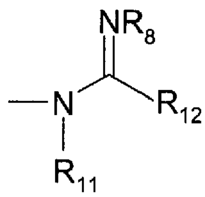 ; and 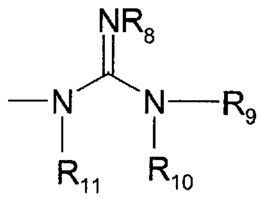 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

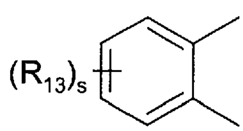 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl.

In some embodiments, the presently disclosed subject matter provides a pharmaceutical formulation comprising a compound of Formula (IV).

In some embodiments, the presently disclosed subject matter provides a method of treating a microbial infection, optionally caused by *Trypanosoma* spp., *Plasmodium* spp., and *Leishmania* spp., the method comprising administering an effective amount of a compound of Formula (IV) to a subject in need of treatment thereof.

Accordingly, it is an object of the presently disclosed subject matter to provide a method of binding quadruplex DNA by contacting quadruplex DNA with a compound of Formula (I-IV).

It is another object of the presently disclosed subject matter to provide a method of binding a dimer of a compound of Formula (I-IV) in a groove of quadruplex DNA.

It is another object of the presently disclosed subject matter to provide a method of reducing telomeric extension by contacting a compound of Formula (I-IV) with telomeric DNA in the presence of telomerase, wherein the compound of Formula(I-IV) stabilizes and maintains the telomeric DNA in a quadruplex secondary structure such that the ability of telomerase to bind to the telomeric DNA is lessened.

It is another object of the presently disclosed subject matter to provide a method of reducing the proliferative capacity in a cell.

It is another object of the presently disclosed subject matter to provide a method of treating a cancer in a subject.

It is another object of the presently disclosed subject matter to provide a method of treating a microbial infection in a subject.

It is another object of the presently disclosed subject matter to provide a novel compound of Formula (IV).

Certain objects of the presently disclosed subject matter having been stated hereinabove, which are addressed in whole or in part by the presently disclosed subject matter, other objects and aspects will become evident as the

description proceeds when taken in connection with the accompanying Examples as best described herein below.

BRIEF DESCRIPTION OF THE DRAWINGS

Figure 1 is a schematic representation of various secondary structures formed by intramolecular G-quadruplexes.
Figure 2A is a prior art drawing showing the groove sizes in a hybrid G-quadruplex containing two pairs of parallel DNA strands. Strand polarity is indicated by (+) and (•) in the ovals representing the nucleotide sugars.
Figure 2B is a prior art drawing showing the relative groove sizes in an antiparallel G-quadruplex.
Figure 3 is the circular dichroism (CD) spectra of presently disclosed compound 8 titrated into 3.29 μM d[$AG_3$($T_2AG_3$)$_3$] (SEQ ID NO: 1) in HEPES buffer containing 50 mM KCl. Compound:DNA ratios range from 0.5:1 to 5:1.
Figure 4 is an enlargement of the DNA region of the CD spectra of Figure 3.
Figure 5A is the CD spectra of presently disclosed compound **8** titrated into 3.23 μM d[$AG_3$($T_2AG_3$)$_3$] (SEQ ID NO: 1) in HEPES buffer containing 50 mM NaCl. Compound:DNA ratios range from 1:1 to 5:1.
Figure 5B is the CD spectra of presently disclosed compound **8** titrated into 3.45 μM d[$AG_3$($T_2AG_3$)$_3$] (SEQ ID NO: 1) in HEPES buffer containing 50 mM LiCl. Compound:DNA ratios range from 1:1 to 5:1.
Figure 6 is the CD spectra of presently disclosed compound **8** titrated into 2.30 μM c-MYC, d[$AG_3TG_4AG_3TG_4A$] (SEQ ID NO: 2), in HEPES buffer containing 50 mM KCl. Compound:DNA ratios range from 1:1 to 5:1.
Figure 7 is the CD spectra of presently disclosed compound **8** titrated into 9.40 μM Tetrahymena telomere, d[($T_2G_4$)$_4$] (SEQ ID NO: 4), in HEPES buffer containing 50 mM KCl. Compound:DNA ratios range from 1:1 to 5:1.
Figure 8 is the CD spectra of presently disclosed compound **8** titrated into 1.83 μM Oxytricha telomere, d[$G_4$($T_4G_4$)$_3$] (SEQ ID NO: 3), in HEPES buffer containing 50 mM KCl. Compound:DNA ratios range from 1:1 to 5:1.
Figure 9 is the CD spectra of presently disclosed compound **8** titrated into 5.63 μM d[(GC)$_7$] (SEQ ID NO: 5) in HEPES buffer containing 50 mM KCl. Compound:DNA ratios range from 1:1 to 5:1.
Figure 10 is the CD spectra of presently disclosed compound **8** titrated into 4.59 μM d[GCGAATTCGC] (SEQ ID NO: 6) in HEPES buffer containing 50 mM KCl. Compound:DNA ratios range from 1:1 to 5:1.
Figure 11 is the CD spectra of presently disclosed compound **8** titrated into 3.32 μM Thrombin-Binding Aptamer, d[$G_2T_2G_2TGTG_2T_2G_2$] (SEQ ID NO: 7), in HEPES buffer containing 50 mM KCl. Compound:DNA ratios range from 1:1 to 4:1.
Figure 12 is the absorbance spectra of d[$AG_3$($T_2AG_3$)$_3$] (SEQ ID NO: 1) titrated into a cuvette containing 8.2 μM presently disclosed compound **8** in a HEPES buffer containing 50 mM KCl, up to a final DNA concentration of 4.4 μM.
Figure 13 is the CD spectra of presently disclosed compound **8** titrated into d[TAGGGUTAGGGT] (SEQ ID NO: 9) hairpin dimer (quadruplex concentration 4 μM) in the presence of 50 mM KCl, 10 mM $K_2HPO_4$, and 0.1 mM EDTA adjusted to pH 7.0. Total $K^+$ concentration is 70 mM.
Figure 14 is the CD spectra of presently disclosed compound **8** titrated into d[TAGGGUTAGGGT] (SEQ ID NO: 9) hairpin dimer (quadruplex concentration 4 μM) in the presence of 50 mM NaCl, 10 mM $Na_2HPO_4$, and 0.01 mM EDTA adjusted to pH 7.0. Total $Na^+$ concentration is 70 mM.
Figure 15 is the CD spectra of presently disclosed compound **8** titrated into d[TAGGGUUAGGGT] (SEQ ID NO: 10) hairpin dimer (quadruplex concentration 4 μM) in the presence of 50 mM KCl, 10 mM $K_2HPO_4$, and 0.1 mM EDTA adjusted to pH 7.0. Total $K^+$ concentration is 70 mM.
Figure 16 is the CD spectra of presently disclosed compound **8** titrated into d[TTAGGGT] (SEQ ID NO: 12; single strand concentration 16 μM) in the presence of 50 mM KCl, 10 mM $K_2HPO_4$, and 0.1 mM EDTA adjusted to pH 7.0. Total concentration is 70 mM.
Figure 17 is the CD spectra of presently disclosed compound **8** titrated into d[TGGGGT] (SEQ ID NO: 13; single strand concentration 16 μM) in the presence of 50 mM KCl, 10 mM $K_2HPO_4$, and 0.1 mM EDTA adjusted to pH 7.0. Total $K^+$ concentration is 70 mM.
Figure 18 shows the surface plasmon resonance (SPR) steady-state binding plots for presently disclosed compound **8** with d[$AG_3$($T_2AG_3$)$_3$] (SEQ ID NO: 1) and the hairpin duplex d[CGAATTCGTCTCCGAATTCG] (SEQ ID NO: 8) obtained in HEPES buffer containing 200 mM KCl. The concentration axis is the unbound compound **8** concentration in the flow solution; RU represents the instrument response (resonance units). Fitting errors due to random point scatter are less than ± 5%.
Figure 19 is a Scatchard plot of fluorescence titration. Excitation was set at 393.5 nm and emission was monitored at the fluorescence peak (469 nm). d[$AG_3$($T_2AG_3$)$_3$] (SEQ ID NO: 1) was incrementally added to a cuvette containing 4.975 μM of presently disclosed compound **8** in HEPES buffer containing 50 mM KCl, up to a total DNA concentration of 4.04 μM. $C_f$ is defined as the concentration of unbound compound **8,** and r is defined as the concentration of bound compound **8** divided by the concentration of DNA.
Figure 20 is the fluorescence excitation spectra of 0.3 μM presently disclosed compound **8** alone and in the presence

of 8 μM d[$AG_3(T_2AG_3)_3$] (SEQ ID NO: 1) in HEPES buffer containing 50 mM KCl. The emission wavelength was set at 469 nm.

Figure 21 is $H^1$ NMR spectra showing the imino protons of d[TAGGGUTAGGGT] (SEQ ID NO: 9) hairpin dimer (0.1 mM quadruplex concentration) at 0:1, 1:1, and 2:1 mole ratios of presently disclosed compound **8** (bottom to top) obtained at 35 °C in 50 mM KCl, 10 mM $K_2HPO_4$, 0.1 mM EDTA and 0.05 mM DSS as an internal standard. The guanine imino protons that are involved in the G-tetrad formation resonate between 12.5 to 10.5 ppm. The free DNA was annealed overnight before recording the spectra.

Figure 22 are $H^1$ NMR spectra showing the imino protons of d[TAGGGUUAGGGT] (SEQ ID NO: 10) hairpin dimer (0.1 mM quadruplex concentration) at 0:1, 1:1, and 2:1 mole ratios of presently disclosed compound **8** (bottom to top) obtained at 35°C in 50 mM KCl, 10 mM $K_2HPO_4$, 0.1 mM EDTA and 0.05 mM DSS as an internal standard. The guanine imino protons that are involved in the G-tetrad formation resonate between 12.5 to 10.5 ppm. The free DNA was annealed for 2 hours prior to obtaining the spectra.

Figure 23 are $H^1$ NMR spectra showing the imino protons of d[TAGGGUTAGGGU] (SEQ ID NO: 11) hairpin dimer (0.1 mM quadruplex concentration) at 0:1, 1:1, and 2:1 mole ratios of presently disclosed compound **8** (bottom to top) obtained at 35 °C in 50 mM KCl, 10 mM $K_2HPO_4$, 0.1 mM EDTA and 0.05 mM DSS as an internal standard. The guanine imino protons that are involved in the G-tetrad formation resonate between 12.5 to 10.5 ppm. The free DNA was annealed overnight before collecting the spectra.

Figure 24 shows the expanded TOCSY (60 ms mixing time) spectra of uracil H5-H6 cross-peaks at 35 °C for d [TAGGGUUAGGGT] (SEQ ID NO: 10; 0.1 to 0.5 mM quadruplex concentration) at 0:1 and 2:1 mole ratios of presently disclosed compound **8** to DNA. Peaks labeled U6 and U7 correspond to the major parallel form whereas the other two peaks correspond to the minor antiparallel form. After titrating with the compound, the crosspeak intensity of the antiparallel form decreases, which is evident in the bottom spectrum suggesting that the compound preferentially binds to the parallel form and stabilizes it. Spectra were obtained with a mixing time of 60 ms at 35 °C in the presence of 50 mM KCl, 10 mM $K_2HPO_4$, 0.1 mM EDTA and 0.05 mM DSS as an internal reference.

Figure 25 shows a section of the TOCSY NMR spectra of d[TAGGGUUAGGGT] (SEQ ID NO: 10) and presently disclosed compound **8** at 0:1 and 2:1 ratios of compound to DNA. The cross-peaks correspond to the $CH_3$ protons of C5 and the H6 proton of thymine residues. The peak on the left side of the top spectrum is overlapping peaks corresponding to T1 and T12 of the major parallel species whereas the peak on the right is the overlapping peaks corresponding to the minor antiparallel form. After titrating with compound **8,** one of the peaks of the major species shifts upfield to a new position indicated by the arrow on the bottom spectrum. Spectra were obtained with a mixing time of 60 ms at 35°C in the presence of 50 mM KCl, 10 mM $K_2HPO_4$, 0.1 mM EDTA, and 0.05 mM DSS as an internal reference.

Figure 26A is the TOCSY spectrum for uracil H5-H6 of d[TAGGGUTAGGGU] (SEQ ID NO: 11) sequence of 35 °C in the presence of 50 mM KCl, 10 mM $K_2HPO_4$, 0.1 mM EDTA and 0.05 mM DSS as an internal reference.

Figure 26B is the TOCSY spectrum for Uracil H5-H6 of d[TAGGGUTAGGGU] (SEQ ID NO: 11) at 35 °C at a 1:1 mole ratio of **8.** The spectrum was obtained with a mixing time of 60 ms at 35 °C in the presence of 50 mM KCl, 10 mM $K_2HPO_4$, 0.1 mM EDTA and 0.05 mM DSS as an internal reference.

Figure 26C is the TOCSY spectrum for Uracil H5-H6 of d[TAGGGUTAGGGU] (SEQ ID NO: 11) at 35 °C at a 2:1 mole ratio of presently disclosed compound **8.** The spectrum was obtained with a mixing time of 60 ms at 35 °C in the presence of 50 mM KCl, 10 mM $K_2HPO_4$, 0.1 mM EDTA and 0.05 mM DSS as an internal reference.

## DETAILED DESCRIPTION

The presently disclosed subject matter will now be described more fully hereinafter with reference to the accompanying Examples, in which representative embodiments are shown. The presently disclosed subject matter can, however, be embodied in different forms and should not be construed as limited to the embodiments set forth herein. Rather, these embodiments are provided so that this disclosure will be thorough and complete, and will fully convey the scope of the embodiments to those skilled in the art.

Unless otherwise defined, all technical and scientific terms used herein have the same meaning as commonly understood by one of ordinary skill in the art to which this presently described subject matter belongs. All publications, patent applications, patents, and other references mentioned herein are incorporated by reference in their entirety.

Throughout the specification and claims, a given chemical formula or name shall encompass all optical and stereoisomers, as well as racemic mixtures where such isomers and mixtures exist.

## I. Definitions

As used herein the term "apoptosis" refers to programmed cell death, a cellular process comprising the self-destruction of a cell in a multicellular organism. The process is often the result of unrepaired DNA damage, such as

shortened telomeres.

The term "binding" or "bind" as used herein, refers to the noncovalent association of one or more molecules with another molecule. The molecules involved in binding can be small molecules produced by organic synthesis, portions of DNA or RNA molecules, proteins or combinations thereof. Thus, "binding" can involve hybridization or more general hydrogen bonding and/or other non-covalent interactions, such as ionic bonding, hydrophobic interactions, interactions based on Van der Waals forces or London dispersion forces, and dipole-dipole interactions.

The term "cancer" as used herein refers to diseases caused by uncontrolled cell division and the ability of cells to metastasize, or to establish new growth in additional sites. The terms "malignant", "malignancy", "neoplasm", "tumor" and variations thereof refer to cancerous cells or groups of cancerous cells.

Specific types of cancer include, but are not limited to, skin cancers, connective tissue cancers, adipose cancers, breast cancers, lung cancers, stomach cancers, pancreatic cancers, ovarian cancers, cervical cancers, uterine cancers, anogenital cancers, kidney cancers, bladder cancers, colon cancers, prostate cancers, central nervous system (CNS) cancers, retinal cancer, blood, and lymphoid cancers.

As used herein the term "dimer" refers to two molecules of a compound. The two molecules can be covalently linked, either directly or through a linking group, such as a hydrocarbon chain. The hydrocarbon chain can be further substituted or contain one or more heteroatoms along its backbone. Alternatively, the two molecules of a dimer are not covalently linked. In particular, the term "dimer" is used herein to describe two molecules that are both non-covalently bound to a third molecule, such as a DNA sequence.

As used herein, the terms "G-quadruplex" and "quadruplex" can be used interchangeably and refer to polynucleotide secondary structures containing four guanine-rich sequences that form three or more guanine tetrads. The four guanine-rich sequences can all be part of a single polynucleotide molecule. Alternatively, the four guanine-rich sequences can be part of two, three or four polynucleotide molecules.

A "tetrad" or "G-tetrad" as used herein refers to a planar array of four guanine bases.

As used herein, the term "telomeric DNA" refers to a DNA sequence at the end of a chromosome. Such sequences generally contain many guanines. In humans and other vertebrates, the telomeric DNA sequence contains many d[$T_2AG_3$] repeats. During cell division, not all of the telomeric sequence is copied, and the telomeric sequence becomes shorter. After many rounds of cell division, the telomeric DNA sequence becomes too short for the cell to be viable and apoptosis is triggered.

The phrase "reducing telomeric extension" as used herein refers to lessening or stopping the addition of nucleotides to the ends of telomeres. For example, reducing telomeric extension can refer to stopping or reducing the ability of telomerase enzyme from adding d[$T_2AG_3$] repeat sequences to the ends of telomeric DNA in humans or other vertebrates.

As used herein the term "alkyl" refers to $C_{1-20}$ inclusive, linear (*i.e.*, "straight-chain"), branched, or cyclic, saturated or at least partially and in some cases fully unsaturated (*i.e.*, alkenyl and alkynyl) hydrocarbon chains, including for example, methyl, ethyl, propyl, isopropyl, butyl, isobutyl, *tert*-butyl, pentyl, hexyl, octyl, ethenyl, propenyl, butenyl, pentenyl, hexenyl, octenyl, butadienyl, propynyl, butynyl, pentynyl, hexynyl, heptynyl, and allenyl groups. "Branched" refers to an alkyl group in which a lower alkyl group, such as methyl, ethyl or propyl, is attached to a linear alkyl chain. "Lower alkyl" refers to an alkyl group having 1 to about 8 carbon atoms (*i.e.*, a $C_{1-8}$ alkyl), e.g., 1, 2, 3, 4, 5, 6, 7, or 8 carbon atoms. "Higher alkyl" refers to an alkyl group having about 10 to about 20 carbon atoms, e.g., 10, 11, 12, 13, 14, 15, 16, 17, 18, 19, or 20 carbon atoms. In certain embodiments, "alkyl" refers, in particular, to $C_{1-8}$ straight-chain alkyls. In other embodiments, "alkyl" refers, in particular, to $C_{1-8}$ branched-chain alkyls.

Alkyl groups can optionally be substituted (a "substituted alkyl") with one or more alkyl group substituents, which can be the same or different. The term "alkyl group substituent" includes but is not limited to alkyl, substituted alkyl, halo, arylamino, acyl, hydroxyl, aryloxyl, alkoxyl, alkylthio, arylthio, aralkyloxyl, aralkylthio, carboxyl, alkoxycarbonyl, oxo, and cycloalkyl. There can be optionally inserted along the alkyl chain one or more oxygen, sulfur or substituted or unsubstituted nitrogen atoms, wherein the nitrogen substituent is hydrogen, lower alkyl (also referred to herein as "alkylaminoalkyl"), or aryl.

Thus, as used herein, the term "substituted alkyl" includes alkyl groups, as defined herein, in which one or more atoms or functional groups of the alkyl group are replaced with another atom or functional group, including for example, alkyl, substituted alkyl, halogen, aryl, substituted aryl, alkoxyl, hydroxyl, nitro, amino, alkylamino, dialkylamino, sulfate, and mercapto.

The term "aryl" is used herein to refer to an aromatic substituent that can be a single aromatic ring, or multiple aromatic rings that are fused together, linked covalently, or linked to a common group, such as, but not limited to, a methylene or ethylene moiety. The common linking group also can be a carbonyl, as in benzophenone, or oxygen, as in diphenylether, or nitrogen, as in diphenylamine. The term "aryl" specifically encompasses heterocyclic aromatic compounds. The aromatic ring(s) can comprise phenyl, naphthyl, biphenyl, diphenylether, diphenylamine and benzophenone, among others. In particular embodiments, the term "aryl" means a cyclic aromatic comprising about 5 to about 10 carbon atoms, e.g., 5, 6, 7, 8, 9, or 10 carbon atoms, and including 5- and 6-membered hydrocarbon and heterocyclic

aromatic rings.

The aryl group can be optionally substituted (a "substituted aryl") with one or more aryl group substituents, which can be the same or different, wherein "aryl group substituent" includes alkyl, substituted alkyl, aryl, substituted aryl, aralkyl, hydroxyl, alkoxyl, aryloxyl, aralkyloxyl, carboxyl, acyl, halo, nitro, alkoxycarbonyl, aryloxycarbonyl, aralkoxycarbonyl, acyloxyl, acylamino, aroylamino, carbamoyl, alkylcarbamoyl, dialkylcarbamoyl, arylthio, alkylthio, alkylene, and -NR'R", wherein R' and R" can each be independently hydrogen, alkyl, substituted alkyl, aryl, substituted aryl, and aralkyl.

Thus, as used herein, the term "substituted aryl" includes aryl groups, as defined herein, in which one or more atoms or functional groups of the aryl group are replaced with another atom or functional group, including for example, alkyl, substituted alkyl, halogen, aryl, substituted aryl, alkoxyl, hydroxyl, nitro, amino, alkylamino, dialkylamino, sulfate, and mercapto.

Specific examples of aryl groups include, but are not limited to, cyclopentadienyl, phenyl, furan, thiophene, pyrrole, pyran, pyridine, imidazole, benzimidazole, isothiazole, isoxazole, pyrazole, pyrazine, triazine, pyrimidine, quinoline, isoquinoline, indole, carbazole, and the like.

A structure represented generally by a formula such as:

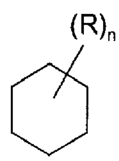 or 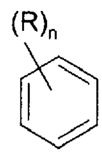

as used herein refers to a ring structure, for example, but not limited to a 3-carbon, a 4-carbon, a 5-carbon, a 6-carbon, and the like, aliphatic and/or aromatic cyclic compound comprising a substituent R group, wherein the R group can be present or absent, and when present, one or more R groups can each be substituted on one or more available carbon atoms of the ring structure. The presence or absence of the R group and number of R groups is determined by the value of the integer n. Each R group, if more than one, is substituted on an available carbon of the ring structure rather than on another R group. For example, the structure:

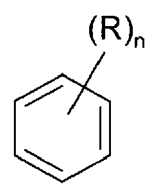

wherein n is an integer from 0 to 2 comprises compound groups including, but not limited to:

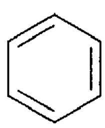 ; 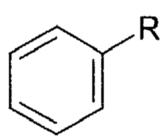 ; 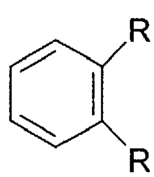 ; 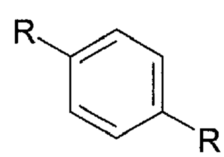 ;

and the like.

A dashed line representing a bond in a cyclic ring structure indicates that the bond can be either present or absent in the ring. That is a dashed line representing a bond in a cyclic ring structure indicates that the ring structure is selected from the group consisting of a saturated ring structure, a partially saturated ring structure, and an unsaturated ring structure.

In some embodiments, the compounds described by the presently disclosed subject matter contain a linking group. As used herein, the term "linking group" comprises a chemical moiety, such as a furanyl, phenylene, thienyl, and pyrrolyl radical, which is bonded to two or more other chemical moieties, in particular aryl groups, to form a stable structure.

When a named atom of an aromatic ring or a heterocyclic aromatic ring is defined as being "absent," the named atom is replaced by a direct bond. When the linking group or spacer group is defined as being absent, the linking group

or spacer group is replaced by a direct bond.

"Alkylene" refers to a straight or branched bivalent aliphatic hydrocarbon group having from 1 to about 20 carbon atoms, e.g., 1, 2, 3, 4, 5, 6, 7, 8, 9, 10, 11, 12, 13, 14, 15, 16, 17, 18, 19, or 20 carbon atoms. The alkylene group can be straight, branched or cyclic. The alkylene group also can be optionally unsaturated and/or substituted with one or more "alkyl group substituents." There can be optionally inserted along the alkylene group one or more oxygen, sulfur or substituted or unsubstituted nitrogen atoms (also referred to herein as "alkylaminoalkyl"), wherein the nitrogen substituent is alkyl as previously described. Exemplary alkylene groups include methylene ($-CH_2-$); ethylene ($-CH_2-CH_2-$); propylene ($-(CH_2)_3-$); cyclohexylene ($-C_6H_{10}-$); -CH=CH-CH=CH-; $-CH=CH-CH_2-$; $-(CH_2)_q-N(R)-(CH_2)_r-$, wherein each of q and r is independently an integer from 0 to about 20, e.g., 0, 1, 2, 3, 4, 5, 6, 7, 8, 9, 10, 11, 12, 13, 14, 15, 16, 17, 18, 19, or 20, and R is hydrogen or lower alkyl; methylenedioxyl ($-O-CH_2-O-$); and ethylenedioxyl ($-O-(CH_2)_2-O-$). An alkylene group can have about 2 to about 3 carbon atoms and can further have 6-20 carbons.

As used herein, the term "acyl" refers to an organic carboxylic acid group wherein the -OH of the carboxyl group has been replaced with another substituent (i.e., as represented by RCO-, wherein R is an alkyl or an aryl group as defined herein). As such, the term "acyl" specifically includes arylacyl groups, such as an acetylfuran and a phenacyl group. Specific examples of acyl groups include acetyl and benzoyl.

"Cyclic" and "cycloalkyl" refer to a non-aromatic mono- or multicyclic ring system of about 3 to about 10 carbon atoms, e.g., 3, 4, 5, 6, 7, 8, 9, or 10 carbon atoms. The cycloalkyl group can be optionally partially unsaturated. The cycloalkyl group also can be optionally substituted with an alkyl group substituent as defined herein, oxo, and/or alkylene. There can be optionally inserted along the cyclic alkyl chain one or more oxygen, sulfur or substituted or unsubstituted nitrogen atoms, wherein the nitrogen substituent is hydrogen, alkyl, substituted alkyl, aryl, or substituted aryl, thus providing a heterocyclic group. Representative monocyclic cycloalkyl rings include cyclopentyl, cyclohexyl, and cycloheptyl. Multicyclic cycloalkyl rings include adamantyl, octahydronaphthyl, decalin, camphor, camphane, and noradamantyl.

"Alkoxyl" refers to an alkyl-O- group wherein alkyl is as previously described. The term "alkoxyl" as used herein can refer to, for example, methoxyl, ethoxyl, propoxyl, isopropoxyl, butoxyl, t-butoxyl, and pentoxyl. The term "oxyalkyl" can be used interchangably with "alkoxyl".

"Aryloxyl" refers to an aryl-O- group wherein the aryl group is as previously described, including a substituted aryl. The term "aryloxyl" as used herein can refer to phenyloxyl or hexyloxyl, and alkyl, substituted alkyl, halo, or alkoxyl substituted phenyloxyl or hexyloxyl.

"Aralkyl" refers to an aryl-alkyl- group wherein aryl and alkyl are as previously described, and included substituted aryl and substituted alkyl. Exemplary aralkyl groups include benzyl, phenylethyl, and naphthylmethyl.

"Aralkyloxyl" refers to an aralkyl-O- group wherein the aralkyl group is as previously described. An exemplary aralkyloxyl group is benzyloxyl.

"Dialkylamino" refers to an -NRR' group wherein each of R and R' is independently an alkyl group and/or a substituted alkyl group as previously described. Exemplary alkylamino groups include ethylmethylamino, dimethylamino, and diethylamino.

"Alkoxycarbonyl" refers to an alkyl-O-CO- group. Exemplary alkoxycarbonyl groups include methoxycarbonyl, ethoxycarbonyl, butyloxycarbonyl, and t-butyloxycarbonyl.

"Aryloxycarbonyl" refers to an aryl-O-CO- group. Exemplary aryloxycarbonyl groups include phenoxy- and naphthoxy-carbonyl.

"Aralkoxycarbonyl" refers to an aralkyl-O-CO- group. An exemplary aralkoxycarbonyl group is benzyloxycarbonyl.

"Carbamoyl" refers to an $H_2N-CO-$ group.

"Alkylcarbamoyl" refers to a R'RN-CO- group wherein one of R and R' is hydrogen and the other of R and R' is alkyl and/or substituted alkyl as previously described.

"Dialkylcarbamoyl" refers to a R'RN-CO- group wherein each of R and R' is independently alkyl and/or substituted alkyl as previously described.

"Acyloxyl" refers to an acyl-O- group wherein acyl is as previously described.

"Acylamino" refers to an acyl-NH- group wherein acyl is as previously described.

The term "amino" refers to the $-NH_2$ group.

The term "carbonyl" refers to the -(C=O)- group.

The term "carboxyl" refers to the -COOH group.

The terms "halo", "halide", or "halogen" as used herein refer to fluoro, chloro, bromo, and iodo groups.

The term "hydroxyl" refers to the -OH group.

The term "hydroxyalkyl" refers to an alkyl group substituted with an -OH group.

The term "mercapto" refers to the -SH group.

The term "oxo" refers to a compound described previously herein wherein a carbon atom is replaced by an oxygen atom.

The term "nitro" refers to the $-NO_2$ group.

The term "thio" refers to a compound described previously herein wherein a carbon or oxygen atom is replaced by a sulfur atom.

The term "sulfate" refers to the $-SO_4$ group.

When the term "independently selected" is used, the substituents being referred to (e.g., R groups, such as groups $R_1$ and $R_2$, or groups X and Y), can be identical or different. For example, both $R_1$ and $R_2$ can be substituted alkyls, or $R_1$ can be hydrogen and $R_2$ can be a substituted alkyl, and the like.

A named "R", "X ", "A", "B," "D", "E" or "Q" group will generally have the structure that is recognized in the art as corresponding to a group having that name, unless specified otherwise herein. For the purposes of illustration, certain representative "R," "X," and "A" groups as set forth above are defined below. These definitions are intended to supplement and illustrate, not preclude, the definitions that would be apparent to one of ordinary skill in the art upon review of the present disclosure.

The term "reflux" and grammatical derivations thereof refer to boiling a liquid, such as a solvent, in a container, such as a reaction flask, with which a condenser is associated, thereby facilitating continuous boiling without loss of liquid, due to the condensation of vapors on the interior walls of the condenser.

The term "aprotic solvent" refers to a solvent molecule which can neither accept nor donate a proton. Typical aprotic solvents include, but are not limited to, acetone, acetonitrile, benzene, butanone, butyronitrile, carbon tetrachloride, chlorobenzene, chloroform, 1,2-dichloroethane, dichloromethane, diethyl ether, dimethylacetamide, *N,N*-dimethylformamide (DMF), dimethylsulfoxide (DMSO), 1,4-dioxane, ethyl acetate, ethylene glycol dimethyl ether, hexane, *N*-methylpyrrolidone, pyridine, tetrahydrofuran (THF), and toluene. Certain aprotic solvents are polar solvents. Examples of polar aprotic solvents include, but are not limited to, acetone, acetonitrile, butanone, *N,N*-dimethylformamide, and dimethylsulfoxide. Certain aprotic solvents are non-polar solvents. Examples of nonpolar, aprotic solvents include, but are not limited to, diethyl ether, aliphatic hydrocarbons, such as hexane, aromatic hydrocarbons, such as benzene and toluene, and symmetrical halogenated hydrocarbons, such as carbon tetrachloride.

The term "protic solvent" refers to a solvent molecule which contains a hydrogen atom bonded to an electronegative atom, such as an oxygen atom or a nitrogen atom. Typical protic solvents include, but are not limited to, carboxylic acids, such as acetic acid, alcohols, such as methanol and ethanol, amines, amides, and water.

## II. General Considerations

### II.A. Telomerase

Human telomerase has at least two subunits. One subunit, the hTR, or human Telomerase RNA unit, is a 450 bp long sequence of untranslated RNA that contains a 3'-CCCAAUCCC base sequence. The hTR 3'CCCAAUCCC sequence hybridizes to the last three guanines of a telomere. The second subunit of telomerase, the Telomerase Reverse Transcriptase, or hTERT unit, is a 1131 amino acid polypeptide that, like other reverse transcriptases, creates single stranded DNA using single stranded RNA as a template. In the case of hTERT, the enzyme catalyzes the addition of a new 5'-TTAGGG sequence onto the end of the telomere using the hybridized hTR unit as a template.

As described hereinabove, normal mammalian somatic cells do not contain active telomerase. When such a cell divides, not all of the telomere sequence is replicated. Progressive rounds of cell division result in shortening of the telomeres by between 50 and 200 nucleotides per round. Once the telomeres are shortened beyond a certain length, the cell no longer divides and apoptosis is triggered. Almost all tumor cells have shortened telomeres that are maintained at a constant length by telomerase. Thus, telomerase activity is associated with uncontrolled cell growth and the immortality of tumor cells.

### II.B. G-Quadruplexes

A key structural feature of quadruplex DNA is a series of stacked guanine tetrads held together in a coplanar cyclic array by Hoogsteen and Watson-Crick hydrogen bonds. This cyclic tetrad array is shown below in Scheme 1.

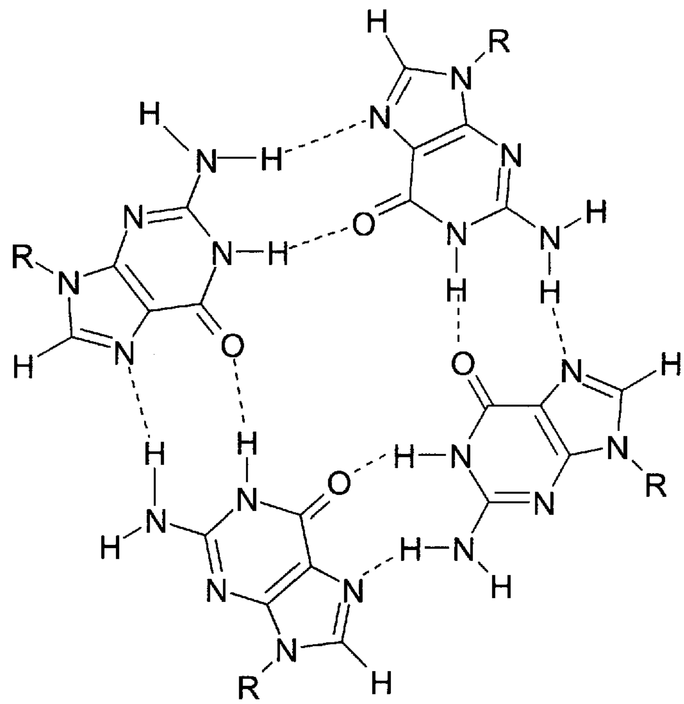

**Scheme 1**. G-Tetrad Array.

Single-stranded guanine-rich sequences fold into a variety of secondary structures containing three stacked tetrads which are further stabilized by $\pi$-$\pi$ stacking interactions, as well as by coordination with cations located between or within the tetrads. All four strands of the intramolecular G-quadruplex can be oriented in the same direction, forming a parallel quadruplex. Alternatively, the strands can alternate direction, forming an antiparallel quadruplex. Some of the different quadruplex secondary structures reported for telomeric DNA are shown in Figure 1. An anti-parallel, basket-type quadruplex structure has been reported for human telomeric DNA sequences in the presence of $Na^+$. See Wang, Y., and Patel, D. J., Structure, 1, 263-282 (1993). In the presence of $K^+$, a propeller-type parallel structure has been reported for human telomeric DNA. See Parkinson, G. N., et al., Nature, 876-880 (2002). A mixed-type quadruplex structure has been reported from NMR studies of a *Tetrahymena* ciliated protozoa's telomeric DNA sequence. See Wang, Y., and Patel, D. J., Structure, 2, 1141-1156 (1994). The *Tetrahymena* sequence contains a d[$T_2G_4$] repeat instead of the human sequence's d[$T_2AG_3$] repeat. Intermolecular quadruplexes also can be formed from two or more separate G-rich DNA strands.

Unlike in most DNA, wherein the glycosidic bond is *anti,* guanines in G-quadruplex tetrads can be either *anti* or *syn.* In particular, if adjacent guanines in the same tetrad are on parallel strands, they each have the same glycosidic torsional angle. If the adjacent guanines are on antiparallel strands, they have opposite glycosidic torsional angles. Stuctures showing the two glycosidic torsional angles are shown in Scheme 2, below. The different combinations of strand direction and glycosidic bond angle lead to a variety of possible groove sizes. Two examples are shown in Fig. 2A and Fig. 2B. See Simonsson, T., Biol. Chem., 382, 621-628 (2001). Fig. 2A shows the grooves of a quadruplex with two pairs of parallel strands. The two grooves between the parallel stands are of an intermediate size, while those between the anti-parallel stands are either narrow or wide. Fig. 2B shows a quadruplex wherein the four DNA strands alternate direction. The grooves of the fully anti-parallel quadruplex are either narrow or wide.

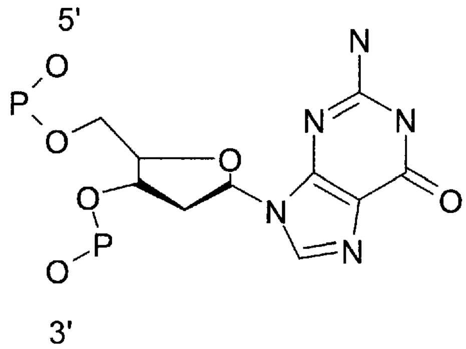

Syn

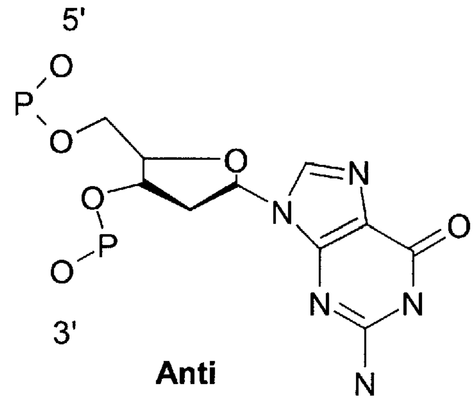

Anti

**Scheme 2**. Syn and Anti Glycosidic Bonds.

II.C G-Quadruplex/Small Molecule Interactions

As discussed hereinabove, the binding of several small molecules with DNA quadruplexes has been studied. Most of these molecules are thought to stack on the ends of quadruplexes, in the loop regions. Two examples of such molecules are telomestatin, a natural product from *Streptomyces anulatus* 3533-SV4, and the expanded porphyrin-type molecule, Se2SAP. See Rezler, E. M., et al., J. Am. Chem. Soc., 127, 9439-9447 (2005). Further examples of quadruplex end-binding compounds include *N*,*N*'-bis(2-piperdinoethyl)-3,4,9,10-perylenetetracarboxylic acid diimide (PIPER) and related perylenetetracarboxylic acid diimides. See, for example, Han, H., et al., Biochemistry, 38, 6981-6986 (1999); and U.S. Patent No. 6,689,887 to Kerwin, S. M., et al. The structures of these quadruplex end-binding compounds are shown in Scheme 3, below.

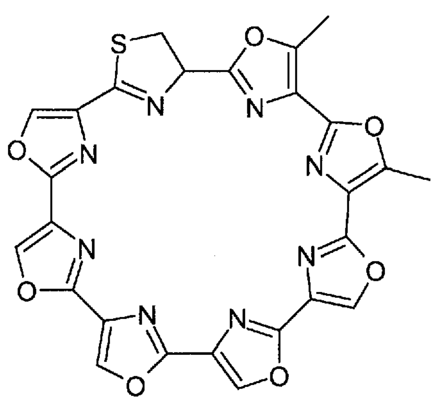

Telomestatin

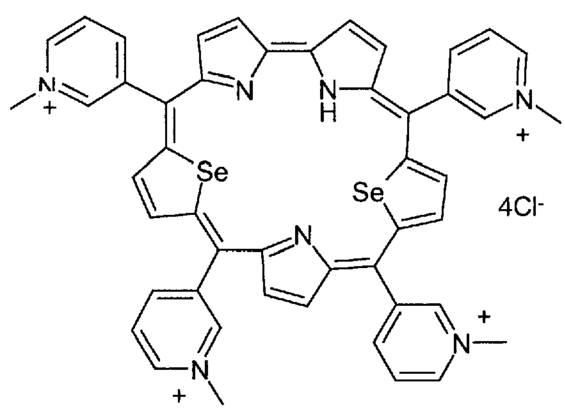

Se2SAP

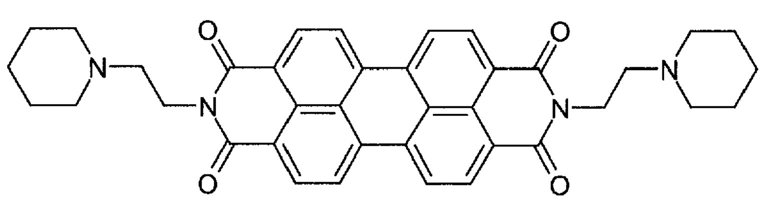

PIPER

**Scheme 3**. G-Quadruplex End-Stacking Compounds.

Ethidium bromide has been proposed to intercalate between tetrads; however, structural evidence to support this claim is lacking. See Guo, Q., et al., Biochemistry, 31, 2451-2455 (1992). In general, G-quadruplex intercalation is not considered to be a viable binding mode due to its high energetic cost. See Han, H., et al., J. Am. Chem. Soc., 123, 8902-8913 (2001). Further, with regard to the development of a quadruplex-binding, anti-cancer therapeutic, intercalation is less desirable a binding mode due to the similarity with duplex DNA intercalation.

Quadruplex groove-binding offers an attractive, alternative strategy for exploiting the structural differences between duplex and quadruplex DNA, leading to possible increased selectivity of recognition over traditional planar end-stacking molecules. Since groove dimensions vary significantly according to the type of quadruplex, groove-binding also offers the opportunity for obtaining increased selectivity for a particular quadruplex structure. The geometry of the G-quartet is not greatly affected by the glycosidic (syn/anti) conformation or the strand orientation of the quadruplex. See Mergny, J.-L., et al., Anti-Cancer Drug Design, 14, 327-339 (1999). Groove dimensions, however, are strongly dependent on these factors, resulting in a wide variety of possible groove geometries, which vary in size, electrostatic potential, hydrogen-bonding characteristics, steric effects, and hydration. See Randazzo, A., et al., Nucleosides, Nucleotides, and Nucleic Acids, 21, 535-545 (2002) and Simonsson, T., Biol. Chem., 382, 621-628, (2001). The groove structural variation allows for the targeting of certain quadruplex sequences with a high degree of selectivity.

Groove binding of carbocyanine dye compounds to DNA quadruplexes formed from nonhuman G-rich DNA sequences has been suggested. Chen et al. have investigated the interaction of the carbocyanine dye 3,3'-diethyloxadicarbocyanine (DODC) with a two-stranded hairpin G-quadruplex. See Chen, Q., et al., Proc. Natl. Acad. Sci. USA, 93, 2635-2639 (1996). Spectroscopic data suggest that this molecule binds in one or more of the quadruplex grooves. Satellite hole spectroscopy studies by Chang and coworkers support the groove-binding model for this particular ligand with quadruplexes. See Cheng, J.-Y., et al., J. Phys. Chem., 102, 5542-5546 (1998); Chiang, C.-C., et al., Proceedings of the National Science Council ROC(A), 23, 679-694 (1999). David et al. have used electrospray mass spectrometry to investigate the interaction of the carbocyanine dye *N,N'*-diethylthiacarbocyanine iodide (DTC) with a quadruplex formed by [d($T_2G_5T$)] sequences. See David, W. M., et al., Anal. Chem., 74, 2029-2033 (2002). The fragmentation pattern upon ionization suggests that this molecule binds in the grooves of this quadruplex, as well. Kerwin and coworkers had previously described that DTC binds to a quadruplex of [d($T_2AG_3$)] sequences selectively over triplex DNA and inhibits human telomerase. See Kerwin, S. M., et al., Bioorg. Med. Chem. Lett., 11, 2411-2414 (2001).

Additionally, Randazzo et al. have investigated the interaction of distamycin with a four-stranded intermolecular quadruplex. See Randazzo, A., et al., Nucleosides, Nucleotides, and Nucleic Acids, 21, 535-545 (2002). NMR results suggest that distamycin binds in one quadruplex groove as a dimer, but is capable of binding to two grooves at higher concentrations. Distamycin, however, appears to have higher affinity for duplex DNA than for quadruplex DNA. Further, NMR studies by Maizels and coworkers have shown with NMR that distamycin binds *via* an end-stacking mechanism to several four-stranded intermolecular quadruplexes, including one formed from the human telomeric sequence. See Cocco, M. J., et al., Nuc. Acids Res., 31, 2944-2951 (2003). The structures of DODC, DTC, and distamycin A are shown below in Scheme 4.

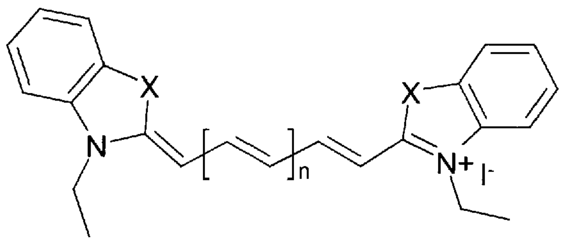

**DODC**: X = O, n = 1
**DTC**: X = S, n = 0

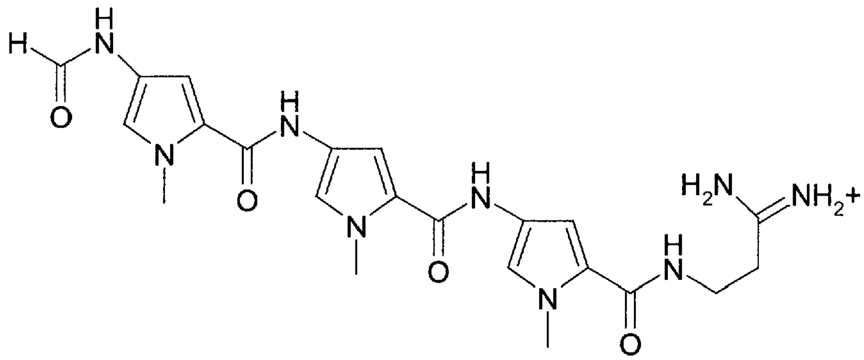

**Distamycin A**

**Scheme 4**. Reported Quadruplex Groove Binding Compounds

III. Dicationic Polyaryl Quadruplex Binding Compounds

III.A. Compounds of Formulas (I-IV)

Disclosed herein is a class of dicationic polyaryl compounds that bind to G-quadruplex DNA. In some embodiments, the compounds selectively bind to G-quadruplex DNA as compared to duplex or triplex DNA. In some embodiments, the dicationic polyaryl compound that binds to G-quadruplex DNA is a compound having the structure of Formula (I):

$$Am_1Ar_1\text{-}Ar_2\text{-}Ar_3\text{-}(Ar_4)_m\text{-}Am_2 \qquad (I)$$

wherein:

m is an integer from 0 to 1;
$Ar_1$, $Ar_2$, $Ar_3$, and $Ar_4$ are independently selected from the group consisting of:

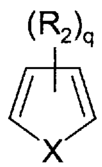 , 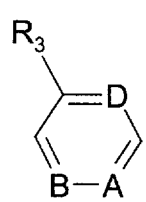 , 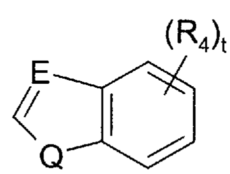 ,

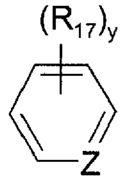, and 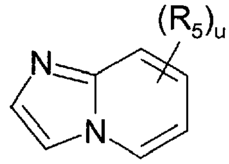,

wherein:

each X is independently selected from the group consisting of O, S, CH, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, aryloxyl, and substituted aryl;
each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;
each E is independently selected from the group consisting of $CR_{18}$ and N, wherein $R_{18}$ is selected from the group consisting of H, halo, hydroxyl, alkyl, alkoxyl, substituted alkyl, cycloalkyl, aryl, aryloxyl, and substituted aryl;
each Z is independently selected from the group consisting of $CR_{19}$ and N, wherein $R_{19}$ is selected from the group consisting of H, halo, hydroxyl, alkyl, alkoxyl, substituted alkyl, cycloalkyl, aryl, aryloxyl, and substituted aryl;
each q is independently an integer from 0 to 2;
each y is independently an integer from 0 to 3;
each t is independently an integer from 0 to 3;
each u is independently an integer from 0 to 3;
each $R_2$, $R_3$, $R_4$, $R_5$, and $R_{17}$ is independently selected from the group consisting of H, alkyl, substituted alkyl, cycloalkyl, halo, hydroxyl, alkoxyl, aryl, aryloxyl, substituted aryl, and aralkyloxyl;

$Am_1$ and $Am_2$ are each independently selected from the group consisting of:

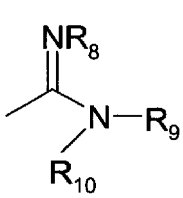; 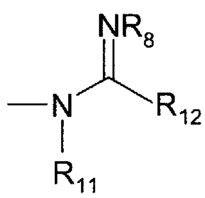; and 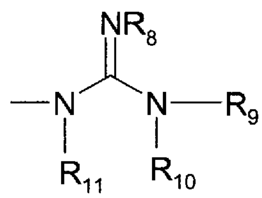;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

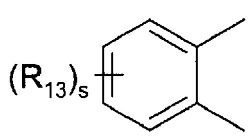;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl.

In compounds of Formula (I) above, $Am_1$ and $Am_2$ can be linked to $Ar_1$, $Ar_3$ or $Ar_4$ through a direct bond to any

available carbon not already substituted with another named substituent. Similarly, each Ar group can be linked to any other Ar group through a direct bond at any available carbon.

In some embodiments, at least one Argroup of the compound of Formula (I) is

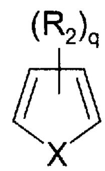,

wherein:

X is selected from the group consisting of O, S, CH, Se, Te, and $NR_1$, wherein $R_1$ is selected from the group consisting of H, alkyl, substituted alkyl, cycloalkyl, aryl, and substituted aryl;
q is an integer from 0 to 2; and
each $R_2$ is independently selected from the group consisting of H, alkyl, substituted alkyl, cycloalkyl, halo, hydroxyl, alkoxyl, aryl, aryloxyl, substituted aryl, and aralkyloxyl.

In some embodiments, $Ar_2$ and $Ar_3$ are each five-membered rings and the compound of Formula (I) has the following formula:

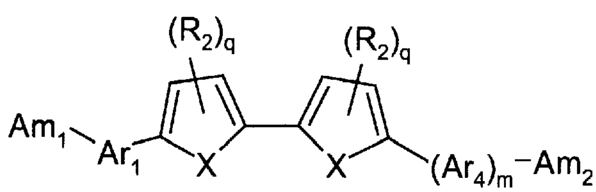 (II)

wherein:

m is an integer from 0 to 1;
$Ar_1$ and $Ar_4$ are independently selected from the group consisting of:

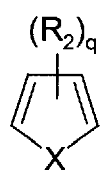, 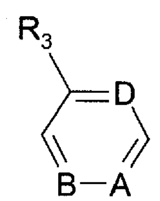, 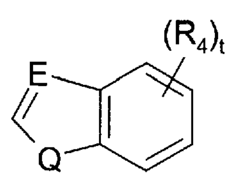,

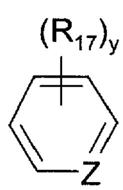, and 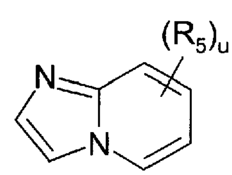,

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl;

each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;
each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;
each E is independently selected from the group consisting of $CR_{18}$ and N; wherein $R_{18}$ is selected from the group consisting of H, halo, hydroxyl, alkyl, alkoxyl, substituted alkyl, cycloalkyl, aryl, aryloxyl, and substituted aryl;
each Z is independently selected from the group consisting of $CR_{19}$ and N; wherein $R_{19}$ is selected from the group consisting of H, halo, hydroxyl, alkyl, alkoxyl, substituted alkyl, cycloalkyl, aryl, aryloxyl, and substituted aryl;
each q is independently an integer from 0 to 2;
each y is independently an integer from 0 to 3;
each t is independently an integer from 0 to 3;
each u is independently an integer from 0 to 3;
each $R_2$, $R_3$, $R_4$, $R_5$ and $R_{17}$ is independently selected from the group consisting of H, alkyl, substituted alkyl, aryl, substituted aryl, halo, hydroxyl, alkoxyl, aryloxyl, and aralkyloxyl;
$Am_1$ and $Am_2$ are each independently selected from the group consisting of:

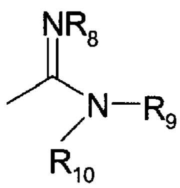
;
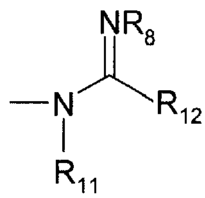
; and
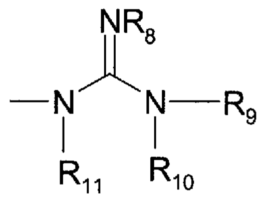
;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

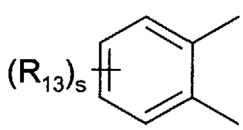
;

wherein s is an integer from 1 to 4, and $R_{13}$ is H or- $CONHR_{14}NR_{15}R_{16}$, wherein $R_{14}$ is alkyl, and $R_{15}$ and $R_{16}$ are each independently selected from the group consisting of H and alkyl.

In some embodiments, $Ar_2$ and $Ar_3$ are each five-membered rings, $Ar_1$ is a six-membered aromatic ring, $Ar_4$, if present, is a five-membered or a six-membered ring, and the compound of Formula (I) has the following formula:

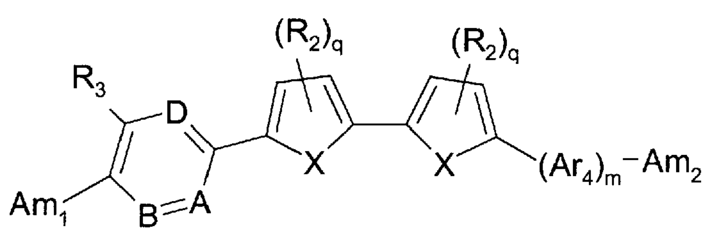
(III)

wherein:

m is selected from 0 and 1;
$Ar_4$ is selected from the group consisting of:

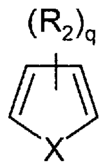 and 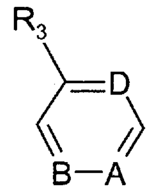 ;

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;
each q is independently an integer from 0 to 2;
each $R_2$ and $R_3$ is independently selected from the group consisting of H, alkyl, substituted alkyl, cycloalkyl, aryl, substituted aryl, halo, hydroxyl, alkoxyl, aryloxyl, and aralkyloxyl;
$Am_1$ and $Am_2$ are each independently selected from the group consisting of:

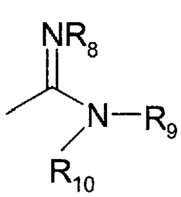 ; 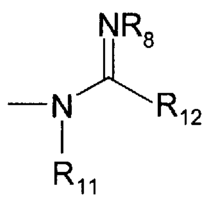 ; and 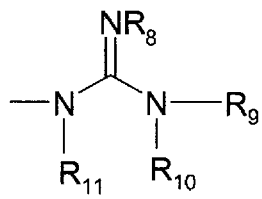 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

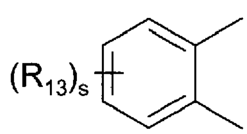 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl.

In some embodiments, m is 0 and $Ar_1$, $Ar_2$, and $Ar_3$ are each five-membered rings and the compound of Formula (I) is a novel compound having the following formula:

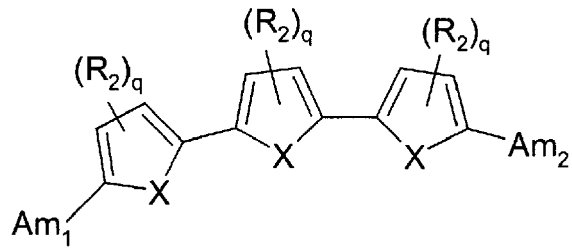

(IV)

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl, provided that at least one X is selected from Se, Te, and $NR_1$, wherein $R_1$ is selected from aryl and substituted aryl;
each q is independently an integer from 0 to 2;
each $R_2$ is independently selected from the group consisting of H, alkyl, substituted alkyl, cycloalkyl, aryl, substituted aryl, halo, hydroxyl, alkoxyl, aryloxyl, and aralkyloxyl;
$Am_1$ and $Am_2$ are each independently selected from the group consisting of:

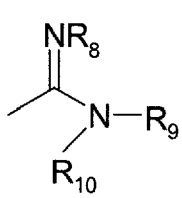 ; 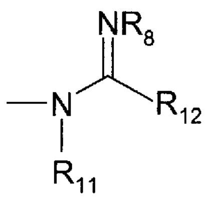 ; and 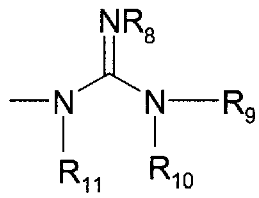 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

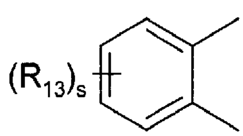 ;

wherein s is an integer from 1 to 4, and $R_{13}$ is H or $-CONHR_{14}NR_{15}R_{16}$ , wherein $R_{14}$ is alkyl, and $R_{15}$ and $R_{16}$ are each independently selected from the group consisting of H and alkyl.

In some embodiments, a compound of Formula (I) has one of the following structures:

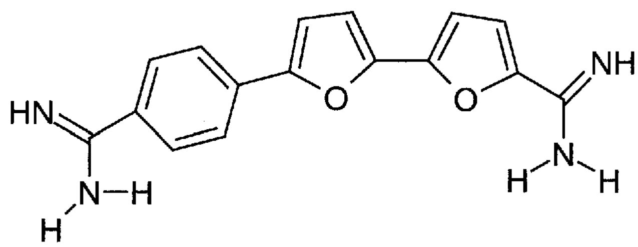

,

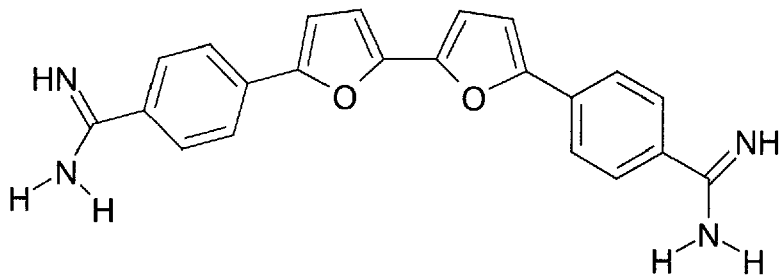

,

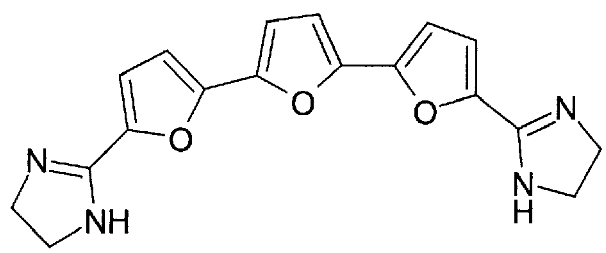

,

and

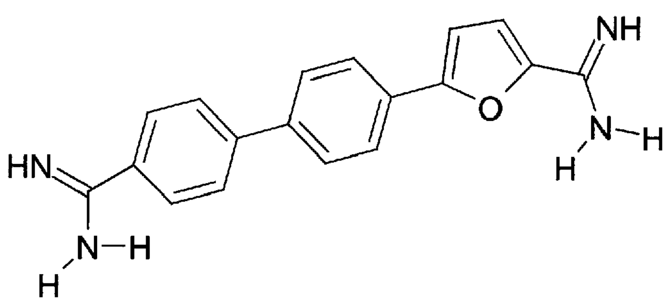

.

In some embodiments, a compound of Formula (I-IV) binds to quadruplex DNA with more affinity that it binds to double-stranded or triple-stranded DNA. Selective binding to quadruplex DNA versus duplex or triplex DNA is believed to reduce the toxicity of the compound to normal (*i.e.*, noncancerous) cells. In some embodiments, a compound of Formula (I-IV) binds selectively to intramolecular G-quadruplexes formed by telomeric DNA. In some embodiments, a compound of Formula (I-IV) selectively binds to G-quadruplexes formed by DNA containing d[$T_2AG_3$] repeat sequences. In some embodiments, a compound of Formula (I-IV) binds selectively to quadruplexes formed by human telomeric DNA sequences. In some embodiments, a compound of Formula (I-IV) binds selectively to nemotodal or protozoan telomeric DNA sequences. Further, in some embodiments, the protozoan telomeric DNA sequence is a sequence from a *Trypanosoma* spp., *Plasmodium* spp., and *Leishmania* spp.

In some embodiments, a compound of Formula (I-IV) selectively binds to a quadruplex telomeric structure, stabilizing the quadruplex form so that telomerase enzyme cannot bind to and/or catalyze the addition of polynucleotides to the telomeric DNA. Thus, in some embodiments, a compound of Formula (I-IV) can be used to reduce or prevent telomeric extension. In some embodiments, a compound of Formula (I-IV) can be used to promote apoptosis in a cell. Thus, in some embodiments, a compound of Formula (I-IV) can be used in a method of reducing cell proliferation.

Further, ellipticity in CD spectral studies described herein below indicate that compounds of Formula (I-IV) can bind in a groove of G-quadruplex DNA. Thus, in some embodiments, a compound of Formula (I-IV) binds to G-quadruplex in a groove of the quadruplex structure. The exciton splitting of the CD spectra of compounds described herein further indicates that the compounds can bind to the quadruplex as dimers. Therefore, in some embodiments, two molecules of a compound of Formula (I-IV) bind as a dimer in a groove of the quadruplex DNA. In some embodiments, the dimer is an offset dimer. Such an offset dimer also can be referred to as a J-aggregate. In some embodiments, dimers of compounds of Formula (I-IV) bind in more than one groove of a G-quadruplex.

III.B. Prodrugs

In representative embodiments, compounds disclosed herein are prodrugs. A prodrug refers to a compound that, upon administration to a recipient, is capable of providing (directly or indirectly) a compound of the presently disclosed subject matter or an inhibitorily active metabolite or residue thereof. Prodrugs can increase the bioavailability of the compounds of the presently disclosed subject matter when such compounds are administered to a subject (e.g., by allowing an orally administered compound to be more readily absorbed into the blood) or can enhance delivery of the parent compound to a biological compartment (e.g., the brain or lymphatic system) relative to a metabolite species, for example. A number of the compounds (e.g., **16**, **19**, **23**, **32**, **39**, **44**, **60a** and **60b**) disclosed herein are prodrugs.

III.C. Pharmaceutically Acceptable Salts

Additionally, the active compounds as described herein can be administered as pharmaceutically acceptable salts. Such pharmaceutically acceptable salts include the gluconate, lactate, acetate, tartarate, citrate, phosphate, maleate, borate, nitrate, sulfate, and hydrochloride salts. The salts of the compounds described herein can be prepared, for example, by reacting the base compound with the desired acid in solution. After the reaction is complete, the salts are crystallized from solution by the addition of an appropriate amount of solvent in which the salt is insoluble. In some embodiments, as described in more detail herein below, the hydrochloride salt of an amidoxime compound is made by passing hydrogen chloride gas into an ethanolic solution of the free base. In some embodiments, as described in more detail herein below, the acetate salt of the presently disclosed diamidine compounds and/or the corresponding *N*-methoxy analogues are made directly from the appropriate *N*-hydroxy analogue. In some embodiments, as described herein below, the maleate salt of the *N*-methoxy analogue of a diamidine compound is prepared by heating the *N*-methoxy analogue with maleic acid in an alcohol for a period of time. Accordingly, in some embodiments, the pharmaceutically acceptable salt is a hydrochloride salt. In some embodiments, the pharmaceutically acceptable salt is an acetate salt. In some embodiments, the pharmaceutically acceptable salt is a maleate salt.

IV. Pharmaceutical Formulations

The compounds of Formula (I-IV), the pharmaceutically acceptable salts thereof, prodrugs corresponding to compounds of Formula (I-IV), and the pharmaceutically acceptable salts thereof, are all referred to herein as "active compounds." Pharmaceutical formulations comprising the aforementioned active compounds also are provided herein. These pharmaceutical formulations comprise active compounds as described herein, in a pharmaceutically acceptable carrier. Pharmaceutical formulations can be prepared for oral, intravenous, or aerosol administration as discussed in greater detail below. Also, the presently disclosed subject matter provides such active compounds that have been lyophilized and that can be reconstituted to form pharmaceutically acceptable formulations for administration, for example, as by intravenous or intramuscular injection.

The therapeutically effective dosage of any specific active compound, the use of which is within the scope of embodiments described herein, will vary somewhat from compound to compound, and patient to patient, and will depend upon the condition of the patient and the route of delivery. As a general proposition, a dosage from about 0.1 to about 50 mg/kg will have therapeutic efficacy, with all weights being calculated based upon the weight of the active compound, including the cases where a salt is employed. Toxicity concerns at the higher level can restrict intravenous dosages to a lower level, such as up to about 10 mg/kg, with all weights being calculated based on the weight of the active base, including the cases where a salt is employed. A dosage from about 10 mg/kg to about 50 mg/kg can be employed for oral administration. Typically, a dosage from about 0.5 mg/kg to 5 mg/kg can be employed for intramuscular injection. Preferred dosages are 1 $\mu$mol/kg to 50 $\mu$mol/kg, and more preferably 22 $\mu$mol/kg and 33 $\mu$mol/kg of the compound for

intravenous or oral administration. The duration of the treatment is usually once per day for a period of two to three weeks or until the condition is essentially controlled. Lower doses given less frequently can be used prophylactically to prevent or reduce the incidence of recurrence of the infection.

In accordance with the presently disclosed methods, pharmaceutically active compounds as described herein can be administered orally as a solid or as a liquid, or can be administered intramuscularly or intravenously as a solution, suspension, or emulsion. Alternatively, the compounds or salts also can be administered by inhalation, intravenously, or intramuscularly as a liposomal suspension. When administered through inhalation the active compound or salt should be in the form of a plurality of solid particles or droplets having a particle size from about 0.5 to about 5 microns, and preferably from about 1 to about 2 microns.

Pharmaceutical formulations suitable for intravenous or intramuscular injection are further embodiments provided herein. The pharmaceutical formulations comprise a compound of Formula (I-IV) described herein, a prodrug as described herein, or a pharmaceutically acceptable salt thereof, in any pharmaceutically acceptable carrier. If a solution is desired, water is the carrier of choice with respect to water-soluble compounds or salts. With respect to the water-soluble compounds or salts, an organic vehicle, such as glycerol, propylene glycol, polyethylene glycol, or mixtures thereof, can be suitable. In the latter instance, the organic vehicle can contain a substantial amount of water. The solution in either instance can then be sterilized in a suitable manner known to those in the art, and typically by filtration through a 0.22-micron filter. Subsequent to sterilization, the solution can be dispensed into appropriate receptacles, such as depyrogenated glass vials. The dispensing is preferably done by an aseptic method. Sterilized closures can then be placed on the vials and, if desired, the vial contents can be lyophilized.

In addition to compounds of Formula (I-IV) or their salts or prodrugs, the pharmaceutical formulations can contain other additives, such as pH-adjusting additives. In particular, useful pH-adjusting agents include acids, such as hydrochloric acid, bases or buffers, such as sodium lactate, sodium acetate, sodium phosphate, sodium citrate, sodium borate, or sodium gluconate. Further, the formulations can contain antimicrobial preservatives. Useful antimicrobial preservatives include methylparaben, propylparaben, and benzyl alcohol. The antimicrobial preservative is typically employed when the formulation is placed in a vial designed for multi-dose use. The pharmaceutical formulations described herein can be lyophilized using techniques well known in the art.

In yet another embodiment of the subject matter described herein, there is provided an injectable, stable, sterile formulation comprising a compound of Formula (I-IV), or a salt thereof, in a unit dosage form in a sealed container. The compound or salt is provided in the form of a lyophilizate, which is capable of being reconstituted with a suitable pharmaceutically acceptable carrier to form a liquid formulation suitable for injection thereof into a subject. The unit dosage form typically comprises from about 10 mg to about 10 grams of the compound salt. When the compound or salt is substantially water-insoluble, a sufficient amount of emulsifying agent, which is physiologically acceptable, can be employed in sufficient quantity to emulsify the compound or salt in an aqueous carrier. One such useful emulsifying agent is phosphatidyl choline.

Other pharmaceutical formulations can be prepared from the water-insoluble compounds disclosed herein, or salts thereof, such as aqueous base emulsions. In such an instance, the formulation will contain a sufficient amount of pharmaceutically acceptable emulsifying agent to emulsify the desired amount of the compound or salt thereof. Particularly useful emulsifying agents include phosphatidyl cholines and lecithin.

Additional embodiments provided herein include liposomal formulations of the active compounds disclosed herein. The technology for forming liposomal suspensions is well known in the art. When the compound is an aqueous-soluble salt, using conventional liposome technology, the same can be incorporated into lipid vesicles. In such an instance, due to the water solubility of the active compound, the active compound will be substantially entrained within the hydrophilic center or core of the liposomes. The lipid layer employed can be of any conventional composition and can either contain cholesterol or can be cholesterol-free. When the active compound of interest is water-insoluble, again employing conventional liposome formation technology, the salt can be substantially entrained within the hydrophobic lipid bilayer that forms the structure of the liposome. In either instance, the liposomes that are produced can be reduced in size, as through the use of standard sonication and homogenization techniques.

The liposomal formulations comprising the active compounds disclosed herein can be lyophilized to produce a lyophilizate, which can be reconstituted with a pharmaceutically acceptable carrier, such as water, to regenerate a liposomal suspension.

Pharmaceutical formulations also are provided which are suitable for administration as an aerosol by inhalation. These formulations comprise a solution or suspension of a desired compound described herein or a salt thereof, or a plurality of solid particles of the compound or salt. The desired formulation can be placed in a small chamber and nebulized. Nebulization can be accomplished by compressed air or by ultrasonic energy to form a plurality of liquid droplets or solid particles comprising the compounds or salts. The liquid droplets or solid particles should have a particle size in the range of about 0.5 to about 10 microns, more preferably from about 0.5 to about 5 microns. The solid particles can be obtained by processing the solid compound or a salt thereof, in any appropriate manner known in the art, such as by micronization. Most preferably, the size of the solid particles or droplets will be from about 1 to about 2 microns.

In this respect, commercial nebulizers are available to achieve this purpose. The compounds can be administered via an aerosol suspension of respirable particles in a manner set forth in U.S. Patent No. 5,628,984, the disclosure of which is incorporated herein by reference in its entirety.

When the pharmaceutical formulation suitable for administration as an aerosol is in the form of a liquid, the formulation will comprise a water-soluble active compound in a carrier that comprises water. A surfactant can be present, which lowers the surface tension of the formulation sufficiently to result in the formation of droplets within the desired size range when subjected to nebulization.

As indicated, both water-soluble and water-insoluble active compounds are provided. As used herein, the term "water-soluble" is meant to define any composition that is soluble in water in an amount of about 50 mg/mL, or greater. Also, as used herein, the term "water-insoluble" is meant to define any composition that has a solubility in water of less than about 20 mg/mL. In some embodiments, water-soluble compounds or salts can be desirable whereas in other embodiments water-insoluble compounds or salts likewise can be desirable.

## V. Methods for Treating Diseases with G-Quadruplex Binding Compounds

### V.A. Methods of Treating Cancer and Other Diseases Involving Telomerase Activity in Mammals

The presently disclosed subject matter provides methods and compositions for inhibiting cell proliferation. Further, the presently disclosed subject matter provides methods of interfering with telomerase activity by binding, optionally selectively binding, the quadruplex form of telomeric DNA in a cell and thereby disrupting telomeric extension. By disrupting telomeric extension during one or more cell divisions, proliferation of the cell ceases and apoptosis is triggered. Thus, the presently disclosed subject matter provides a method of treating diseases involving undesirable telomerase activity.

In some embodiments, the methods for inhibiting cell proliferation comprise administering to a subject in need thereof an active compound as described herein. These active compounds, as set forth above, include compounds of Formula (I-IV), their corresponding prodrugs, and pharmaceutically acceptable salts of the compounds and prodrugs.

In some embodiments, the presently disclosed subject matter provides methods and compositions for disrupting the activity of telomerase enzyme, inhibiting proliferation of telomerase positive cells and for treating cancer in a subject. The methods and compositions of the presently disclosed subject matter also can be used for the treatment of other telomerase mediated conditions or diseases, such as, for example, other hyperproliferative or autoimmune disorders such as psoriasis, rheumatoid arthritis, and other immune system disorders requiring immune system suppression. Because telomerase is only active in tumor, germline, and certain stem cells of the hematopoietic system, other normal cells should not be affected by telomerase interference. If desired, steps can be taken to avoid contact of G-quadruplex binding compounds with germline or stem cells. For example, the G-quadruplex binding compound can be delivered regionally to a particular affected region or regions of the subject's body. Alternatively, systemic delivery of the agent can be more appropriate in certain circumstances, for example, where extensive metastasis has occurred.

The G-quadruplex binding compounds described herein can provide therapy for a wide variety of tumors and cancers including skin cancers, connective tissue cancers, adipose cancers, breast cancers, lung cancers, stomach cancers, pancreatic cancers, ovarian cancers, cervical cancers, uterine cancers, anogenital cancers, kidney cancers, bladder cancers, colon cancers, prostate cancers, central nervous system (CNS) cancers, retinal cancer, blood, and lymphoid cancers.

An "effective amount" is defined herein in relation to the treatment of cancers is an amount that will decrease, reduce, inhibit, or otherwise abrogate the growth of a cancer cell or tumor.

In addition, it will be appreciated that therapeutic benefits for the treatment of cancer can be realized by combining treatment with a G-quadruplex binding compound of the presently disclosed subject matter with one or more additional anti-cancer agents or treatments. The choice of such combinations will depend on various factors including, but not limited to, the type of disease, the age and general health of the subject, the aggressiveness of disease progression, and the ability of the subject to tolerate the agents that comprise the combination. For example, in cases where tumor progression has reached an advanced state, the G-quadruplex binding agent can be combined with other agents and therapeutic regimens that are effective at reducing tumor size (e.g., radiation, surgery, chemotherapy, hormonal treatments, and or gene therapy). Further, in some embodiments, it can be desirable to combine the G-quadruplex binding agent with one or more agents that treat the side effects of a disease or the side effects of one of the therapeutic agents, e.g., providing the subject with an analgesic, or agents effective to stimulate the patient's own immune response (e.g., colony stimulating factor).

Thus, a variety of chemical compounds, also described as "antineoplastic" agents or "chemotherapeutic agents" can be used in combination with one or more of the G-quadruplex compounds of the presently described subject matter. Such compounds include, but are not limited to, alkylating agents, DNA intercalators, protein synthesis inhibitors, inhibitors of DNA or RNA synthesis, DNA base analogs, topoisomerase inhibitors, anti-angiogenesis agents, and other

telomerase inhibitors or telomeric DNA binding compounds. For example, suitable alkylating agents include alkyl sulfonates, such as busulfan, improsulfan, and piposulfan; aziridines, such as a benzodizepa, carboquone, meturedepa, and uredepa; ethylenimines and methylmelamines, such as altretamine, triethylenemelamine, triethylenephosphoramide, triethylenethiophosphoramide, and trimethylolmelamine; nitrogen mustards such as chlorambucil, chlornaphazine, cyclophosphamide, estramustine, iphosphamide, mechlorethamine, mechlorethamine oxide hydrochloride, melphalan, novembichine, phenesterine, prednimustine, trofosfamide, and uracil mustard; nitroso ureas, such as carmustine, chlorozotocin, fotemustine, lomustine, nimustine, and ranimustine.

Antibiotics used in the treatment of cancer include dactinomycin, daunorubicin, doxorubicin, idarubicin, bleomycin sulfate, mytomycin, plicamycin, and streptozocin. Chemotherapeutic antimetabolites include mercaptopurine, thioguanine, cladribine, fludarabine phosphate, fluorouracil (5-FU), floxuridine, cytarabine, pentostatin, methotrexate, and azathioprine, acyclovir, adenine β-1-D-arabinoside, amethopterin, aminopterin, 2-aminopurine, aphidicolin, 8-azaguanine, azaserine, 6-azauracil, 2'-azido-2'-deoxynucleosides, 5-bromodeoxycytidine, cytosine β-1-D-arabinoside, diazooxynorleucine, dideoxynucleosides, 5-fluorodeoxycytidine, 5-fluorodeoxyuridine, and hydroxyurea.

Chemotherapeutic protein synthesis inhibitors include abrin, aurintricarboxylic acid, chloramphenicol, colicin E3, cycloheximide, diphtheria toxin, edeine A, emetine, erythromycin, ethionine, fluoride, 5-fluorotryptophan, fusidic acid, guanylyl methylene diphosphonate and guanylyl imidodiphosphate, kanamycin, kasugamycin, kirromycin, and O-methyl threonine. Additional protein synthesis inhibitors include modeccin, neomycin, norvaline, pactamycin, paromomycine, puromycin, ricin, shiga toxin, showdomycin, sparsomycin, spectinomycin, streptomycin, tetracycline, thiostrepton, and trimethoprim. Inhibitors of DNA synthesis, including alkylating agents such as dimethyl sulfate, mitomycin C, nitrogen and sulfur mustards, intercalating agents, such as acridine dyes, actinomycins, adriamycin, anthracenes, benzopyrene, ethidium bromide, propidium diiodide-intertwining, and agents, such as distamycin and netropsin, also can be combined with compounds of the present invention in pharmaceutical compositions. Topoisomerase inhibitors, such as coumermycin, nalidixic acid, novobiocin, and oxolinic acid, inhibitors of cell division, including colcemide, colchicine, vinblastine, and vincristine; and RNA synthesis inhibitors including actinomycin D, α-amanitine and other fungal amatoxins, cordycepin (3'-deoxyadenosine), dichlororibofuranosyl benzimidazole, rifampicine, streptovaricin, and streptolydigin also can be combined with the G-quadruplex binding compounds of the presently disclosed subject matter to provide a suitable cancer treatment.

Thus, current chemotherapeutic agents that can be used in a combination treatment with a G-quadruplex binding agent of the presently disclosed subject matter include, adrimycin, 5-fluorouracil (5FU), etoposide, camptothecin, actinomycin-D, mitomycin, cisplatin, hydrogen peroxide, carboplatin, procarbazine, mechlorethamine, cyclophosphamide, ifosfamide, melphalan, chjlorambucil, bisulfan, nitrosurea, dactinomycin, duanorubicin, doxorubicin, bleomycin, plicomycin, tamoxifen, taxol, transplatimun, vinblastin, and methotrexate, and the like.

Combination treatments involving a G-quadruplex binding agent and another therapeutic agent, such as another chemotherapeutic agent can be achieved by contacting cells with the G-quadruplex binding agent and the other agent at the same time. Such combination treatments can be achieved by contacting the cell with a single composition or pharmacological formulation that includes both agents, or by contacting the cell with two distinct compositions or formulations, at the same time, wherein one composition includes the G-quadruplex binding agent and the other includes the other agent.

Alternatively, the G-quadruplex binding agent can precede or follow treatment with the other agent by intervals ranging from minutes to weeks. In embodiments where the other agent and the G-quadruplex-based therapy are applied separately to the cell, one would generally ensure that a significant period of time did not expire between the time of each delivery, such that the agent and the G-quadruplex-based treatment would still be able to exert an advantageously combined effect on the cell. In such instances, it is provided that one would contact the cell with both modalities within about 12-24 hours of each other and, optionally, within about 6-12 hours of each other. In some situations, it can be desirable to extend the time period for treatment significantly, however, where several days (2, 3, 4, 5, 6 or 7) to several weeks (1, 2, 3, 4, 5, 6, 7 or 8) lapse between the respective administrations. Also, under some circumstances, more than one administration of either the telomerase-based treatment or of the other agent will be desired.

In another embodiment, a G-quadruplex binding compound of the presently disclosed subject matter is either combined with or covalently bound to a cytotoxic agent bound to a targeting agent, such as a monoclonal antibody (*e.g.*, a murine or humanized monoclonal antibody). It will be appreciated that the latter combination can allow the introduction of cytotoxic agents into cancer cells with greater specificity. Thus, the active form of the cytotoxic agent (*i.e.,* the free form) will be present only in cells targeted by the antibody.

Additional cancer treatments also can be used in combination with administration of a G-quadruplex binding compound. For example, a G-quadruplex binding compound of the presently disclosed subject matter can be used as part of a treatment course further involving attempts to surgically remove part or all of a cancerous growth. For instance, a G-quadruplex binding agent of the presently disclosed subject matter can be administered after surgical treatment of a subject to treat any remaining neoplastic or metastasized cells. Treatment with a G-quadruplex binding agent of the presently disclosed subject matter also can precede surgery, in an effort to shrink the size of a tumor to reduce the

amount of tissue to be excised, thereby making the surgery less invasive and traumatic.

Treating cancer with a G-quadruplex binding agent of the presently disclosed subject matter can further include one or more treatment courses with a radiotherapeutic agent to induce DNA damage. Radiotherapeutic agents, include, for example, gamma irradiation, X-rays, UV-irradiation, microwaves, electronic emissions, radioisotopes, and the like. Therapy can be achieved by irradiating the localized tumor site with the above-described forms of radiation.

A combination therapy also can involve immunotherapy directed at tumor antigen markers that are found on the surface of tumor cells. Treatment of a cancer with a G-quadruplex binding agent of the presently disclosed subject matter can further be combined with a gene therapy based treatment, targeted towards oncogenes and/or cell cycle controlling genes, such as p53, p16, p21, Rb, APC, DCC, NF-1, NF-2, BRCA2, FHIT, WT-1, MEN-I, MEN-II, BRCA1, VHL, FCC, MCC, ras, myc, neu, raf, erb, src, fms, jun, trk, ret, gsp, hst, bcl, and abl, which are often mutated versions of their normal cellular couterparts in cancerous tissues.

The G-quadruplex binding compounds of the presently disclosed subject matter can be tested to measure their ability to inhibit growth of cancer cells, to induce cytotoxic events in cancer cells, to induce apoptosis of the cancer cells, to reduce tumor burden, and to inhibit metastases. For example, one can measure cell growth according to the MTT assay. Growth assays as measured by the MTT assay are well known in the art. In the MTT assay, cells (*e.g.*, telomerase-positive cells) are incubated with various concentrations of anti-cancer compound, and cell viability is determined by monitoring the formation of a colored formazan salt of the tetrazolium salt, 3-(4,5-dimethylthiazol-2-yl)-2,5-diphenyltetrazolium bromide (MTT). Other known assays for measuring cell death also can be employed.

*In vivo* testing can be performed using a mouse xenograft model, for example, in which OVCAR-5 tumor cells are grafted onto nude mice, in which mice treated with a compound of Formula (I-IV) are expected to have tumor masses that, on average, increase for a period following initial dosing, but will shrink in mass with continuing treatment. In contrast, mice treated with a control (*e.g.*, DMSO) are expected to have tumor masses that continue to increase.

Additionally, the *in vitro* or *in vivo* activity of a compound of Formula (I-IV) can be measured by ascertaining changes in telomeric sequence length. For example, treatment of telomerase-positive cell lines, such as HEK-293 and HeLa cells, with a compound of Formula (I-IV) is expected to induce a reduction of telomere length. Compounds of Formula (I-IV) also are expected to induce telomere reduction during cell division in human tumor cell lines, such as the ovarian tumor cell lines OVCAR-5 and SK-OV-3. Importantly, however, in normal human cells, such as BJ cells of fibroblast origin, the observed reduction in telomere length is expected to be no different from cells treated with a control substance, *e.g.*, dimethyl sulfoxide (DMSO).

Terminal restriction fragment (TRF) analysis can be used to determine telomere length. In TRF, DNA from tumor cells is analyzed by digestion with restriction enzymes specific for sequences other than the telomeric $T_2AG_3$ sequence. Following digestion of the DNA, gel electrophoresis is performed to separate the restriction fragments according to size. The separated fragments are then probed with nucleic acid probes specific for telomeric sequences to determine the lengths of the terminal fragments containing telomere DNA of the cells in the sample. By measuring the length of telomeric DNA in cells taken from a subject during treatment, one also can estimate how long a telomerase inhibitor should be administered and whether other methods of therapy (*e.g.*, surgery, chemotherapy and/or radiation) should also be employed.

V.B. Methods for Treating Infections

The compositions and methods of the presently disclosed subject matter can be used to treat to diseases caused by other organisms displaying telomerase activity. These organisms include agricultural phytopathogenic organisms including nematodes, such as *Ceanorhabditis elegans,* in which telomerase activity has been found, and fungi, which are believed to have telomerase activity based on the determination that the DNA of the fungus *Ustilago maydis* exhibits telomeres having tandem $T_2AG_3$ repeats maintained by telomerase. Thus, the quadruplex-binding compounds described herein can be administered to plants and soil infected with phytopathogenic organisms having telomerase activity alone or in combination with other agents to control plant diseases. Also, as discussed hereinabove, protozoans have $T_2AG_3$ telomeres and cause human disease.

The determination that nematodes, protozoans, and possibly fungi have telomerase activity also indicates that the G-quadruplex-binding compounds provided by the presently disclosed subject matter can be used to treat nematode infections in humans and animals of veterinary interest, such as dogs and cats. Nematode infection in humans and animals often is in the form of hookworm or roundworm infection and leads to a host of deadly secondary illnesses such as meningitis, myocarditis, and various neurological diseases. Thus, it will be appreciated that administration of quadruplex binding compounds, either alone, or in combination with a telomerase inhibiting compound and/or another therapeutic compound, can be used to control nematode, protozoan, and fungal infections in humans and animals.

Exemplary protozoan infections that can be treated by the method of the presently disclosed subject matter include, but are not limited to, infections caused by *Trypanosoma* spp. (e.g., *Trypanosoma brucei rhodesiense, Trypanosoma brucei gambiense, Trypanosoma brucei brucei,* and *Trypanosoma cruzi*), *Plasmodium* spp. (e.g., *Plasmodium*

*falciparum*), *Leishmania donovani,* and *Leishmania mexicana amazonensis.* As used herein the terms *Trypanosoma* spp., *Plasmodium* spp., and *Leishmania* spp. encompass microbes classified under the genera *Trypanosoma, Plasmodium,* and *Leishmania,* respectively. The methods of the presently disclosed subject matter are useful for treating these conditions in that they inhibit the onset, growth, or spread of the condition, cause regression of the condition, cure the condition, or otherwise improve the general well-being of a subject afflicted with, or at risk of, contracting the condition. Thus, in accordance with the presently disclosed subject matter, the terms "treat," "treating," and grammatical variations thereof, as well as the phrase "method of treating," are meant to encompass any desired therapeutic intervention, including but not limited to a method for treating an existing infection in a subject, and a method for the prophylaxis (i.e., preventing) of infection, such as in a subject that has been exposed to a microbe as disclosed herein or that has an expectation of being exposed to a microbe as disclosed herein.

The methods for treating infections comprise administering to a subject in need thereof an active compound as described herein. These active compounds, as set forth above, include compounds of Formula (I-IV), their corresponding prodrugs, and pharmaceutically acceptable salts of the compounds and prodrugs.

In some embodiments, the compound of Formula (I-IV) is administered in the form of a pharmaceutically acceptable salt. In some embodiments, the pharmaceutically acceptable salt comprises a hydrochloride salt. In some embodiments, the pharmaceutically acceptable salt comprises an acetate salt. In some embodiments, the pharmaceutically acceptable salt comprises a maleate salt.

In some embodiments, the compound of Formula (I-IV) is administered to a subject with an existing microbial infection. In some embodiments, the compound of Formula (I-IV) is administered prophylactically to prevent a microbial infection or to prevent the recurrence of a microbial infection. Thus, in some embodiments, the compound of Formula (I-IV) is administered prophylactically to prevent or reduce the incidence of one of: (a) a microbial infection in a subject at risk of infection; (b) a recurrence of the microbial infection; and (c) combinations thereof.

In some embodiments, the subject is a plant or a soil sample. In particular, the plant can be a plant cultivated for food or having some economic importance. Thus, the plant can be a vegetable, fruit or grain. Such plants can include, but are not limited to, corn, wheat, soybeans, sunflower, oats, alfalfa, tomato, potato, and rice. The soil sample can include a soil sample in which such a plant is being grown or in which such a plant is intended to be grown.

In some embodiments, the subject treated in the presently disclosed subject matter is desirably a human subject, although it is to be understood the methods described herein are effective with respect to all vertebrate species, which are intended to be included in the term "subject." The methods described herein are particularly useful in the treatment and/or prevention of infectious diseases in warm-blooded vertebrates. Thus, the methods can be used as treatment for mammals and birds.

More particularly, provided herein is the treatment of mammals, such as humans, as well as those mammals of importance due to being endangered (such as Siberian tigers), of economical importance (animals raised on farms for consumption by humans) and/or social importance (animals kept as pets or in zoos) to humans, for instance, carnivores other than humans (such as cats and dogs), swine (pigs, hogs, and wild boars), ruminants (such as cattle, oxen, sheep, giraffes, deer, goats, bison, and camels), and horses. Also provided herein is the treatment of birds, including the treatment of those kinds of birds that are endangered, kept in zoos, as well as fowl, and more particularly domesticated fowl, i.e., poultry, such as turkeys, chickens, ducks, geese, guinea fowl, and the like, as they also are of economical importance to humans. Thus, embodiments of the methods described herein include the treatment of livestock, including, but not limited to, domesticated swine (pigs and hogs), ruminants, horses, poultry, and the like.

## VI. General Processes for the Synthesis of Compounds of Formula (IV)

The synthetic procedures provided herein below comprise representative embodiments of methods of producing the presently disclosed compounds. In particular, methods are outlined in Scheme 15 presented herein below for the synthesis of novel compounds of Formula (IV).

In some embodiments, the presently disclosed subject matter provides a method for preparing a compound of Formula (IV) and pharmaceutically acceptable salts thereof, the method comprising:

(a) contacting one of selenophene and tellurophene with *N*-bromosuccinimide (NBS) to form a mono-bromide;
(b) contacting one of selenophene and tellurophene with *n*-butyl lithium and then with a trialkyltin chloride to form a 2,5-bis(trialkyltin) compound;
(c) contacting the 2,5-bis(trialkyltin) compound with two molar equivalents of the mono-bromide in the presence of a palladium catalyst to form a tri-aryl compound;
(d) contacting the tri-aryl compound with NBS to form a di-bromide;
(e) contacting the di-bromide with copper cyanide to from a di-nitrile;
(f) contacting the di-nitrile with one of:

(i) hydroxylamine hydrochloride and a base to form a bis-amidoxime of Formula (IV); and
(ii) a strong acid and an alcohol, followed by a diamine to form a bis-imidazole compound of Formula (IV).

In some embodiments, the method described immediately hereinabove further comprises contacting the bis-amidoxime with acetic acid and acetic anhydride followed by hydrogen, a palladium on carbon catalyst, and a protic solvent to form a bis-amidine of Formula (IV).

In some embodiments, the trialkyltin chloride comprises tri-n-butyltin chloride.

In some embodiments, the base comprises potassium *tert*-butoxide.

In some embodiments, the strong acid comprises hydrochloric acid.

In some embodiments, the alcohol comprises an alkyl alcohol. In some embodiments, the alkyl alcohol is selected from the group consisting of ethanol and methanol.

In some embodiments, the palladium catalyst comprises tetrakis(triphenylphosphine)palladium.

The methods described immediately hereinabove thus provide tri-selenophene and tri-tellurophene compounds of Formula (IV), as well as compounds of Formula (IV) comprising both tellurophene and selenophene rings. The methods also can be modified such that one or more furan, thiophene or pyrrole compound can be substituted for one or two of the selenophene or tellurophene rings to form compounds of Formula (IV) containing different combinations of heteroaryl groups. For example, a 2,5-bis(trialkyltin)selenophene can be contacted with two molar equivalents of a bromofuran to form a tri-aryl structure comprising a 2,5-bis(furanyl)selenophene.

EXAMPLES

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed without departing from the scope of the presently disclosed subject matter.

Synthetic Methods and Materials

Melting points were recorded using a Thomas-Hoover (Uni-Melt) capillary melting point apparatus and are uncorrected. TLC analysis was carried out on silica gel 60 $F_{254}$ precoated aluminum sheets and detected under UV light. $^{1}H$ and $^{13}C$ NMR spectra were recorded employing a Varian Unity Plus 300 spectrometer, and chemical shifts (δ) are in ppm relative to TMS as internal standard. Mass spectra were recorded on a VG analytical 70-SE spectrometer. Elemental analyses were obtained from Atlantic Microlab Inc. (Norcross, Georgia, United States of America) or the GSU CHN unit and are within $\pm 0.4$ of the theoretical values. All chemicals and solvents were purchased from Aldrich Chemical Co., Fisher Scientific.

The synthesis of furamidine has been previously described. See Das, B. P., and Boykin, D. W., J. Med. Chem., 20, 531-536 (1977). Synthetic methods useful in preparing compounds of the presently disclosed subject matter, including the specific syntheses of compounds **46-48**, **50-51**, and **53** also have been previously described. See Czarny, A., et al., J. Heterocycl. Chem., 33, 1393-1397 (1996); Hopkins, K. T., et al., J. Med. Chem., 41, 3872-3878 (1998); Lansiaux, A., et al., J. Med. Chem., 45, 1994-2002 (2002); U. S. Published Patent Application No. 20050148646 to Boykin, D. W., et al; and published PCT application WO 2005/086808 to Boykin, D. W., et al. Methods for the synthesis of trifuran and trithiophene compounds similar to compound **49** have been described. See Bilik, P., et al., CHEMBIOCHEM, 2, 559-569 (2001). The syntheses of additional compounds of the presently disclosed subject matter are described herein below.

Example 1

5'-(4-Amidinophenyl)-2,2'-bifuran-5-carboxamidine

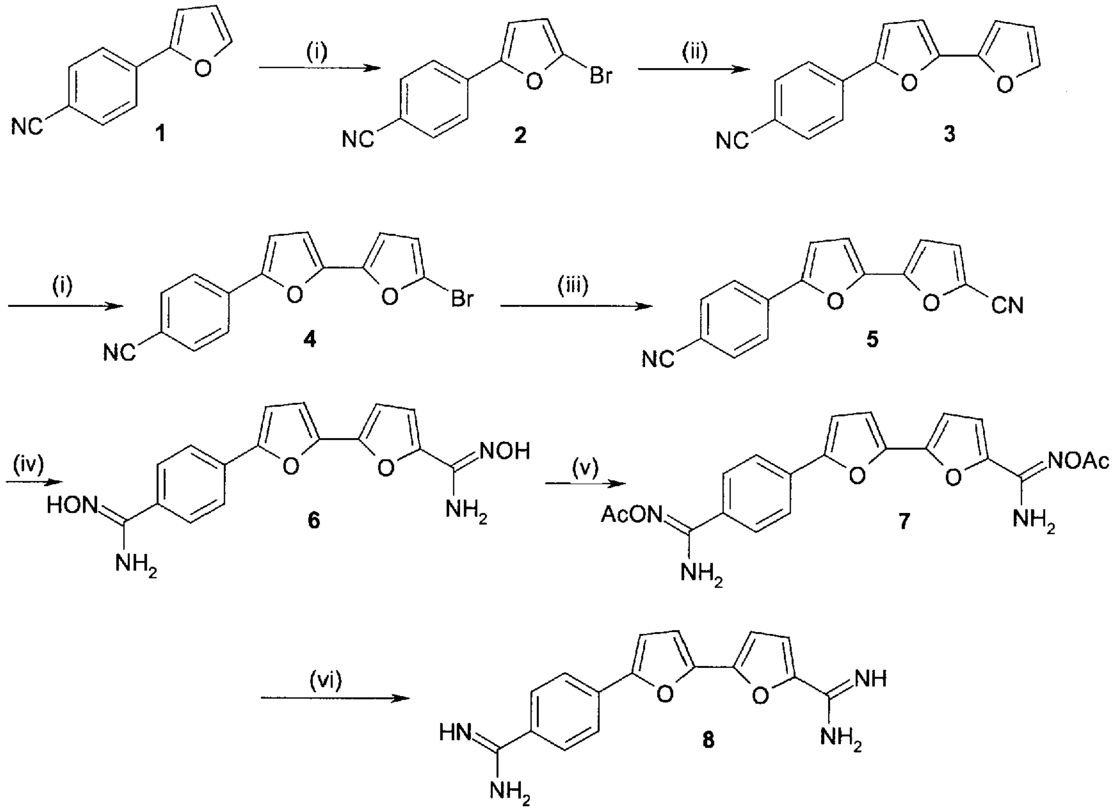


Reagents and conditions: (i) NBS, DMF; (ii) 2-tributyltin furan, $Pd(PPh_3)_4$; (iii) CuCN, DMF, 110-120 °C; (iv) $NH_2OH$•HCl, KO-*t*-Bu, DMSO; (v) AcOH/$Ac_20$; (vi) $H_2$/Pd-C, AcOH.

**Scheme 5.** Synthesis of 5'-(4-Amidinophenyl)-2,2'-bifuran-5-carboxamidine.

**4-(5-Bromofuran-2-yl)-benzonitrile (2)**. Referring now to Scheme 5 above, to a solution of **1** (8.45 g, 50 mmol) in DMF (30 mL) was added portionwise *N*-bromosuccinimide (NBS, 9.79 g, 55 mmol) with stirring. The reaction mixture was stirred overnight, then poured onto cold water. The precipitate which formed was collected, washed with water and dried to give the analytically pure product **2** in 94.2% yield, mp 94-94.5 ˚C. $^1$H NMR ($CDCl_3$); δ 6.45 (d, J = 3.6 Hz, 1H), 6.76 (d, J = 3.6 Hz, 1H), 7.66 (d, J = 8.4 Hz, 2H), 7.70 (d, J = 8.4 Hz, 2H). $^{13}$C NMR ($CDCl_3$); δ 153.7, 133.5, 132.6, 123.8, 123.6, 118.7, 114.0, 110.7, 110.3. EIMS (m/z, rel.int.); 247 ($M^+$, 50), 140 (100), 113 (10). Calcd for $C_{11}H_6BrNO$: C, 53.26; H, 2.44; N, 5.64. Found. C, 53.22; H, 2.43; N, 5.59.

**4-(2,2'-Bifuran-5-yl)-benzonitrile (3).** A mixture of **2** (2.48 g, 10 mmol), 2-tributyltin furan (3.58 g, 10 mmol), and tetrakis(triphenylphosphine) palladium (200 mg) in dry dioxane (60 mL) was heated under nitrogen at reflux (100-110 ˚C) for 24 h. The solvent was evaporated under reduced pressure and the resulting solid was dissolved in ethyl acetate. This solution was passed through celite to remove Pd. The solution was evaporated, and the solid was collected via filtration and washed with hexanes to furnish compound 3 in 79.5 % yield, mp 104-105 ˚C. $^1$H NMR ($CDCl_3$); δ 6.51 (dd, J = 3.6 Hz, J = 1.8 Hz, 1H), 6.68 (m, 2H), 6.87 (d, J = 3.6 Hz, 1H), 7.46 (d, J = 1.8 Hz, 1H), 7.66 (d, J = 8.4 Hz, 2H), 7.77 (d, J = 8.4 Hz, 2H). $^{13}$C NMR ($CDCl_3$); δ 150.9, 147.5, 145.8, 142.4, 134.1, 132.5, 123.7, 118.9, 111.6, 110.1, 107.5,106.3. EIMS (m/z, rel.int.); 235 ($M^+$, 100), 206 (10), 178 (15). Calcd for $C_{15}H_9NO_2$: C, 76.59; H, 3.86; N, 5.95. Found. C, 76.35; H, 3.88; N, 5.92.

**4-(5'-Bromo-2,2'-bifuran-5-yl)-benzonitrile (4)**. The same procedure described for **2** was used starting with **3**. Yield 65%, mp 127 ˚C. $^1$H NMR ($CDCl_3$); δ 6.42 (d, J = 3.6 Hz, 1H), 6.63 (d, J = 3.6 Hz, 1H), 6.68 (d, J = 3.6 Hz, 1H), 6.86 (d, J = 3.6 Hz, 1H), 7.66 (d, J = 8.4 Hz, 2H), 7.76 (d, J = 8.4 Hz, 2H). $^{13}$C NMR ($CDCl_3$); δ 151.2, 147.6, 146.2, 134.0, 132.5, 124.0, 123.8, 122.3, 113.4, 110.4, 110.0, 108.4, 108.1. EIMS (m/z, rel.int.); 314 ($M^+$, 40), 234 (10), 206

(100). Calcd for $C_{15}H_8BrNO_2$: C; 57.32, H; 2.56; N, 4.46. Found. C; 56.93, H; 2.55; N, 4.39.

**5'-(4-Cyanophenyl)-2,2'-bifuran-5-carbonitrile (5).** A mixture of **4** (740 mg, 2.35 mmol) and Cu(I)CN (423 mg, 4.7 mmol) in dry DMF (25 mL) was refluxed for 48 h. The reaction mixture was poured onto water/ammonia and extracted with methylene chloride. The extract was washed with water and brine, dried over $Na_2SO_4$, then passed on silica gel to give analytically pure product **5** in 40% yield, mp 194-195 °C. $^1$H NMR (DMSO-*d$_6$*); δ 7.17 (d, J = 3.6 Hz, 1H), 7.21 (d, J = 3.6 Hz, 1H), 7.43 (d, J = 3.6 Hz, 1H), 7.76 (d, J = 3.6 Hz, 1H), 7.91 (d, J = 8.4 Hz, 2H), 7.99 (d, J = 8.4 Hz, 2H). $^{13}$C NMR (DMSO-*d$_6$*); δ 152.3, 149.3, 144.3, 133.0, 132.9, 125.6, 124.3, 124.1, 118.7, 112.0, 111.8, 111.5, 110.0, 107.9. EIMS (m/z, rel.int.); 260 ($M^+$, 100), 231 (15), 203 (25), 177 (25), 140 (10), 130 (55). Calcd for $C_{16}H_8N_2O_2$: C, 73.84; H, 3.09; N, 10.76. Found. C, 73.62; H, 3.18; N, 10.92.

***N*-Hydroxy-5'-[4-(N-hydroxyamidino)-phenyl]-2,2'-bifuran-5-carboxamidine** (6). A mixture of hydroxylamine hydrochloride (695 mg, 10 mmol, 10 eq.) in anhydrous DMSO (8 mL) was cooled to 5 °C under nitrogen and potassium *t*-butoxide (1120 mg, 10 mmol, 10 eq.) was added in portions. The mixture was stirred for 30 min. This mixture was added to the bis-cyano derivative **5** (260 mg, 1 mmol, 1 eq.). The reaction mixture was stirred overnight at room temperature. The reaction mixture was then poured slowly onto ice water (20 mL water and 20 mL ice). The precipitate was filtered and washed with water and then ethanol to afford **6** (free base) in 93% yield, mp 205-206 °C. $^1$H NMR (DMSO-*d$_6$*); δ 5.83 (br s, 4H), 6.86-6.89 (m, 3H), 7.14 (s, 1H), 7.75 (s, 4H), 9.70 (s, 1H), 9.75 (s, 1H). $^{13}$C NMR (DMSO-*d$_6$*); δ 152.3, 150.3, 146.7, 145.0, 144.9, 144.1, 132.3, 129.9, 125.8, 123.1, 109.7, 108.5, 107.2. EIMS (m/z, rel.int.); 327 ($M^+$+1, 40), 307 (100), 299 (60), 273 (10), 220 (30). High resolution calcd for $C_{16}H_{15}N_4O_4$ ms 327.10933. Observed 327.11373.

***N*-Acetoxy-5'-[4-(*N*-acetoxyamidino)-phenyl]-2,2'-bifuran-5-carboxamidine** (7). To a solution of **6** (262 mg, 0.8 mmol) in glacial acetic acid (8 mL) was slowly added acetic anhydride (0.28 mL). After stirring overnight, TLC indicated complete acylation of the starting material. The reaction mixture was poured onto ice water, the precipitate was filtered, washed with water and dried to give **7** in 89% yield, mp 212-213 °C. $^1$H NMR (DMSO-*d$_6$*); δ 2.17 (s, 6H), 6.85 (br s, 4H),. 6.98 (d, J = 3.6 Hz, 1H), 7.00 (d, J = 3.6 Hz, 1H), 7.14 (d, J = 3.6 Hz, 1H), 7.24 (d, J = 3.6 Hz, 1H), 7.79 (d, J = 8.4 Hz, 2H), 7.87 (d, J = 8.4 Hz, 2H). $^{13}$C NMR (DMSO-d$_6$); δ 168.4, 168.1, 155.8, 152.4, 148.9, 146.1, 144.8, 144.3, 131.2, 130.6, 127.2, 123.3, 113.1, 109.6, 109.3, 107.6, 19.8, 19.7. Calcd for $C_{20}H_{18}N_4O_6$: C, 58.53; H, 4.42. Found. C, 58.71; H, 4.50.

**5'-(4-Amidinophenyl)-2,2'-bifuran-5-carboxamidine acetate salt (8a).** To a solution of 7 (246 mg, 0.6 mmol) in glacial acetic acid (8 mL), and ethanol (20 mL) was added 10% palladium on carbon (60 mg). The mixture was placed in a Parr hydrogenation apparatus at 50 psi for 4 h at room temperature. The mixture was filtered through hyflo and the filter pad washed with water. The filtrate was evaporated under reduced pressure and the precipitate was collected and washed with ether to give **8a** in 67% yield, mp 240-242 °C. $^1$H NMR ($D_2O$/DMSO-*d$_6$*); δ 1.80 (s, 2x$CH_3$), 7.06 (d, J = 3.6 Hz, 1H), 7.11 (d, J = 3.6 Hz, 1H), 7.36 (d, J = 3.6 Hz, 1H), 7.39 (d, J = 3.6 Hz, 1H), 7.89 (d, J = 8.4 Hz, 2H), 7.98 (d, J = 8.4 Hz, 2H). Calcd for $C_{16}H_{14}N_4O_2$-2.0AcOH-2.4$H_2O$: C, 52.48; H, 5.87; N, 12.23. Found. C, 52.28; H, 5.49; N, 11.91.

**5'-(4-Amidinophenyl)-2,2'-bifuran-5-carboxamidine (8)**. The free base of **8a** was prepared by dissolving the acetate salt (50 mg) in water (5 mL) and by neutralization with 1 N NaOH. The precipitate was filtered, dried to afford free amidine **8**, mp 202-203.5 °C. $^1$H NMR (DMSO-*d$_6$*); δ 6.93 (d, J = 3.6 Hz, 1H), 7.01 (d, J = 3.6 Hz, 1H), 7.11 (d, J = 3.6 Hz, 1H), 7.7.22 (d, J = 3.6 Hz, 1H), 7.83 (s, 4H). $^{13}$C NMR (DMSO-*d$_6$*); δ 162.2, 153.9, 152.4, 147.4, 145.7, 145.0, 134.1, 131.1, 127.3, 123.1, 111.9, 109.4, 109.2, 107.7. EIMS (m/z, rel.int.); 294 ($M^+$, 50), 277 (100), 261 (25). High resolution mass calcd. for $C_{16}H_{14}N_4O_2$: 294.11168. Observed: 294.11013.

Example 2

6-(5'-Amidino-2,2'-bifuran-5-yl)-nicotinamidine

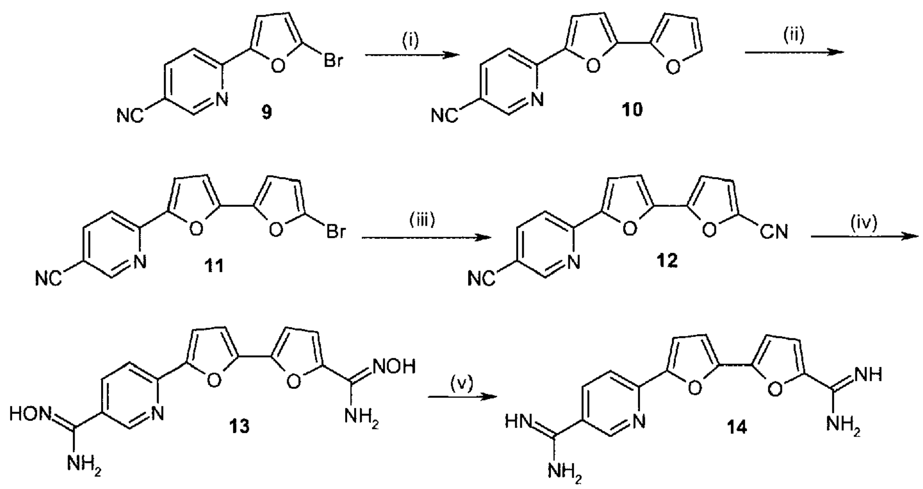


Reagents and conditions: (i) 2-tributyltin furan, $Pd(PPh_3)_4$; (ii) NBS, DMF; (iii) CuCN, DMF 110-120 °C; (iv) $NH_2OH$•HCl, KO-*t*-Bu, DMSO; (v) a) $AcOH/Ac_2O$; b) $H_2$/Pd-C, AcOH.

**Scheme 6.** Synthesis of 6-(5'-Amidino-2,2'-bifuran-5-yl)-nicotinamidine.

**6-(2,2'-Bifuran-5-yl)-nicotinonitrile (10).** Referring now to Scheme 6, the same procedure described for 3 was used starting with **9**. Yield 78 %, mp 169-170 °C. $^1$H NMR ($CDCl_3$); δ 6.52 (dd, J = 3.6 Hz, J = 1.8 Hz, 1H), 6.74 (m, 2H), 7.31 (d, J = 3.6 Hz, 1H), 7.49 (d, J = 1.8 Hz, 1H), 7.79 (d, J = 8.4 Hz, 1H), 7.95 (dd, J = 8.4, 2.1 Hz, 1H), 8.80 (d, J = 2.1 Hz, 1H). $^{13}$C NMR; δ 152.5, 151.3, 151.0, 148.7, 145.5, 142.9, 139.6, 117.6, 117.0, 114.3, 111.7, 108.1, 107.1, 106.6. Calcd for $C_{14}H_8N_2O_2$: C, 71.18; H, 3.41; N, 11.85. Found. C, 70.83; H, 3.61; N, 11.84.

**6-(5'-Bromo-2,2'-bifuran-5-yl)-nicotinonitrile (11).** The same procedure described for **4** was used starting with **10.** Yield 58%, mp 143-145 °C. $^1$H NMR ($CDCl_3$); δ 6.44 (d, J = 3.6 Hz, 1H), 6.70 (d, J = 3.6 Hz, 1H), 6.75 (d, J = 3.6 Hz, 1H), 7.30 (d, J = 3.6 Hz, 1H), 7.79 (d, J = 8.4 Hz, 1H), 7.95 (dd, J = 8.4, 2.1 Hz, 1H), 8.80 (d, J = 2.1 Hz, 1H). $^{13}$C NMR; δ 152.5, 151.2, 151.1, 147.5, 147.3, 139.7, 122.8, 117.7, 117.0, 114.3, 113.5, 109.3, 108.6, 106.9. MS (m/z, rel.int.); 314 ($M^+$, 60), 285 (10), 235 (20), 207 (100), 179 (10). High resolution calcd for $C_{14}H_7BrN_2O_2$ ms 313.96909. Observed 313.96614.

**6-(5'-Cyano-2,2'bifuran-5-yl)-nicotinonitrile (12)**. The same procedure described for **5** was used starting with **11**. Yield 27%, mp 209-210.5 °C. $^1$H NMR (DMSO-$d_6$); δ 7.23 (d, J = 3.6 Hz, 1H), 7.27 (d, J = 3.6 Hz, 1H), 7.52 (d, J = 3.6 Hz, 1H), 7.79 (d, J = 3.6 Hz, 1H), 8.04 (d, J = 8.4 Hz, 1H), 8.39 (dd, J = 8.4, 2.4 Hz, 1H), 9.03 (d, J = 2.4 Hz, 1H). Anal. ($C_{15}H_7N_3O_2$) C, H.

***N*-Hydroxy-6-[5'-(*N*-hydroxyamidino)-2,2'-bifuran-5-yl]-nicotinamidine (13)**. The same procedure described for **6** was used starting with **12**. Yield 89%, mp 248-250 °C. $^1$H NMR (DMSO-*d*$_6$); δ 5.88 (s, 2H), 6.04 (s, 2H), 6.92-6.96 (m, 3H), 7.29 (d, J = 3.6 Hz, 1H), 7.84 (d, J = 8.4 Hz, 1H), 8.11 (dd, J = 8.4, 2.1 Hz, 1H), 8.88 (d, J = 2.1 Hz, 1H), 9.80 (s, 1H), 9.92 (s, 1H). $^{13}$C NMR; δ 152.2, 148.7, 147.8, 147.0, 146.6, 146.0, 144.6, 144.0, 133.6, 127.3, 117.7, 111.3, 109.7, 108.5, 107.9. MS (m/z, rel.int.); 327 ($M^+$, 15), 311 (5), 295 (10), 278 (85), 261 (100). High resolution calcd for $C_{15}H_{13}N_5O_4$ ms 327.09675. Observed 327.09742.

**6-(5'-Amidino-2,2'-bifuran-5-yli)-nicotinamidine acetate salt (14).** Reduced using palladium on carbon analogously to **8a.** Yield 59%, mp 269-271 °C dec. $^1$H NMR (DMSO-*d*$_6$); δ 1.8 (s, 2x$CH_3$), 7.15 (d, J = 3.6 Hz, 1H), 7.23 (d, J = 3.6 Hz, 1H), 7.47 (d, J = 3.6 Hz, 1H), 7.57 (d, J = 3.6 Hz, 1H), 8.04 (d, J = 8.4 Hz, 1H), 8.30 (d, J = 8.4 Hz, 1H), 8.99 (s, 1H). MS (m/z, rel.int. thioglycerol); 296 ($M^+$+1, 100), 273 (12), 239 (40). High resolution calcd for $C_{15}H_{14}N_5O_2$ ms 296.1147. Observed 296.1189. Calcd for $C_{15}H_{13}N_5O_2$-2.0AcOH-2.65$H_2O$-0.5EtOH: C, 49.41; H, 6.07; N, 14.40. Found. C, 49.72; H, 5.96; N, 14.02.

Example 3

5,5'-bis-(4-*N*-Hydroxybenzamidine)-2,2'-bifuran **(16)** and 5,5'-bis-(4-Amidinophenyl)-2,2'-bifuran hydrochloride salt **(17)**

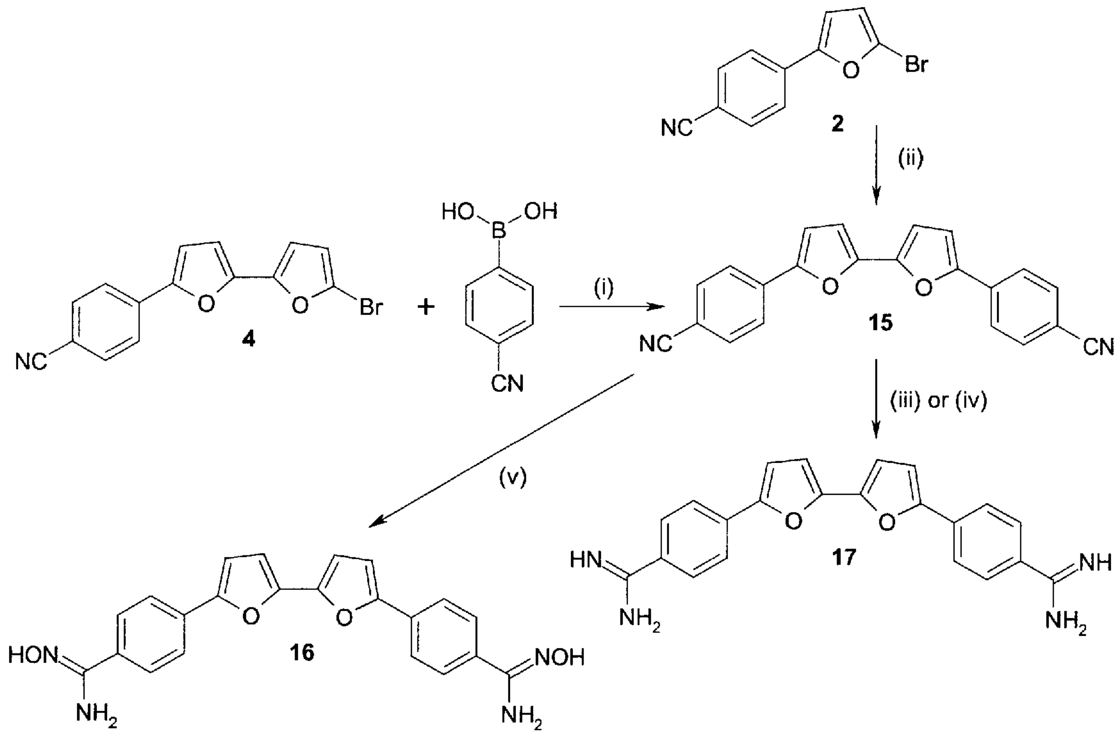


2 4 15 16 17

Reagents and conditions: (i) $Pd(PPh_3)_4$, $Na_2CO_3$, toluene, 80 °C, 24 h; (ii) Bis(tributyltin), $Pd(PPh_3)_4$, toluene, 120 °C, 4 h; (iii) a) $LiN(TMS)_2$, THF, r.t., overnight; b) HCl (gas), dry ethanol, r.t., overnight; (iv) a) HCl, EtOH; b) $NH_3$, EtOH; (v) $NH_2OH$•HCl, KO-*t*-Bu, DMSO, r.t., overnight.

**Scheme 7.** Synthesis of 5,5'-bis-(4-*N*-Hydroxybenzamidine)-2,2'-bifuran (**16**) and 5,5'-bis-(4-Amidinophenyl)-2,2'-bifuran hydrochloride salt (**17**).

**5,5'-bis-(4-Cyanophenyl)-2,2'-bifuran (15)**.

Method (i): Referring now to Scheme 7, to a stirred solution of **4** (1.256 g, 4 mmol), and tetrakis(triphenylphosphine) palladium (230 mg) in toluene (8 mL) under a nitrogen atmosphere was added 4 mL of a 2 M aqueous solution of $Na_2CO_3$ followed by 4-cyanophenyl boronic acid (658 mg, 4.8 mmol) in 4 mL of methanol. The vigorously stirred mixture was warmed to 80 °C for 24 h, then cooled, and the precipitate was filtered. The precipitate was partitioned between methylene chloride (250 mL) and 2 M aqueous $Na_2CO_3$ (20 mL) containing 2.4 mL of concentrated ammonia. The organic layer was dried ($Na_2SO_4$), and then concentrated to dryness under reduced pressure to afford **15** in 52% yield; mp 298-299 °C (DMF). $^1H$ NMR (DMSO-$d_6$); δ 7.09 (d, J = 3.6 Hz, 2H), 7.39 (d, J = 3.6 Hz, 2H), 7.89 (d, J = 8.1 Hz, 4H), 7.97 (d, J = 8.1 Hz, 4H). $^{13}C$ NMR; δ 151.1, 145.8, 133.3, 132.7, 123.8, 118.6, 111.4, 109.45, 109.40. MS (m/z, rel.int.); 336 ($M^+$, 100), 307 (5), 279 (5), 206 (15), 168 (15). High resolution calcd for $C_{22}H_{12}N_2O_2$ ms 336.08988. Observed 336.08978. Calcd for $C_{22}H_{12}N_2O_2$-$0.75H_2O$: C, 75.52; H, 3.86; N, 8.00. Found. C, 75.12; H, 3.48; N, 7.74

Method (ii): Stille homocoupling using bis(n-tributyltin) as catalyst, similar to the Stille coupling described in the synthesis of 3, yield 78%.

**5,5'-bis-(4-*N*-Hydroxybenzamidine)- 2,2'- bifuran (16)**. The same procedure described for 6 was used starting with 15. Free base of 16, yield 82%; mp 309 °C dec. $^1$H NMR (DMSO-$d_6$); δ 5.86 (s, 4H), 6.97 (d, J = 3.6 Hz, 2H), 7.17 (d, J = 3.6 Hz, 2H), 7.76(d, J = 8.4 Hz, 4H), 7.80 (d, J = 8.4 Hz, 4H), 9.72 (s, 2H). $^{13}$C NMR; δ 152.3,150.3,145.2,132.3,129.9,125.8,123.1, 108.6, 108.5. MS (m/z, rel.int.); 403 ($M^+$+1, 90), 388 (15), 370 (10), 201 (100). **Hydrochloride salt of 16.** mp>320 °Cdec. Calcd for $C_{22}H_{18}N_4O_4$-2.0HCl-1.0$H_2O$: C, 53.56; H, 4.49; N, 14.37; Cl, 14.37. Found C, 53.52; H, 4.40; N, 11.00; Cl, 14.13.

**5,5'-bis-(4-Amidinophenyl)-2,2'-bifuran hydrochloride salt (17).**

Method (iii): Prepared by using Li-amide method in 90% yield, starting with **15**. The dinitrile **15** (1.67 mmol), suspended in freshly distilled THF (5 mL), was treated with lithium trimethylsilylamide (2% solution in THF, 3.67 mmol) and the reaction was stirred overnight. The reaction mixture was then cooled to 0 °C and HCl (g) saturated ethanol was added whereupon a precipitate started forming. The reaction was allowed to run overnight, whereafter it was diluted with ether and the formed solid was filtered to give the diamidine salt.

Method (iv): Prepared from **15** by using the Pinner method. See Das, B. P. and D. W. Boykin, J. Med. Chem., 20, 531-536 (1977); McFarland, J. W. and H. L. Howes, Jr., J. Med. Chem., 15, 365-368 (1972). Yield 30%, mp 325-327.5 °C dec. $^1$H NMR (DMSO-$d_6$); δ 7.16 (d, J = 3.6 Hz, 2H), 7.47 (d, J = 3.6 Hz, 2H), 7.96 (d, J = 8.4 Hz, 4H), 8.05 (d, J = 8.4 Hz, 4H), 9.17 (s, 2H), 9.45 (s, 2H). $^{13}$C NMR; δ 164.7, 151.4, 145.9, 134.1, 129.0, 126.3, 123.5, 111.4, 109.5. MS (m/z, rel.int.); 371 (8), 337 (50), 201 (100). Calcd for $C_{22}H_{18}N_4O_2$-2.0HCl-1.0$H_2O$: C, 57.28; H, 4.75; N, 12.03. Found C, 57.56; H, 4.75; N, 12.03.

Example 4

5,5'-Bis-[4-(N-methoxyamidino)phenyl]-2,2'-bifuran Maleate (**19**)

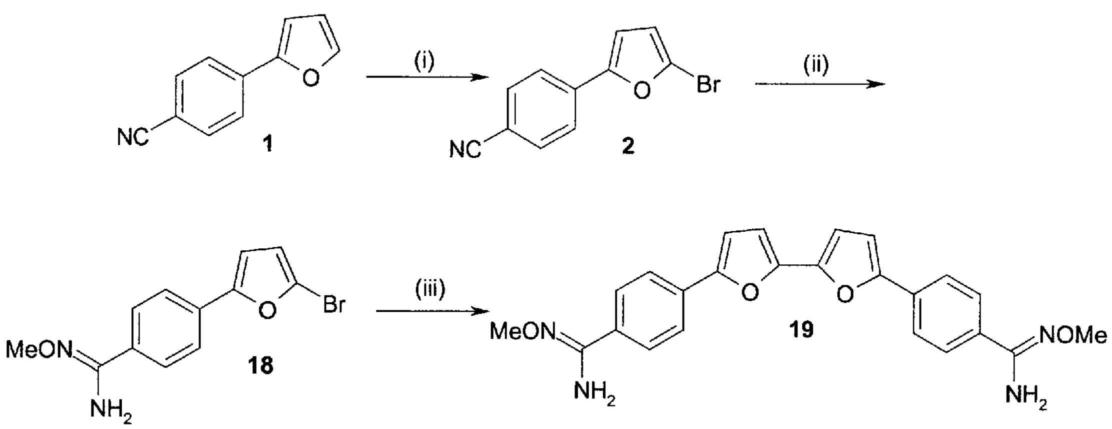

1 2 18 19

Reagents and conditions: (i) NBS, DMF; (ii) a) $NH_2OH$•HCl, KO-*t*-Bu, DMSO; b) LiOH, $(Me)_2SO_4$, dioxane/DMSO; (iii) $(Bu_3Sn)_2$, $Pd(PPh_3)_4$, toluene, 120 °C, 3 h.

**Scheme 8.** Synthesis of 5,5'-Bis-[4-(N-methoxyamidino)phenyl]-2,2'-bifuran Maleate (**19**).

**2-Bromo-5-(4-cyanophenyl)furan (2)**. Referring now to Scheme 8 above, to a chilled (ice/water bath) solution

of 2-(4-cyanophenyl)furan (28.25g, 0.167 mol) in DMF (100 mL) was added portionwise NBS (31.20 g, 1.05 eq., freshly recrystallized from nitromethane) with stirring (approximately 1 g portions over course of approximately 40 minutes). The resulting solution was stirred for 2 h at room-temperature, at which point TLC showed consumption of starting material. During the course of the reaction, the color went from yellow to orange and then finally to red. The solution was then diluted with water (approximately 300 ml) to give a pink/red solid, which was collected, washed with water, and air dried. Yield: 39.0 g, 94%. A small sample was recrystallized from MeOH/water to give a pale red crystalline solid, mp 96.5-97 ˚C. $^1$H NMR (DMSO-$d_6$): 6.80 (d, J = 3.6 Hz, 1H), 7.29 (d, J = 3.6 Hz, 1H), 7.87 (m, 4H). IR (cm$^{-1}$): 3142, 3128, 3060, 2226, 1613, 1515, 1475, 1015, 929, 833, 787, 545.

**2-Bromo-5-[4-(N-methoxyamidino)phenyl]furan (18).** To a chilled suspension of hydroxylamine hydrochloride (17.25 g, 0.25 mol) in DMSO (150 ml) was added portionwise KO-t-Bu (28.0 g, 0.25 mol) and the mixture was stirred under nitrogen for 30 min. 2-Bromo-5-(4-cyanophenyl)furan (17.37 g, 0.07 mol) was then added and the mixture was stirred overnight at room-temperature. The resulting solution was diluted with excess water to give 2-bromo-5-[4-(N-hydroxyamidino)phenyl]furan as an off-white solid, which was collected and washed with water. Yield: 19.45 g, 99%; mp 162-164 ˚C. $^1$H NMR (DMSO-$d_6$): 5.84 (broad s, 2H), 6.71 (d, J = 3.6 Hz, 1H), 7.05 (d, J = 3.6 Hz, 1H), 7.65 (d, J = 8.4 Hz, 2H), 7.72 (d, J = 8.4 Hz, 2H), 9.70 (s, 1H). IR (cm$^{-1}$): 3475, 3369, 3209 (broad), 1639, 1482, 1341, 1017, 927, 787. The intermediate amidoxime (38.9 g, 0.138 mol) was dissolved in a mixture of DMSO (60 ml) and dioxane (300 ml) and with chilling was treated with a solution of LiOH hydrate (11.61 g, 0.277 mol) in water (60 ml). At room temperature, the resulting suspension was then treated dropwise via an addition funnel with dimethyl sulfate (26.18 g. 0.208 mol) over the course of approximately 30 min. Following the addition, the mixture became slightly warm and the solids dissolved. After stirring overnight, the mixture was diluted with excess water and extracted with EtOAc. Purification of the residue by column chromatography on silica gel eluting with 10% EtOAc in hexane gave the slightly impure product, which was further purified by recrystallization from MeOH/water in multiple crops to give an off-white solid, mp 116-117 ˚C. Yield: 28.0 g, 69%. $^1$H NMR (DMSO-*$d_6$*, an apparent mixture of stereoisomers): 3.74 (2s, 3H), 6.09 (broad s, 2H), 6.72 (2d, J = 3.6 Hz, 1H), 7.09 (2d, J = 3.6 Hz, 1H), 7.70 (m, 4H). IR(cm$^{-1}$): 3459, 3312, 3177, 2957, 2935, 2901, 2818, 1634, 1403, 1051,910,842,785.

**5,5'-Bis-[4-(*N*-methoxyamidino) phenyl]- 2,2'- bifuran (19)**. A mixture of 2-bromo-5-[4-(N-methoxyamidino) phenyl]furan (27.90 g, 94.5 mmol), hexa-n-butylditin (28.37 g, 48.9 mmol) and tetrakis(triphenylphosphine)palladium(0) (1.40 g, 1.2 mmol) in toluene (400 ml) was heated under nitrogen in an oil bath set at 120 ˚C for 3 h and then cooled to room temperature. After standing 1 h, the resulting precipitate was collected and rinsed with diethyl ether to give a yellow fluffy solid (12.25 g, 60%). The product was recrystallized by being dissolved in hot DMF (100 ml) and then adding MeOH (200 ml). After chilling for several hours, the product was collected and rinsed with MeOH to yield a fine yellow crystalline solid, mp 258-259 ˚C. Yield: 10.75 g, 53%. $^1$H NMR (DMSO-$d_6$): 3.76 (s, 6H), 6.11 (br s, 4H), 6.99 (d, J = 3.6 Hz, 2H), 7.20 (d, J = 3.6 Hz, 2H), 7.73 (d, J = 8.7 Hz, 4H), 7.80 (d, J = 8.7 Hz, 4H). $^{13}$C NMR(DMSO-*$d_6$*): 60.6, 108.5, 108.8, 123.1, 126.2, 130.3, 131.4, 145.2, 150.5, 152.2. IR(cm$^{-1}$) 3520, 3412, 3125, 2991, 2961, 2897, 2817, 1622, 1415, 1402, 1056, 904, 842, 789. MS (EI): *m/z* 431 (MH$^+$). Anal. Calcd. for $C_{24}H_{22}N_4O_4$ (430.46): C, 66.96; H, 5.15; N, 13.02. Found: C, 66.91; H, 5.14; N, 13.01.

**5,5'-Bis-[4-(*N*-methoxyamidino) phenyl]- 2,2'- bifuran Maleate (19a).** The free base (8.61 g, 20.0 mmol) and maleic acid (2.33g, 20.0 mmol) were heated overnight in EtOH (200 ml) at 50-60 ˚C and then refluxed for 30 min. The suspension was then concentrated in vacuo, and the residue was triturated with diethyl ether, filtered and vacuum dried at 50-60 ˚C for 24 hr to give a fluffy yellow solid (10.50 g). Combustion analysis showed that only 0.8 molar equivalent of maleic acid was present in the product. $^1$H NMR (DMSO-*$d_6$*): 3.76 (s, 6H), 6.23 (broad s) and 6.24 (s): maleate vinyl Hs overlapping with 2$NH_2$, integration 0.83% of expected, 6.99 (d, J = 3.6 Hz, 2H), 7.21 (d, J = 3.6 Hz, 2H), 7.74 (d, J = 8.7 Hz, 4H), 7.82 (d, J = 8.7 Hz, 4H). Calcd. for $C_{24}H_{22}N_4O_4$-0.8$C_4H_4O_4$ (523.32): C, 62.42; H, 4.85; N, 10.71. Found: C, 62.72; H, 4.94; N, 10.74.

Example 5

5,5'-Bis-(5-amidino-2-pyridyl)-2,2'-bifuran **(22)** and 5,5'-Bis-15-(*N*-methyoxyamidino)-2-pyridy]-2,2'-bifuran **(23)**

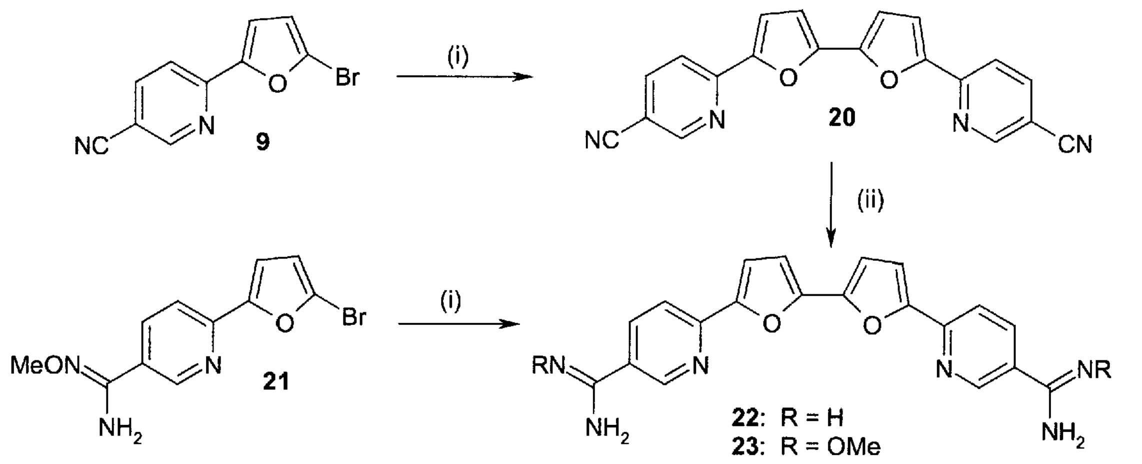


Reagents and conditions: (i) Bis(tributyltin), $Pd(PPh_3)_4$, toluene, 120 °C, 4 h; (ii) a) $LiN(TMS)_2$, THF, r.t., overnight; b) HCl (gas), dry ethanol, r.t., overnight.

**Scheme 9.** Synthesis of Compounds **22** and **23**.

**Compound 20**. Referring now to Scheme 9 above, compound **20** was prepared *via* Stille homocoupling of **9,** analogously to the synthesis of **15** from **2.** Yield 81 %, mp >300 °C. $^1$H NMR (DMSO-$d_6$); δ 7.17 (d, J = 3.6 Hz, 2H), 7.48 (d, J = 3.6 Hz, 2H), 8.00 (d, J = 8.1 Hz, 2H), 8.31 (dd, J = 8.1, 2.1 Hz, 2H), 8.98 (d, J = 2.1 Hz, 2H). $^{13}$C NMR; δ 152.3, 151.5, 149.9, 146.5, 140.3, 117.9, 116.6, 114.2, 110.1, 906.4. MS (m/z, rel.int.); 339 ($M^+$+1, 100), 319 (15), 277 (10).

**5,5'-Bis-(5-amidino-2-pyridyl)-2,2'-bifuran (22)**. Nitrile reaction using $LiN(TMS)_2$ analogous to 17. Yield 92%, mp >300 °C. $^1$H NMR ($D_2O$/DMSO-$d_6$); δ 7.21 (d, J = 3.6 Hz, 2H), 7.52 (d, J = 3.6 Hz, 2H), 8.07 (d, J = 8.4 Hz, 2H), 8.31 (dd, J = 8.4, 2.1 Hz, 2H), 9.00 (d, J = 2.1 Hz, 2H). $^{13}$C NMR; δ 164.1, 152.2, 151.9, 149.3, 147.3, 137.7, 122.5, 118.8, 114.9, 110.9. MS (m/z, rel.int.); 373 ($M^+$+1, 60), 356 (5), 187 (100). Calcd for $C_{20}H_{16}N_6O_2$-4.0HCl-0.75$H_2O$: C, 45.17; H, 4.08; N, 15.80. Found C, 45.17; H, 4.25; N, 15.53.

**5,5'-Bis-[5-(N-methyoxyamidino)-2-pyridyl]-2,2'-bifuran (23).** Stille homocoupling analogous to **19.** Free base yield 95%, mp 292-294 °C. $^1$H NMR (DMSO-$d_6$); δ 3.80 (s, 6H), 6.07 (s, 4H), 7.06 (d, J = 3.6 Hz, 2H), 7.30 (d, J = 3.6 Hz, 2H), 7.85 (d, J = 8.4 Hz, 2H), 8.11 (d, J = 8.4 Hz, 2H), 8.88 (s, 2H). $^{13}$C NMR; δ 152.4, 148.8, 148.0, 146.7, 145.8, 133.7, 126.4, 117.5, 111.3, 109.0, 60.4. MS (m/z, rel.int.); 432 ($M^+$, 100), 385 (20), 370 (60).

**Hydrochloride salt of 23:** mp 254-256 °C. Calcd for $C_{22}H_{20}N_6O_4$-4.0HCl-2.5$H_2O$: C, 42.39; H, 4.68; N, 13.48. Found C, 42.32; H, 4.52; N, 13.35.

Example 6

5,5'-Bis-4-amidinophenyl)-2,2'-bithiophene **(26a)** and 5,5'-Bis-(4-amidino-2-pyridyl)-2,2'-bithiophene**(26b)**

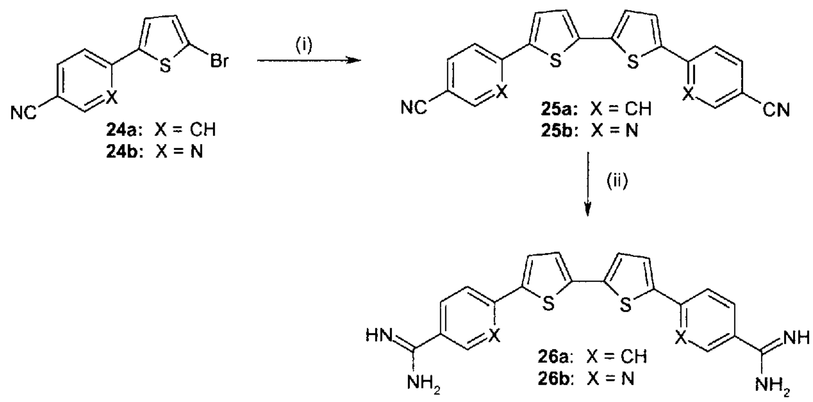


Reagents and conditions: (i) Bis(tributyltin), $Pd(PPh_3)_4$, toluene, 120 °C, 4 h; (ii) a) $LiN(TMS)_2$, THF, r.t., overnight; b) HCl (gas), dry ethanol, r.t., overnight.

**Scheme 10**. Synthesis of compounds **26a** and **26b**.

**Compound 25a**. Referring now to Scheme 10, **25a** was prepared via Stille homocoupling of **24a**. Yield 91%, mp 298-300 °C. $^1H$ NMR(DMSO-$d_6$); δ 7.45 (d, J = 4.2 Hz, 2H), 7.68 (d, J = 4.2 Hz, 2H), 7.85-7.89 (m, 8H). $^{13}C$ NMR; δ 140.3, 137.0, 132.5, 126.8, 125.7, 125.4, 118.0, 109.6. Calcd for $C_{22}H_{12}N_2S_2$: C, 71.71; H, 3.28. Found C, 71.48; H, 3.40.

**Compound 25b.** Prepared via Stille homocoupling of **24b.** Yield 85%, mp 300°C. $^1H$ NMR (DMSO-$d_6$); δ 7.53 (d, J = 4.2 Hz, 2H), 7.94 (d, J = 4.2 Hz, 2H), 8.07 (d, J = 8.1 Hz, 2H), 8.23 (d, J = 8.1 Hz, 2H), 8.90 (s, 2H). MS (m/z, rel.int.); 370 ($M^+$, 100), 337 (30), 305 (5), 292 (5), 223 (20), 185 (60), 163 (80). Calcd for $C_{20}H_{10}N_4S_2$: C, 64.84; H, 2.72. Found C, 64.59; H, 2.88.

**5,5'-Bis-(4-amidinophenyl)-2,2'-bithiophene (26a).** Nitrile reaction of **25a** using $LiN(TMS)_2$. Yield 73%, mp >300 °C. $^1H$ NMR($D_2O$/DMSO-$d_6$); δ 7.52 (d, J = 3.9 Hz, 2H), 7.75 (d, J = 3.9 Hz, 2H), 7.88 (d, J = 8.4 Hz, 4H), 7.94 (d, J = 8.4 Hz, 4H). $^{13}C$ NMR; δ 165.6, 141.3, 138.8, 137.8, 129.4, 127.7, 126.7, 126.0. MS (m/z, rel.int.); 403 ($M^+$+1, 20), 386 (25), 368 (40), 185 (95), 171 (100). Calcd for $C_{22}H_{18}N_4S_2$-2.0HCl-2.4$H_2O$: C, 51.05; H, 4.79; N, 10.80. Found C, 51.12; H, 4.57; N, 10.50.

**5,5'-Bis-(4-amidino-2-pyridyl)-2,2'-bithiophene (26b)**. Nitrile reaction of **25b** using $LiN(TMS)_2$. Yield 89%, mp >300 °C. $^1H$ NMR ($D_2O$/DMSO-$d_6$); δ 7.55 (d, J = 3.9 Hz, 2H), 7.94 (d, J = 3.9 Hz, 2H), 8.09 (d, J = 8.4 Hz, 2H), 8.24 (dd, J = 8.4, 2.1 Hz, 2H), 8.93 (d, J = 2.1 Hz, 2H). MS (m/z, rel.int.); 405 ($M^+$+1, 50), 231 (15), 203 (100). Calcd for $C_{20}H_{16}N_6S_2$-4.0HCl-0.75$H_2O$: C, 42.60; H, 3.84; N, 14.90. Found C, 42.56; H, 3.83; N, 14.66.

Example 7

5,5'-Bis-(4-amidinophenyl)-2,2'-biselenophene (**29a**) and 5,5'-Bis- 4-amidino-2-pyridyl)-2,2'-biselenophene (**29b**)

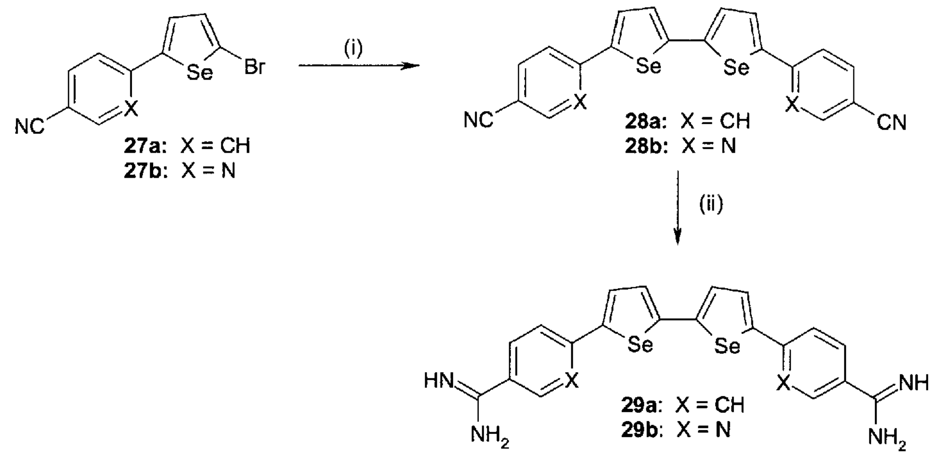

27a: X = CH
27b: X = N
28a: X = CH
28b: X = N
29a: X = CH
29b: X = N

Reagents and conditions: (i) Bis(tributyltin), $Pd(PPh_3)_4$, toluene, 120 °C, 4 h; (ii) a) $LiN(TMS)_2$, THF, r.t., overnight; b) HCl (gas), dry ethanol, r.t., overnight.

**Scheme 11.** Synthesis of Compounds **29a** and **29b**.

**Compound 28a.** Referring to Scheme 11 above, Stille homocoupling of **27a** gave **28a.** Yield 92%, mp 285-286.5 ˚C. $^1H$ NMR(DMSO-$d_6$); δ 7.52 (d, J = 3.9 Hz, 2H), 7.76-7.84 (m, 10H). $^{13}C$ NMR; δ 146.6, 144.5, 139.2, 132.6, 129.1, 128.6, 125.9, 118.2, 109.6. MS (m/z, rel.int.); 462 ($M^+$, 90), 464 ($M^+$+2, 100), 384 (5), 302 (15). Calcd for $C_{22}H_{12}N_2Se_2$: C, 57.16; H, 2.62. Found C, 56.98; H, 2.63.

**Compound 28b.** Stille homocoupling of **27b.** Yield 85%, mp > 300 ˚C. $^1H$ NMR (DMSO-$d_6$); δ 7.62 (d, J = 3.9 Hz, 2H), 7.98-8.30 (m, 6H), 8.93 (d, J = 2.1 Hz, 2H). MS (m/z, rel.int.); 464 ($M^+$, 60), 466 ($M^+$+2, 100), 385 (25), 305 (40).

**5,5'-Bis-(4-amidinophenyl)-2,2'-biselenophene (29a)**. Nitrile reduction of **28a** analogous to preparation of **17.** Yield 80%, mp > 300 ˚C. $^1H$ NMR ($D_2O$/DMSO *$d_6$);* δ 7.46 (d, J = 3.9 Hz, 2H), 7.72-7.82 (m, 10H). $^{13}C$ NMR; δ 165.4, 147.4, 145.1, 140.6, 129.5, 129.3, 129.2, 126.9, 126.3. MS (m/z, rel.int.); 496 ($M^+$, 10), 498 ($M^+$+2, 20), 250 (100). Calcd for $C_{22}H_{18}N_4Se_2$-2.0HCl-1.5$H_2O$: C, 44.31; H, 3.90; N, 9.36. Found C, 44.28; H, 3.90; N, 9.13.

**5,5'-Bis-(4-amidino-2-pyridyl)-2,2'-biselenophene (29b)**. Nitrile reduction of **28b** analogous to the preparation of **17**. Yield 71%, mp >300 ˚C. $^1H$ NMR ($D_2O$/DMSO-$d_6$) δ 7.50 (s, 2H), 7.91-8.09 (m, 6H), 8.77 (s, 2H). MS (m/z, rel.int.); 499 ($M^+$+1, 25), 484 (15), 290 (30), 251 (100). Calcd for $C_{20}H_{16}N_6Se_2$-3.0HCl-0.4EtOH: C, 39.90; H, 3.41; N, 13.40. Found C, 39.98; H, 3.19; N, 13.07.

Example 8

Compounds **32** and **33**

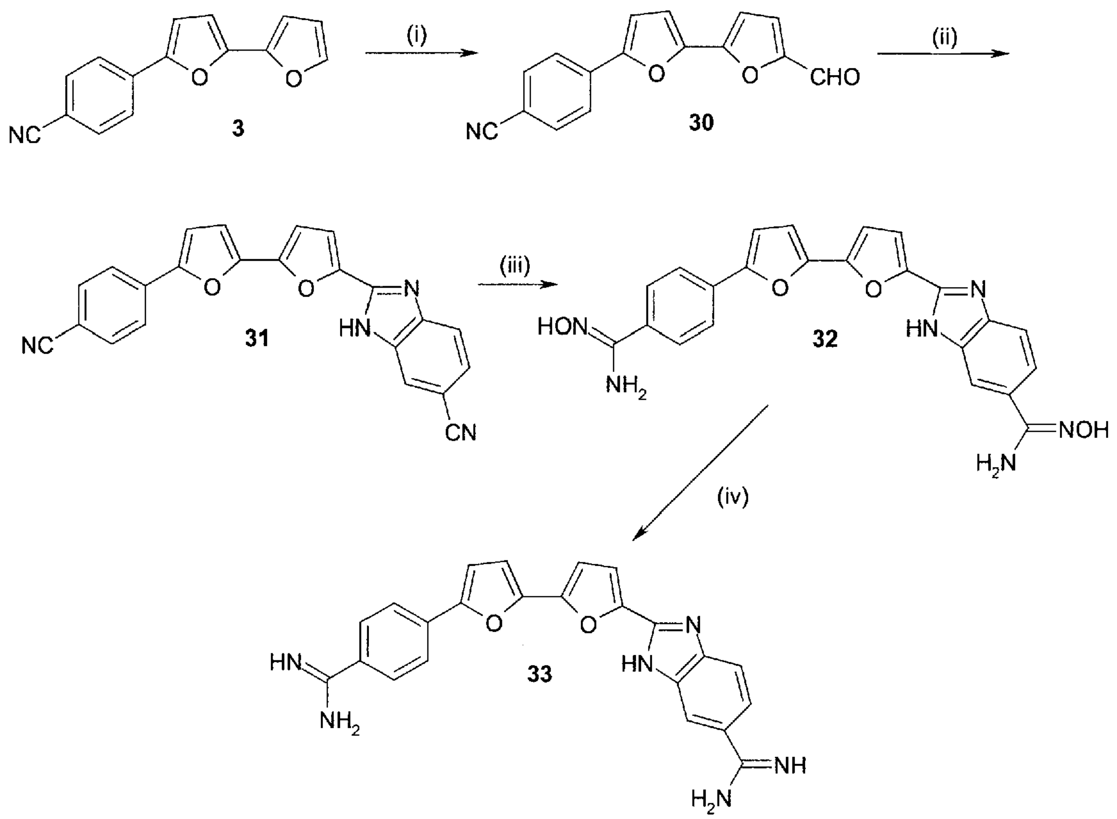


3, 30, 31, 32, 33

Reagents and conditions: (i) DMF/$POCl_3$; (ii) 3,4-diaminobenzonitrile, 1,4-benzoquinone; (iii) $NH_2OH$•HCl, KO-*t*-Bu, DMSO; (iv) a) AcOH/$Ac_2O$; b) $H_2$/Pd-C, AcOH.

**Scheme 12.** Synthesis of Compounds **32** and **33.**

**Compound 30**. Referring to Scheme 12 above, freshly distilled DMF (4.2 mL) was stirred in an ice bath and treated dropwise with $POCl_3$ (14 mL) and then portionwise **3** (1.645 g, 7 mmol) in methylene chloride (12 mL) was added. The reaction mixture was stirred under heating at 60 °C for 2 h. The methylene chloride was distilled off under reduced pressure, then the remaining solution was poured into ice water and the product was extracted into EtOAc. The extract was dried and evaporated to give **30** in 85.7%, mp 176 °C. $^1H$ NMR ($CDCl_3$); δ 6.86 (d, J = 3.9 Hz, 1H), 6.95 (d, J = 3.6 Hz, 1H), 7.03 (d, J = 3.6 Hz, 1H), 7.35 (d, J = 3.9 Hz, 1H), 7.70 (d, J = 8.4 Hz, 2H), 7.82 (d, J = 8.4 Hz, 2H), 9.66 (s, 1H). $^{13}C$ NMR; δ 176.9, 153.1, 151.9, 150.4, 145.5, 133.5, 132.6, 124.2, 123.3, 118.6, 111.9, 111.1, 110.4, 108.2. Calcd for $C_{16}H_9NO_3$: C, 73.00; H, 3.44; N, 5.32. Found. C, 73.09; H, 3.58; N, 5.25.

**Compound 31.** A solution of **30** (526 mg, 2 mmol), 3,4-diaminobenzonitrile (266 mg, 2 mmol), and benzoquinone (216 mg, 2 mmol) in ethanol (25 mL) was allowed to reflux under nitrogen for overnight. The reaction mixture was distilled under reduced pressure. The residue was triturated with ether and filtered to afford **31** in 79.7%, mp 295-296 °C. $^1H$ NMR (DMSO-$d_6$); δ 7.10 (d, J = 3.6 Hz, 1H), 7.17 (d, J = 3.6 Hz, 1H), 7.44 (d, J = 3.6 Hz, 2H), 7.62 (d, J = 7.2 Hz, 1H), 7.69 (d, J = 7.5 Hz, 0.5H), 7.80 (d, J = 7.2 Hz, 0.5H), 8.18 (s, 1H), 13.58 (s, 1H). $^{13}C$ NMR (DMSO-*$d_6$*); δ 151.5, 146.6, 145.6, 144.2, 133.3, 132.9, 124.0, 119.9, 118.8, 114.3, 111.6, 110.1, 109.6, 109.3, 104.2. EIMS (m/z, rel.int.); 376 ($M^+$, 100), 319 (5), 246 (10), 218 (10), 188 (15). High resolution calcd for $C_{23}H_{12}N_4O_2$ ms 376.09603. Observed 376.09468. Anal. Calcd for $C_{23}H_{12}N_4O_2$: C, 73.39; H, 3.21; N, 14.88. Found. C, 73.12; H, 3.23; N, 14.87.

**Compound 32.** Analogous to the preparation of **6,** starting with **31.** Yield 93%, mp >300 °C. $^1H$ NMR (DMSO-*$d_6$*); δ 5.86 (s, 2H), 6.20 (s, 2H), 7.02-7.17 (m, 2H), 7.19 (d, J = 3.6 Hz, 1H), 7.37 (d, J = 3.6 Hz, 1H), 7.58 (s, 2H), 7.76-7.97 (m, 5H), 9.66 (s, 2H), 13.00 (br s, 1H). EIMS (m/z, rel.int.); 443 ($M^+$+1, 60), 428 (25), 241 (20), 222 (100).

**Compound 33.** Analogous to the preparation of **14,** starting with **32.** Yield 67%, mp 248-250 °C. $^1H$ NMR ($D_2O$/DMSO-*$d_6$)*; δ 1.87 (s, 3 x $CH_3$), 7.15 (s, 2H), 7.32-7.46 (m, 2H), 7.66-7.79 (m, 2H), 7.95-8.24 (m, 5H). MS (m/z, rel.int.); 410 ($M^+$, 10), 392 (100), 365 (90), 350 (80), 336 (5). Calcd for $C_{23}H_{18}N_6O_2$-3.0AcOH-0.35$H_2O$: C, 58.35; H, 5.14; N,

14.09. Found. C, 58.03; H, 4.88; N, 14.20.

Example 9

Compound 39

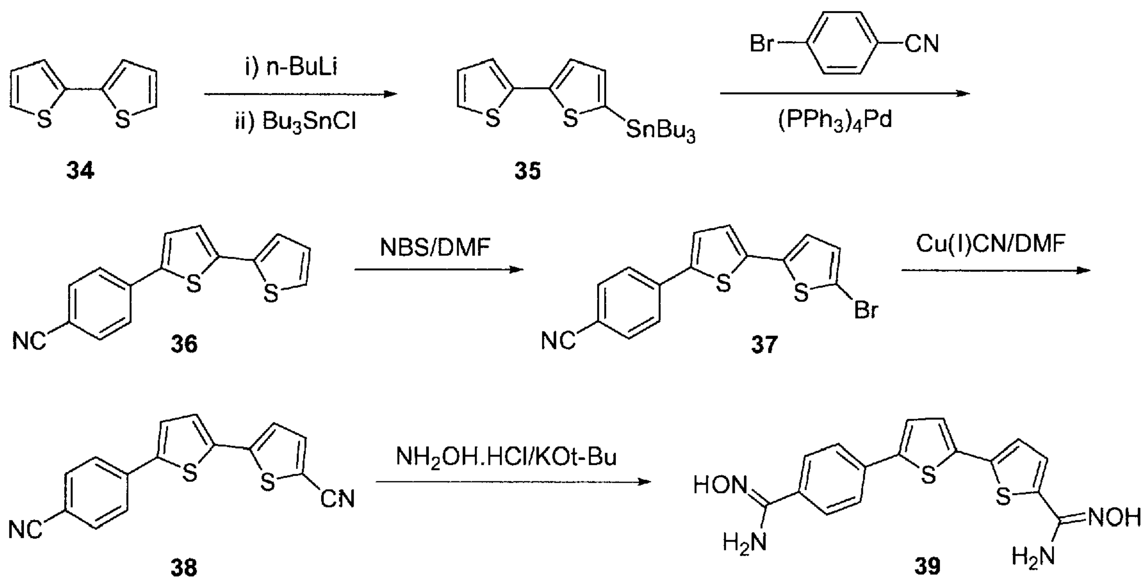


34 35 36 37 38 39

**Scheme 13.** Synthesis of Compound **39**.

Example 10

Compound 45

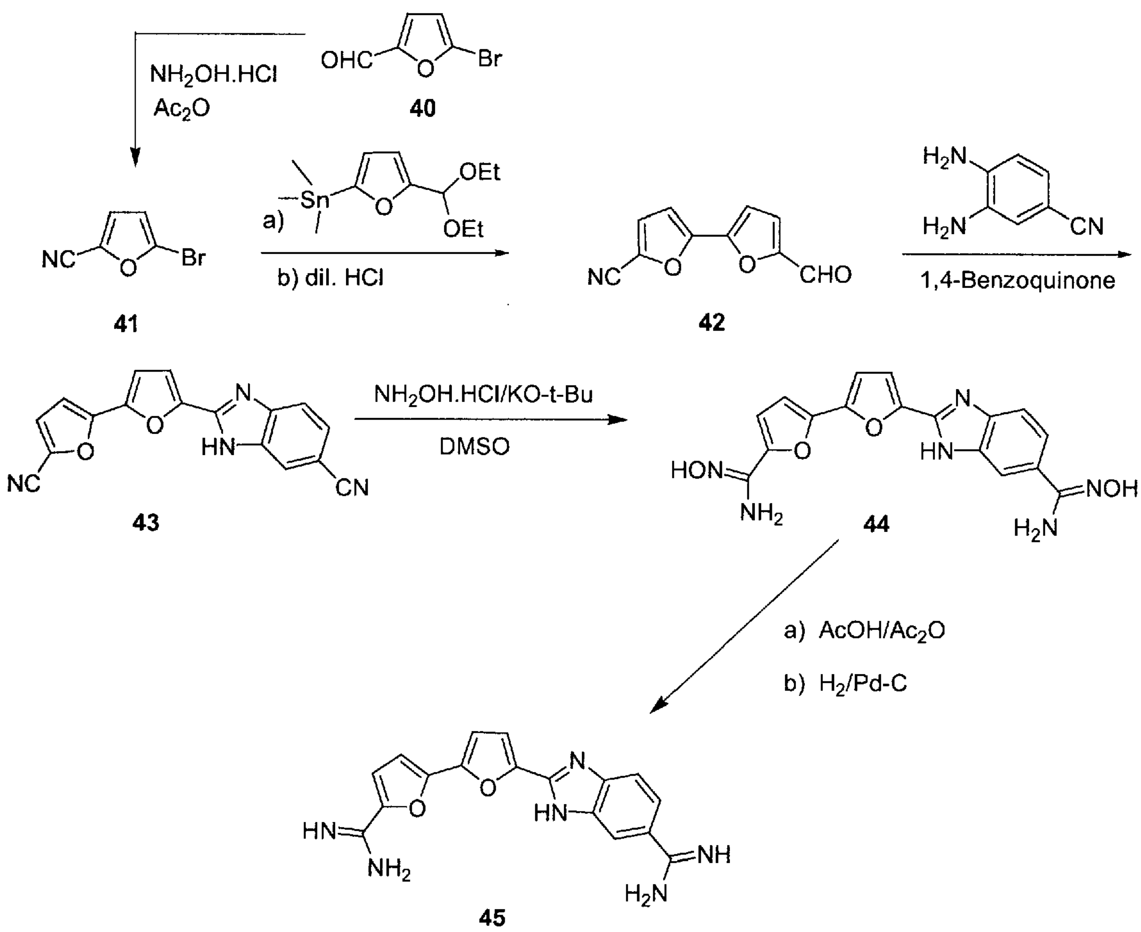


**Scheme 14.** Synthesis of Compound **45**

Example 11

Synthesis of compounds of **61a, 61b, 62a,** and **62b**

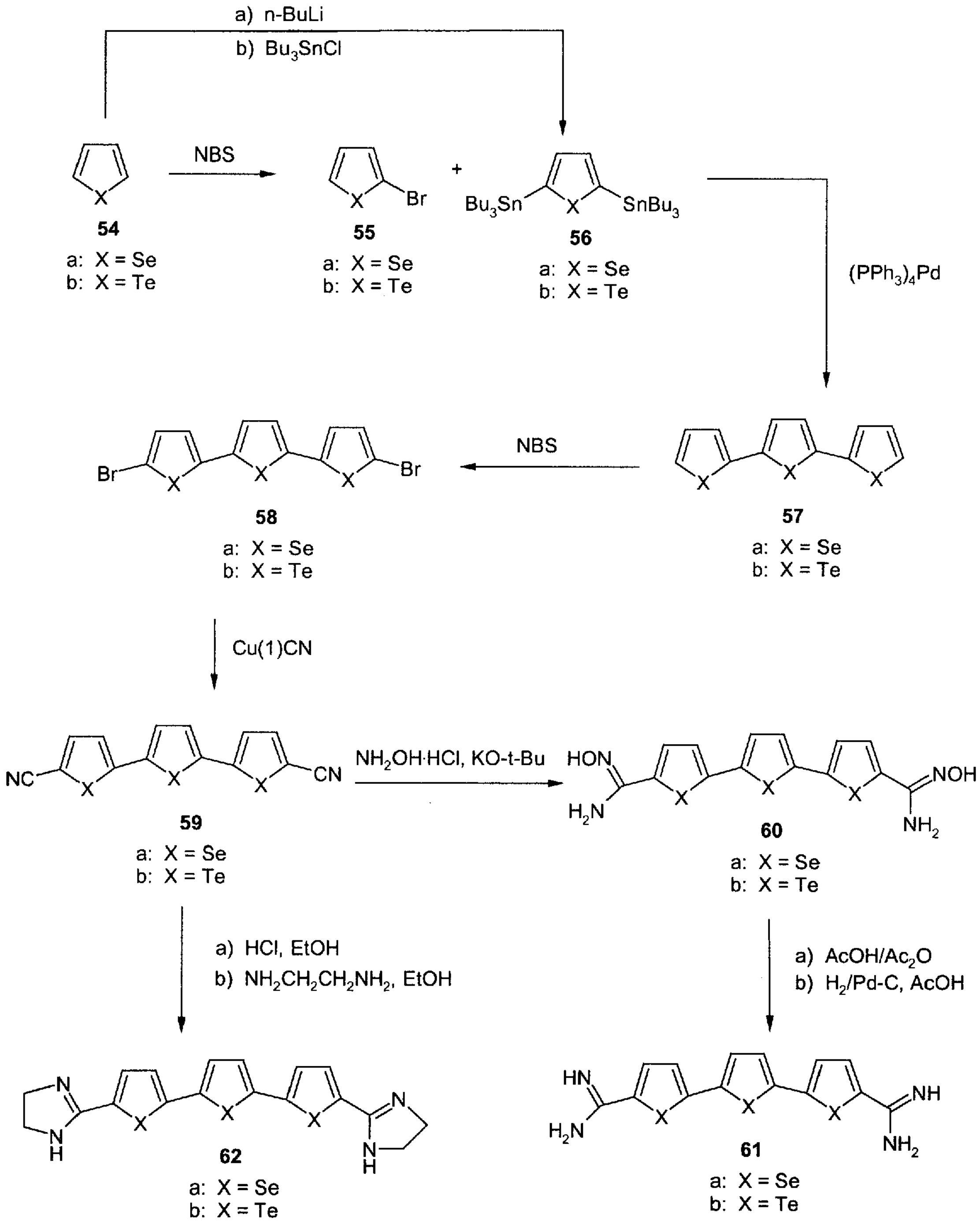


**Scheme 15**. Synthesis of Compounds **61a**, **61b**, **62a**, and **62b**.

Example 12

Absorbance and CD Measurements

The oligonucleotides d[$AG_3(T_2AG_3)_3$] (SEQ ID NO: 1), d[$AG_3TG_4AG_3TG_4A$] (SEQ ID NO: 2), d[$G_4(T_4G_4)_3$] (SEQ ID NO: 3), d[$(T_2G_4)_4$] (SEQ ID NO: 4), d[$(GC)_7$] (SEQ ID NO: 5), d[GCGAATTCGC] (SEQ ID NO: 6), d [$G_2T_2G_2TGTG_2T_2G_2$] (SEQ ID NO: 7), d[CGAATTCGTCTCCGAATTCG] (SEQ ID NO: 8), d[TAGGGUTAGGGT] (SEQ

ID NO: 9), d[TAGGGUUAGGGT] (SEQ ID NO: 10), d[TAGGGUTAGGU] (SEQ ID NO: 11), d[TTAGGGT] (SEQ ID NO: 12), and d[TGGGGT] (SEQ ID NO: 13) were purchased with HPLC purification from Midland Certified Reagent Company (The Midland Certified Reagent Company, Inc., Midland, Texas, USA). The concentration of each DNA sample was determined spectrophotometrically at 260 nm using the nearest neighbor extinction coefficient.

Stock solutions of individual dicationic polyaryl compounds were prepared in double distilled water and diluted to working concentrations immediately before use with buffer. All measurements were performed at 20 °C in a 10 mM HEPES buffer (pH 7.4) containing 3 mM EDTA and 50 mM KCl, NaCl, or LiCl. Absorbance spectra were obtained on a Varian Cary 300 BIO UV-visible spectrophotometer (Varian, Inc., Palo Alto, California, United States of America) in a quartz cell with a 1 cm pathlength. CD spectra were recorded using a Jasco J-810 spectrapolarimeter (Jasco, Inc., Easton, Maryland, United States of America) in a 1-cm cell using an instrument scanning speed of 50 nm/min with a response time of 1 s. The spectra were averaged over four scans. A buffer baseline scan was collected in the same cuvette and subtracted from the average scan for each sample. Appropriate amounts of dicationic compound stock solution or DNA were added sequentially to increase the molar ratio.

Example 13

Surface Plasmon Resonance Studies

Surface plasmon resonance (SPR) measurements were performed with a four-channel Biacore® 3000 optical biosensor system (Biacore International AB, Uppsala, Sweden). 5'-biotin labeled DNA was immobilized onto streptavidin-coated Biacore® SA sensor chips as previously described. See Lacy, E.R., et al., J. Amer. Chem. Soc., 124, 2153-2163 (2002); Mazur, S., et al., J. Mol. Biol., 300, 321-337 (2000); Nguyen, B., et al., Biopolymers, 63, 281-297 (2002); Wang, L., et al., Proc. Natl. Acad. Sci. USA, 97, 12-16 (2000); and Wang, L., et al., Biochemistry, 40, 2511-2521 (2001). Two flow cells were used to immobilize the DNA oligomer samples, while a third cell was left blank as a control. The SPR studies were performed at 25 °C in a filtered, degassed, 10 mM HEPES buffer (pH 7.4) containing 200 mM KCl, 3 mM EDTA and 0.005% surfactant P20 (Biacore International AB). Compound solutions were prepared by serial dilutions from stock solution and injected from 7-mm plastic vials with pierceable plastic crimp caps.

Solutions of known concentration of **8** ranging from 2 nM to 10 μM were injected through the flow cells until a constant steady-state response was obtained. Compound solution flow was then replaced by buffer flow resulting in dissociation of the complex. The reference response from the blank cell was subtracted from the response in each cell containing DNA to give a signal (RU, response units) that is directly proportional to the amount of bound compound. A set of sensograms at different concentrations of **8** binding to human telomeric DNA were obtained. The instrument response (RU) in the steady-state region was determined by linear averaging over a 50 second time span. The predicted maximum response per bound compound in the steady-state region ($RU_{max}$) was determined from the DNA molecular weight, the amount of DNA on the flow cell, the compound molecular weight, and the refractive index gradient ratio of the compound and DNA, as previously described. The number of binding sites was estimated from fitting plots of RU versus $C_{free}$. To obtain the binding constants, the data were fitted to a two-site equilibrium model using KaleidaGraph (Synergy Software, Reading, Pennsylvania, United States of America) for nonlinear least square optimization of the binding parameters, where $RU_{max}$ is the maximum response per bound compound and $K_1$ and $K_2$ are the macroscopic binding constants for a two-site binding model:

$$RU = RU_{max}*(K_1*C_{free} + 2*K_1*K_2*C_{free}^2)/(1+ K_1*C_{free} + K_1*K_2*K_{Cfree}^2)$$

wherein, for a single binding site model, $K_2$ is equal to zero.

Example 14

Fluorescence Measurements

Fluorescence experiments were performed using a Cary Eclipse fluorescence spectrometer (Varian, Inc., Palo Alto, California, United States of America). All measurements were conducted at 20 °C in a 10-mM HEPES buffer (pH 7.4) containing 3 mM EDTA and 50 mM KCl. A quartz cell with a 0.5-mm pathlength was used. A fluorescence titration was performed by titrating increasing amounts of $d[AG_3(T_2AG_3)_3]$ (SEQ ID NO: 1) into a cuvette containing 4.98 μM of compound **8.** The excitation wavelength was set to 393.5 nm, which is the isosbestic point as determined from the absorbance titration, such that the absorbance of the sample remained constant during the titration. The emission was scanned from 400 to 600 nm, with an emission slit width of 2.5 nm. DNA was added before each scan increasing the

concentration by 0.213 μM with each addition, up to a total DNA concentration of 4.04 μM. A fluorescence energy transfer study also was performed. The emission wavelength was set to 469 nm with an excitation slit width of 5 nm. The excitation spectrum was scanned from 230nm to 330 nm for **8** alone and in the presence of d[$AG_3(T_2AG_3)_3$] (SEQ ID NO: 1).

Example 15

NMR Studies

All 1 D and 2D experiments were performed on 600 MHz Bruker and Varian Unity spectrometers and a 500 mHz Bruker spectrometer equipped with a cryoprobe. Several DNA quadruplex sequences were used in the NMR experiments with concentrations for NMR samples typically from 0.1 to 0.5 mM. The buffer for the NMR studies was 50 mM KCl, 10 mM $K_2HPO_4$, 0.1 mM EDTA, with 0.01 mM DSS as an internal reference, adjusted to pH 7.0. Compounds were titrated with quadruplex DNA with compound:DNA ratios varying from 0.5 to 2. Temperature dependent 1D spectra of imino protons were performed using jump-and-return and WATERGATE pulse sequences for water suppression of water and $D_2O$ samples. Phase sensitive NOESY and TOCSY studies for water samples were performed using WATERGATE solvent suppression method. 2D spectra were collected over 2048 x 2048 data points with a spectral resolution of 2.5 Hz. Mixing time for TOCSY studies varied from 60 to 100 ms, whereas mixing time for NOESY experiments varied from 200 to 300 ms. All 1D NMR studies were performed over a wide range of temperatures varying from 10 ˚C to 50 ˚C. Finally, a temperature of 35 ˚C was chosen to perform 2D studies.

Example 16

Discussion of Structural Studies

Achiral molecules, such as compound **8**, exhibit no CD signal in solution. When an achiral ligand binds tightly to a chiral host, such as DNA, a CD signal is induced in the wavelength region corresponding to the absorbance of the ligand. Figure 3 shows the CD spectra of the human telomeric sequence titrated with **8** in the presence of $K^+$. The spectra show an induced CD signal, which indicates that **8** is binding to the DNA. As compound is added to the DNA, the induced signal exhibits exciton splitting with a positive band centered at 432 nm, a negative band centered at 416 nm, and an isoelliptic point at 424 nm, which also is the absorbance maximum of compound **8** when bound to DNA. An additional negative band centered at 395 nm overlaps with the higher energy exciton band. Since exciton splitting arises from interaction between degenerate transition dipoles, it indicates that **8** is binding to the DNA as one or more dimers. The induced exciton CD exhibits a very large ellipticity as compared to the ellipticity in the DNA region. Restriction of the rotational and translational freedom of the two molecules of the dimer relative to each other will cause an increase in the rotational strength of the induced CD band, resulting in a large ellipticity. See Allenmark, S., Chirality, 15, 409-422 (2003). This suggests that a dimer could be located in one or more quadruplex grooves since the groove provides a geometric constraint that is not present in quadruplex end stacked complexes.

One drug design strategy involving groove-binding related to the compounds of the presently disclosed subject matter is to covalently link the two molecules of a groove-binding dimer together. This could increase the binding and selectivity for the target sequence greatly. Another strategy is to create molecules that combine end-stacking as well as groove-binding features. A groove binder could be linked to an end-stacking molecule, which would bind the quadruplex through a combination of binding modes, similar to the way ethidium bromide binds to duplex DNA. An extension of this approach is to use a groove-binding molecule of appropriate length and covalently attach an end-stacking molecule to each end, to create a "molecular clamp" which would bind to the telomeric DNA in a manner similar to that of duplex bisintercalators.

The addition of **8** to the human telomeric DNA also affects the CD signal in the wavelength region of DNA absorbance (Fig. 4). The DNA has a peak around 290 nm, with a smaller peak around 265 nm. The interpretation of CD spectra is based heavily on pattern recognition. Many parallel-type quadruplexes have a characteristic strong positive CD band at 264 nm and a negative band at 240 nm, whereas antiparallel quadruplexes usually have a positive band between 290 and 295 nm and a negative band at 260-265 nm. See Balagurumoorthy, P., et al., J. Biomol. Struc. Dyn., 8, 15 (1992); Balagurumoorthy, P., et al., Nuc. Acids. Res., 20, 4061-4067 (1992). Based on established CD pattern recognition, this suggests that the conformation of the human telomeric DNA sequence under these conditions can be a hybrid structure, having features of both parallel and anti-parallel structures, or can exist as a mixture of conformations in solution. Upon addition of **8,** the peak at 290 nm decreases, and the peak at 265 nm increases. This suggests that **8** is inducing or trapping out a parallel conformation of the DNA.

The CD spectrum for the human telomeric sequence has previously been shown to be different in the presence of sodium ions versus potassium ions. See Sen, D., and Gilbert, W., *Nature*, 344, 410 (1990). This suggests that this

DNA sequence exhibits a different conformation in the presence of each of these counterions. Differing NMR and crystal structures, obtained in the presence of sodium ions and potassium ions, respectively, support this ion-dependent conformation. The NMR structure of d[$AG_3(T_2AG_3)_3$] (SEQ ID NO: 1) in the presence of potassium ions is an antiparallel basket-like structure. See Wang, Y., and Patel, D., Structure, 1, 263-282 (1993). The crystal structure of this sequence obtained in the presence of sodium ions has a parallel, propeller-like structure. See Parkinson, G. N., et al., Nature, 417, 876-880 (2002). Other structures have been proposed to exist in the presence of potassium including an antiparallel chair-type structure (He. Y., et al., Nuc. Acids Res., 32, 5359-5367 (2004); Li, J., et al., Nuc. Acids Res., 33, 4649-4659 (2005); Qi, J., and Shafer, R., Nuc. Acids Res., 33, 3185-3192 (2005)) as well as a mixed parallel/antiparallel hybrid structure (Rezler, E. M., et al., J. Am. Chem. Soc., 127, 9439-9447 (2005)). See also Figure 1.

To investigate the effect of DNA structure on the binding of **8**, CD titrations were performed for the human telomeric DNA under different salt conditions to determine if **8** would bind as a dimer to different conformations of this DNA sequence. Figures 5a and 5b show the CD spectra for d[$AG_3(T_2AG_3)_3$] (SEQ ID NO: 1) titrated with **8** in the presence of 50 mM sodium, and 50 mM lithium, respectively. Lithium has been shown to have a destabilizing effect on quadruplex structure. See Sen. D., and Gilbert, W., Methods Enzymol., 211, 191-199 (1992). Regardless of the cation present, exciton splitting occurs and the 290 nm peak decreases and the 265 nm peak increases, as is the case when potassium is present. This suggests that regardless of the starting conformation of the DNA, **8** induces the formation of a parallel conformation of DNA to form an optimum binding site for the dimer. Only PIPER and telomestatin have been shown to induce quadruplex formation in human telomeric DNA. See Han, H., et al., Biochemistry, 38, 6981-6986 (1999) ; Phan, A. T., et al., Nature Chem. Biol., 1, 167-234 (2005).

CD spectra (Figures 6-11 and 13-17) were obtained for **8** with other DNA sequences of varying conformations as well as with human telomere sequences modified at different bases and of varying lengths (Table 1). The only sequences with which **8** exhibited exciton splitting are the *Tetrahymena* telomere and modified human telomere sequences. The CD spectrum of d[TGGGGT] (SEQ ID NO: 13) with **8** (Fig. 17), for example, does not show any induced CD or exciton splitting, suggesting that **8** might not be binding to this sequence. The *Tetrahymena* telomere has been shown by NMR to have a mixed parallel/antiparallel hybrid structure with one propeller loop and two lateral loops. This suggests that, if in fact the human telomere exists as a mixed hybrid quadruplex, **8** binds very selectively as a dimer to this type of quadruplex structure.

**Table 1.** Representative DNA Sequences

| DNA | Sequence | SEQ ID NO: | Structural Type |
|---|---|---|---|
| c-MYC promoter | d[$AG_3TG_4AG_3TG_4A$] | 1 | Parallel |
| Oxytricha Telomere | d[$G_4(T_4G_4)_3$] | 3 | Antiparallel basket |
| Tetrahymena Telomere | d[$(T_2G_4)_4$] | 4 | Mixed parallel/anti-parallel hybrid |
| GC7 | d[$(GC)_7$] | 5 | Watson-Crick duplex |
| Duplex | d[GCGAATTCGC] | 6 | Watson-Crick duplex |
| Thrombin-Binding Aptamer | d[$G_2T_2G_2TGTG_2T_2G_2$] | 7 | Antiparallel chair |
| Modified Human Telomere | d[TAGGGUTAGGGT] | 9 | Parallel Hairpin dimer |
| Modified Human Telomere | d[TAGGGUUAGGGT] | 10 | Parallel Hairpin dimer |
| Modified Human Telomere | d[TAGGGUTAGGGU] | 11 | Parallel Hairpin dimer |
| Modified Human Telomere | d[TTAGGGT] | 12 | Parallel (intermolecular) |
| Oligo | d[TGGGGT] | 13 | Parallel (intermolecular) |

The CD data relating to the G-quadruplex binding of several compounds of Formulas (I-IV) are summarized in Table 2 below. Some compounds appeared to bind to the telomeric G-quadruplex DNA sequence d[$AG_3(T_2AG_3)_3$] (SEQ ID NO: 1), as evidenced by induced CD, but the spectra did not show exciton splitting. Relative strength of the induced CD is indicated by the number of (+) marks. Other compounds, including **8, 17, 49,** and **51,** induced exciton splitting, as indicated by one or more (+) marks in the right-hand column.

An absorbance titration was performed to monitor changes in the spectral properties of **8** when bound to human telomeric DNA. Prior to addition of the DNA, the absorbance peak of the **8** monomer appears at 370 nm. As the DNA is added, this monomer peak decreases in intensity and a new peak is formed at around 425 nm, corresponding to the formation of the dimer. See Figure 12. This bathochromic shift is indicative of the formation of a J-aggregate. In a J-aggregate, the transition dipole moments of the two chromophores within the dimer are oriented with a translational

offset. Electronic transitions to the lowest exciton state are allowed, resulting in the red shift of the absorbance maximum. See Jelley, E. E., Nature, 138, 1009 (1936); Schiebe, G., Angew. Chem., 50, 212 (1937). In the case of **8,** the offset of this dimer structure allows the terminal positively-charged amidines to have a larger distance between the amidines on the adjacent molecule, decreasing the electronic repulsion that would exist in an H-aggregate (non-offset) structure. The titration spectra also reveal an isosbestic point at 395 nm, indicative of a two-state transition. This suggests that the only species present in this system are the unbound monomer and the bound dimer.

Surface plasmon resonance (SPR) was used to quantitatively evaluate the interaction between **8** and human telomeric DNA. SPR is a powerful technique to monitor molecular reactions in real time and has been used previously to study the interaction of compounds with quadruplex DNA. The telomeric sequence, d[$AG_3(T_2AG_3)_3$] (SEQ ID NO: 1), was immobilized and a range of concentrations of **8** (2 nM to 10 $\mu$M) were injected to quantitatively evaluate the interaction with the DNA. A second oligonucleotide which forms a duplex hairpin, d[CGAATTCGTCTCCGAATTCG] (SEQ ID NO: 8), was immobilized onto a second flow cell to evaluate the selectivity of **8** for quadruplex DNA versus duplex DNA. Blank injections with running buffer also were performed, and the resulting sensorgrams were subtracted from the compounds' sensorgrams to obtain the final concentration-dependent graphs. The response values in the steady-state region of the sensorgrams were plotted versus the unbound concentration (Cr) of **8** in the flow solution. The stoichiometry of binding arose from the RU value approached with increasing **8** concentration. The significant difference in the limiting RU value for the human telomeric sequence and the duplex sequence defined the binding stoichiometry of 2:1 for the telomere and 1:1 for the duplex. The 2:1 stoichiometry of **8** with the human telomeric sequence is consistent with the CD and absorbance results that suggest binding as a dimer.

No large signal increases are observed at higher concentrations, which indicates compound aggregation is not observed. This type of aggregation can be observed with molecules, such as porphyrins and carbocyanines, which are capable of forming dimers and higher-ordered aggregates. The compound **8** binding site could provide a geometric size constraint that restricts the site to a dimer. If the **8** molecules were stacking externally on the end of the G-tetrad, additional molecules could stack onto the dimer, resulting in a larger aggregate, and a stoichiometry greater than 2:1 would result. It is more likely that groove-binding or intercalation binding modes would provide this type of restricted environment. Intercalation of a cofacial dimer would be extremely unlikely, however, due to the small distance between tetrads as compared to the size of the ligand. Therefore, groove-binding remains the most plausible model for binding of the **8** dimer to the human telomeric sequence.

SPR also was used to determine the binding affinities for **8** with both the human telomeric sequence as well as the duplex sequence (Fig. 18). The RU versus $C_f$ plots were fitted to binding models with one or two binding sites. In the case of the human telomeric sequence, $K_1 = 4.7 \times 10^5\ M^{-1}$ and $K_2 = 4.5 \times 10^4\ M^{-1}$. This binding affinity of **8** for human telomeric DNA also is supported by a $\Delta T_m$ of approximately 10 °C in HEPES buffer containing 50 mM KCI monitored at 295 nm (data not shown). For the duplex sequence, the binding constant for the single site was $K = 1.3 \times 10^5\ M^{-1}$, indicating an approximately 4-fold selectivity of **8** for the human telomeric quadruplex over the duplex DNA.

For **8** binding to the human telomeric sequence, the binding constant for the second binding site is an order of magnitude lower than the constant for the first site, indicating negative cooperativity. This appears to be in conflict with the CD spectra, which seem to exhibit positive cooperativity in the induced region. However, since exciton splitting only arises from interaction between dimer molecules, and not monomer binding, the exciton splitting would only be observed upon binding of the second molecule, which would give the appearance of positive cooperativity, even though the binding occurs with negative cooperativity as shown with SPR.

Fluorescence titration was performed by titrating DNA into a solution of **8.** A Scatchard plot of the data is shown in Figure 19. The stoichiometry can be determined graphically by the extrapolation of the curve in the initial region when r is small, and determining the point of intersection with the x-axis. In this case, the line intersects at around r = 2, indicating that the stoichiometry is two molecules of **8** per molecule of DNA, which is in agreement with the stoichiometry obtained from the SPR experiments. The downward curvature of the plot indicates that the binding occurs with negative cooperativity, which also agrees with the SPR data.

A fluorescence energy transfer study was conducted to determine the extent of energy transfer from the DNA bases to the bound compound **8** dimer. Stacking on the quadruplex ends or intercalation within the tetrads would allow energy transfer resulting in the appearance of a peak at 260 nm in the excitation spectrum due to the absorbance of the DNA. Upon addition of d[$AG_3(T_2AG_3)_3$] (SEQ ID NO: 1) to **8,** the fluorescence decreases across all wavelengths, and a peak at 260 nm does not appear (Fig. 20). This suggests that the dimer of **8** is located in a quadruplex groove, at an orientation which prevents energy transfer from the DNA.

NMR studies on the sequence D[TAGGGUTAGGGT] (SEQ ID NO: 9) in the presence of potassium have shown agreement with previous studies by others suggesting that the sequence forms dimeric hairpin type parallel structures. See Phan, A. T., and Patel, D., J. Am. Chem. Soc., 125, 15021-15027 (2003). Imino proton spectra of modified human telomeres show that the DNA can exist in both parallel (propeller) or anti-parallel (basket) forms and as the temperature is increased, the parallel form is favored. Titration studies of compound **8** with various modified human telomere sequences show that the compound binds very selectively to the parallel structure from a mixture of structures. Upon

titrating with **8,** the peak intensity corresponding to the antiparallel species decreases indicating that the compound binds to the parallel form and stabilizes it. See Figures 21, 22, and 23.

The uracil H5-H6 TOCSY spectrum of free d[TAGGGUUAGGGT] (SEQ ID NO: 10) (Fig. 24) shows the presence of a mixture of structures. The peak at 7.86 ppm corresponds to the $U_6$ H5-H6 crosspeak whereas the crosspeak at 7.80 ppm corresponds to the $U_7$ H5-H6 protons. These two crosspeaks constitute the major form whereas the other two crosspeaks at 7.7 and 7.5 ppm correspond to the $U_6$ and $U_7$ of the minor species. After titrating with **8,** the intensity of the two peaks from minor species, 7.7 and 7.5 ppm, decreases enormously and this also indicates that the compound is favoring a single structure.

A T-methyl region of the TOCSY spectrum of free d[TAGGGUUAGGGT] (SEQ ID NO: 10) (Fig. 25) shows the presence of a mixture of structures. Close observation of the peak at 7.5 ppm indicates that two peaks at this location overlap on one another and correspond to the $T_1$ and $T_{12}$ methyl protons of parallel species whereas the peak at 7.3 ppm corresponds to the minor (antiparallel) form. After adding compound **8,** one of the peaks shifts to the new position at 7.44 ppm whereas the other peak remains at 7.5 ppm. A TOCSY spectrum of Uracil H5-H6 protons of d[TAGGGUTAGGGU] (SEQ ID NO: 11) (Fig. 26a-c) shows that as the ratio of **8** is increased, the crosspeaks of the minor species disappear, indicating that the compound selectively binds to a single conformation.

Example 17

*In Vitro* Antiprotozoan Activity of Dicationic Polyaryls

*In vitro* antiprotozoal activities were measured following established protocols. See Ismail, M. A., et al., J. Med. Chem., 46, 4761-4769 (2003); Stephens, C. E., et al., Bioorg. Med. Chem. Lett., 13, 2065-2069 (2003) (*in vitro* assay against *Leishmania donovani*). The activities of furamidine and compounds **8**, **17**, **45-47**, **49**, and **51-53** against *Trypanosoma brucei rhodesiense (T. b. r.)*, and *Plasmodium falciparum (P. f.)* are shown in Table 3.

Example 18

*In Vivo* Antiprotozoan Activity of Dicationic Polyaryls

The activities of furamidine and compounds **8**, **17**, **45-47**, **49**, and **51-53** against the STIB 900 strain of *Trypanosoma brucei rhodesiense (T. b. r.)* in a mouse model also are shown in Table 3. Groups of four mice were infected intraperitoneally with 2 x $10^5$ bloodstream forms of *T. b. r.* STIB 900 which originates from a patient in Tanzania. On days 3, 4, 5, and 6 post-infection the experimental groups were treated with the drugs either by the intraperitoneal or for prodrugs by the oral route. Usually the highest tolerated dose was used which was determined in a pretoxicological experiment. Parasitemia of the mice was checked daily up to day 14 post-infection and thereafter 2 times per week up to day 60. One group of mice was not treated and acted as control. For relapsing mice, the day of death was recorded and the survival time determined.

**Table 2.** G-Quadruplex Affinity

| Compound | Structure | Induced CD (no exciton) | Induced CD (exciton) |
|---|---|---|---|
| furamidine | 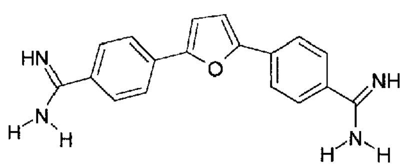 | - | - |
| **46** | 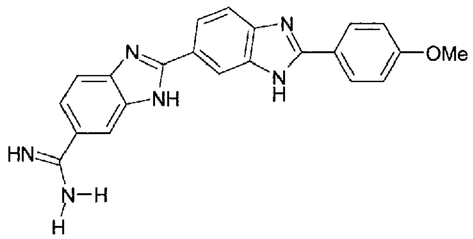 | ++++ | - |

(continued)

| Table 2. G-Quadruplex Affinity | | | |
|---|---|---|---|
| Compound | Structure | Induced CD (no exciton) | Induced CD (exciton) |
| **47** | 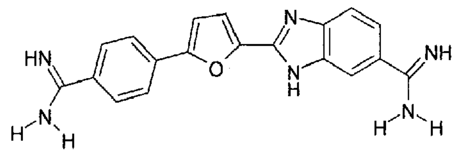 | +++ | - |
| **48** | 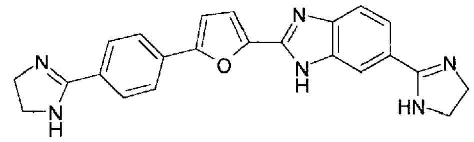 | ++++ | - |
| **49** | 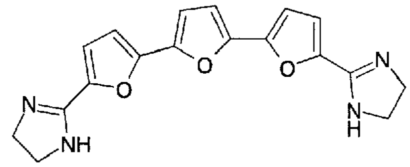 | - | ++ |
| **50** | 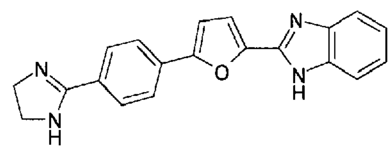 | +++ | - |
| **8** | 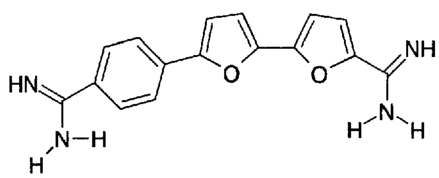 | - | +++++ |
| **17** | 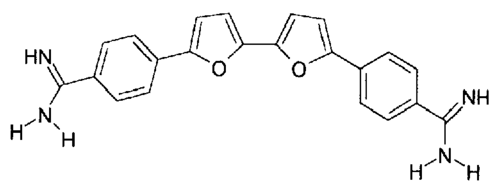 | - | ++++ |
| **51** | 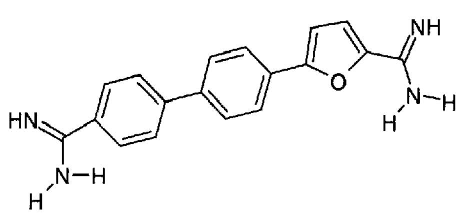 | - | + |
| **52** | 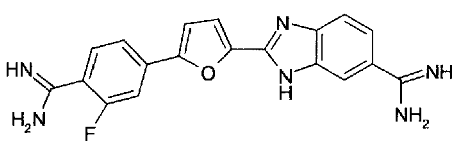 | +++ | - |

(continued)

| Table 2. G-Quadruplex Affinity | | | |
|---|---|---|---|
| Compound | Structure | Induced CD (no exciton) | Induced CD (exciton) |
| **45** | 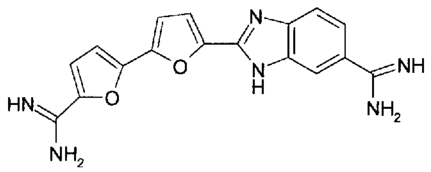 | ++ | - |
| **53** | 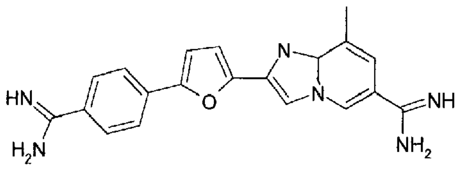 | +++ | - |

| Table 3. Antiprotozoan Data | | | | |
|---|---|---|---|---|
| Compound | Structure | T.b.r. ($IC_{50}$) nM | P.f. ($IC_{50}$)nM | In Vivo T.b.r. |
| furamidine | 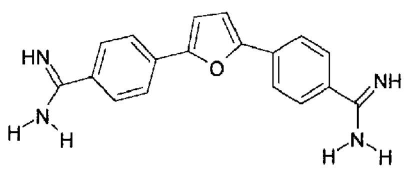 | 4.3 | 15.5 | 5 mg/kg ip 0/4/cures 46.5 d |
| **46** | 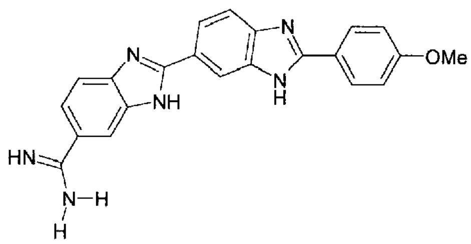 | 16 | 29 | 5 mg/kg ip 014 cures 14.75 d |
| **47** | 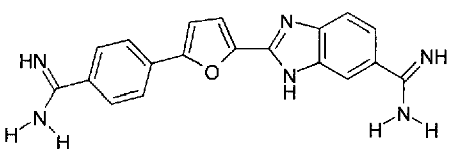 | 122 | 96 | 20 mg/kg ip 1/4 cures >27.75 d |
| **49** | 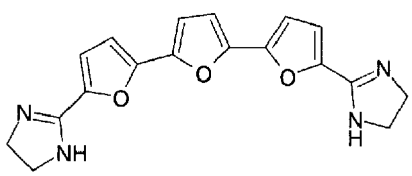 | 793 | 93 | NT |

(continued)

| Table 3. Antiprotozoan Data | | | | |
|---|---|---|---|---|
| Compound | Structure | T.b.r. ($IC_{50}$) nM | P.f. ($IC_{50}$)nM | In Vivo T.b.r. |
| **8** | 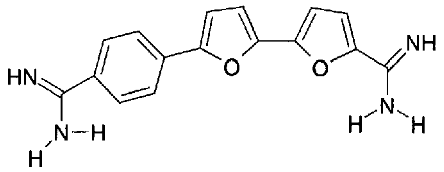 | 12 | 41.5 | 20 mg/kg ip 2/4 cures >45.5 d |
| **17** | 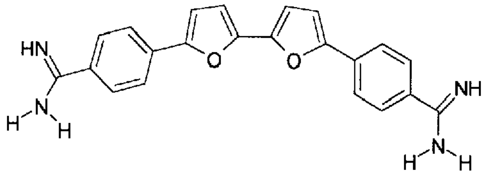 | 126 | 20 | 10 mg/kg ip 0/4 cures 24 d |
| **51** | 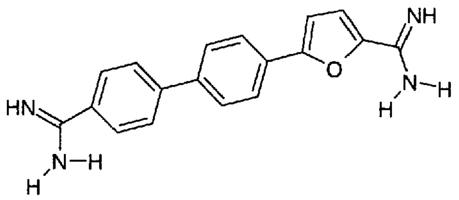 | 5 | 1 | 20 mg/kg ip |
| **52** | 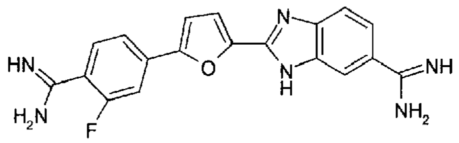 | 52.5 | 31 | 20 mg/kg ip 214 cures > 52 d |
| **45** | 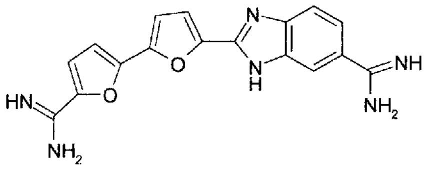 | 102 | 92 | NT |
| **53** | 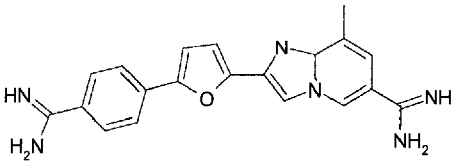 | 9 | 88 | 5 mg/kg ip 4/4 cures >60d |

It will be understood that various details of the presently disclosed subject matter can be changed without departing from the scope of the presently disclosed subject matter. Furthermore, the foregoing description is for the purpose of illustration only, and not for the purpose of limitation.

## Claims

**1.** A method of binding quadruplex deoxyribonucleic acid (DNA), the method comprising contacting quadruplex DNA with a compound of Formula (I):

$$Am_1\text{-}Ar_1\text{-}Ar_2\text{-}Ar_3\text{-}(Ar_4)_m\text{-}Am_2 \qquad (I)$$

wherein:

m is an integer from 0 to 1;
$Ar_1$. $Ar_2$, $Ar_3$, and $Ar_4$ are independently selected from the group consisting of:

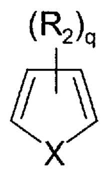 , 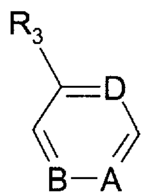 , 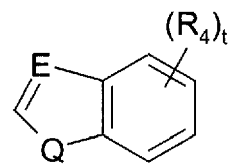 ,

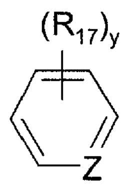 , and 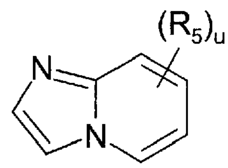 ,

wherein:

each X is independently selected from the group consisting of O, S, CH, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, aryloxyl, and substituted aryl;
each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;
each E is independently selected from the group consisting of $CR_{18}$ and N, wherein $R_{18}$ is selected from the group consisting of H, halo, hydroxyl, alkyl, alkoxyl, substituted alkyl, cycloalkyl, aryl, aryloxyl, and substituted aryl;
each Z is independently selected from the group consisting of $CR_{19}$ and N, wherein $R_{19}$ is selected from the group consisting of H, halo, hydroxyl, alkyl, alkoxyl, substituted alkyl, cycloalkyl, aryl, aryloxyl, and substituted aryl;
each q is independently an integer from 0 to 2;
each y is independently an integer from 0 to 3;
each t is independently an integer from 0 to 3;
each u is independently an integer from 0 to 3;
each $R_2$, $R_3$, $R_4$, $R_5$, and $R_{17}$ is independently selected from the group consisting of H, alkyl, substituted alkyl, cycloalkyl, halo, hydroxyl, alkoxyl, aryl, aryloxyl, substituted aryl, and aralkyloxyl;
$Am_1$ and $Am_2$ are each independently selected from the group consisting of:

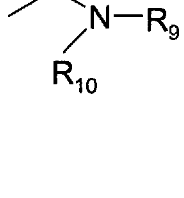 ; 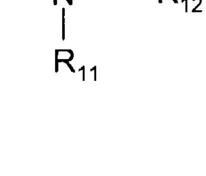 ; and 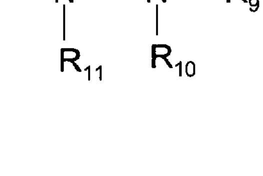 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

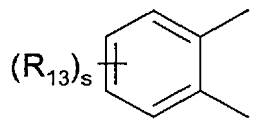
;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof.

**2.** The method of Claim 1 wherein at least one of $Ar_1$, $Ar_2$, $Ar_3$ and $Ar_4$

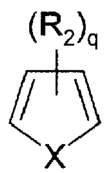
,

wherein:

X is selected from the group consisting of O, S, CH, Se, Te, and $NR_1$, wherein $R_1$ is selected from the group consisting of H, alkyl, substituted alkyl, cycloalkyl, aryl, and substituted aryl;
q is an integer from 0 to 2; and
each $R_2$ is independently selected from the group consisting of H, alkyl, substituted alkyl, cycloalkyl, halo, hydroxyl, alkoxyl, aryl, aryloxyl, substituted aryl, and aralkyloxyl.

**3.** The method of Claim 1, wherein the compound of Formula (I) comprises a compound having the following structure:

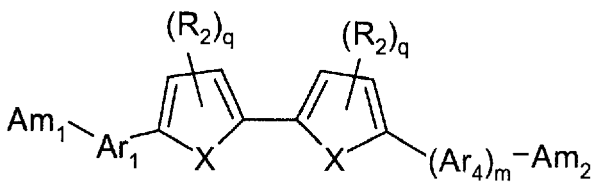
(II)

wherein:

m is an integer from 0 to 1;
$Ar_1$ and $Ar_4$ are independently selected from the group consisting of:

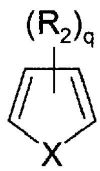 , 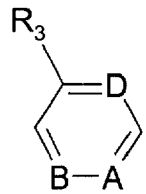 , 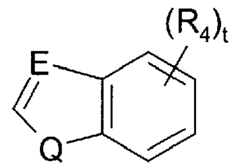 ,

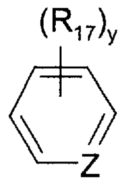 , and 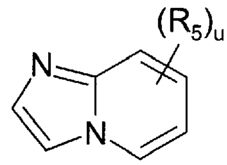 ,

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl;

each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;

each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;

each E is independently selected from the group consisting of $CR_{18}$ and N; wherein $R_{18}$ is selected from the group consisting of H, halo, hydroxyl, alkyl, alkoxyl, substituted alkyl, cycloalkyl, aryl, aryloxyl, and substituted aryl;

each Z is independently selected from the group consisting of $CR_{19}$ and N; wherein $R_{19}$ is selected from the group consisting of H, halo, hydroxyl, alkyl, alkoxyl, substituted alkyl, cycloalkyl, aryl, aryloxyl, and substituted aryl;

each q is independently an integer from 0 to 2;

each y is independently an integer from 0 to 3;

each t is independently an integer from 0 to 3;

each u is independently an integer from 0 to 3;

each $R_2$, $R_3$, $R_4$, $R_5$ and $R_{17}$ is independently selected from the group consisting of H, alkyl, substituted alkyl, aryl, substituted aryl, halo, hydroxyl, alkoxyl, aryloxyl, and aralkyloxyl;

$Am_1$ and $Am_2$ are each independently selected from the group consisting of:

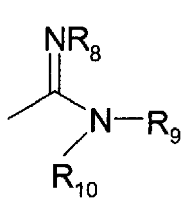 ; 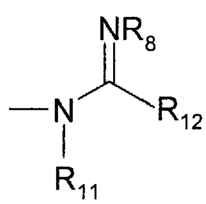 ; and 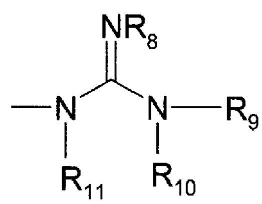 ;

wherein:

each $R_8$ is independently selected from the group consisting of H, hydroxyl, alkyl, substituted alkyl, aryl, substituted aryl, acyloxyl, and alkoxyl;

each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or

$R_8$ and $R_9$ or $R_8$ and $R_{12}$ together represent a $C_2$ to $c_{10}$ alkyl, $C_2$ to $C_{10}$ hydroxyalkyl, or $C_2$ to $C_{10}$ alkylene; or

$R_8$ and $R_9$ or $R_8$ and $R_{12}$ together are:

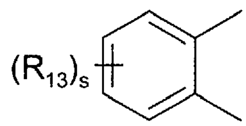

;

wherein s is an integer from 1 to 4, and $R_{13}$ is H or -CONHR$_{14}$NR$_{15}$R$_{16}$, wherein $R_{14}$ is alkyl, and $R_{15}$ and $R_{16}$ are each independently selected from the group consisting of H and alkyl;

or a pharmaceutically acceptable salt thereof.

**4.** The method of Claim 1, wherein the compound of Formula (I) comprises a compound of having the following structure:

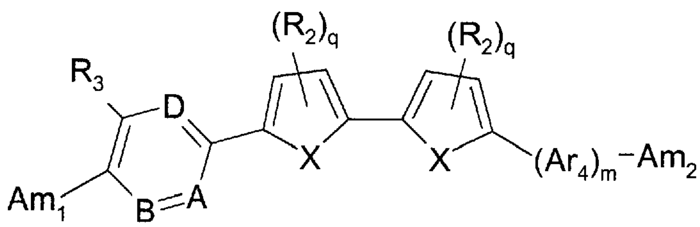

(III)

wherein:

m is selected from 0 and 1;
$Ar_4$ is selected from the group consisting of:

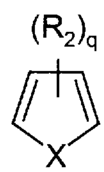

and

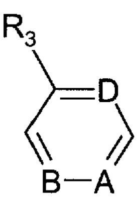

;

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;
each q is independently an integer from 0 to 2;
each $R_2$ and $R_3$ is independently selected from the group consisting of H, alkyl, substituted alkyl, cycloalkyl, aryl, substituted aryl, halo, hydroxyl, alkoxyl, aryloxyl, and aralkyloxyl;
$Am_1$ and $Am_2$ are each independently selected from the group consisting of:

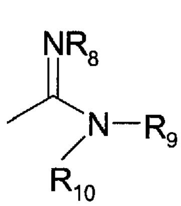

;

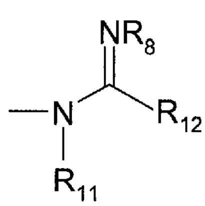

; and

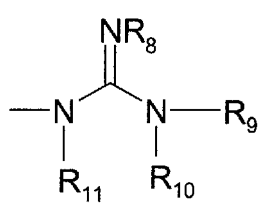

;

wherein:

each $R_8$ is independently selected from the group consisting of H, hydroxyl, alkyl, substituted alkyl, aryl, substituted aryl, acyloxyl, and alkoxyl;

each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or

$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or $R_8$ and $R_9$ or $R_8$ and $R_{12}$ together are:

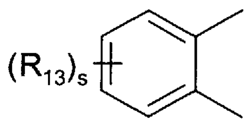

;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof.

**5.** The method of Claim 1, wherein the compound of Formula (I) is selected from the group consisting of:

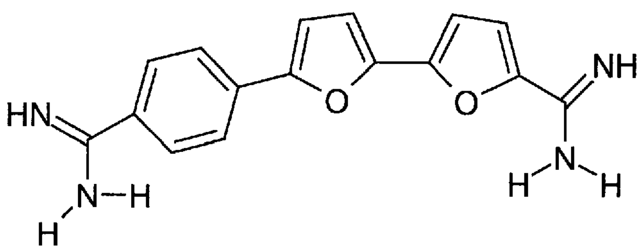

,

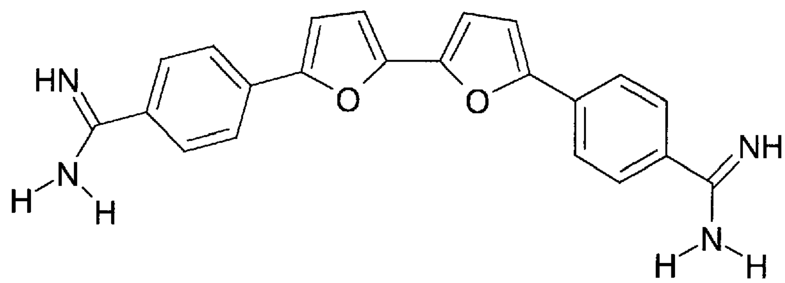

,

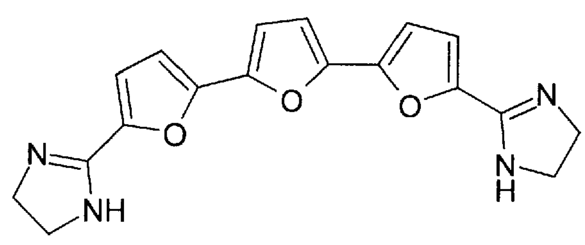

,

and

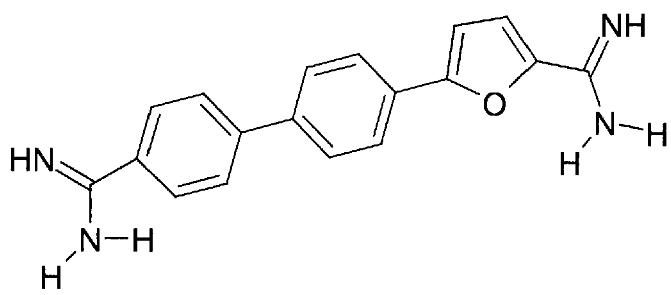

.

**6.** The method of Claim 1, comprising binding a dimer of a compound of Formula (I) in a groove of the quadruplex DNA.

**7.** The method of Claim 1, wherein the quadruplex DNA comprises a telomere.

**8.** The method of Claim 7, wherein the telomere is one of a human telomere, a nematodal telomere, and a protozoan telomere.

**9.** The method of Claim 8, wherein the protozoan telomere is one of a *Plasmodium* species, a *Trypanosoma* species, and a *Leishmania* species.

**10.** A method of reducing telomeric extension, the method comprising:

a) providing a compound of Formula (I):

$$Am_1\text{-}Ar_1\text{-}Ar_2\text{-}Ar_3\text{-}(Ar_4)_m\text{-}Am_2 \qquad (I)$$

wherein:

m is an integer from 0 to 1;
$Ar_1$, $Ar_2$, $Ar_3$ and $Ar_4$ are independently selected from the group consisting of:

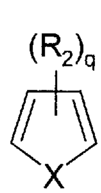 , 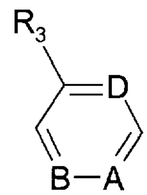 , 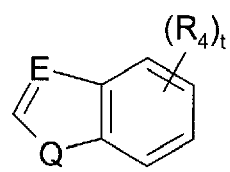 ,

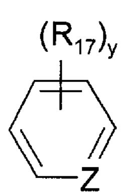 , and 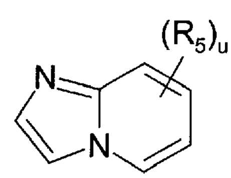 ,

wherein:

each X is independently selected from the group consisting of O, S, CH, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, aryloxyl, and substituted aryl;
each Q is independently selected from the group consisting of O, S, Se, Te, and $NR_7$, wherein $R_7$ is selected

from selected from the group consisting of H, alkyl, substituted alkyl, cycloalkyl, aryl, and substituted aryl;
each E is independently selected from the group consisting of $CR_{18}$ and N, wherein $R_{18}$ is selected from the group consisting of H, halo, hydroxyl, alkyl, alkoxyl, substituted alkyl, cycloalkyl, aryl, aryloxyl, and substituted aryl;
each Z is independently selected from the group consisting of $CR_{19}$ and N, wherein $R_{19}$ is selected from the group consisting of H, halo, hydroxyl, alkyl, alkoxyl, substituted alkyl, cycloalkyl, aryl, aryloxyl, and substituted aryl;
each q is independently an integer from 0 to 2;
each y is independently an integer from 0 to 3;
each t is independently an integer from 0 to 3;
each u is independently an integer from 0 to 3;
each $R_2$, $R_3$, $R_4$, $R_5$, and $R_{17}$ is independently selected from the group consisting of H, alkyl, substituted alkyl, cycloalkyl, halo, hydroxyl, alkoxyl, aryl, aryloxyl, substituted aryl, and aralkyloxyl;
$Am_1$ and $Am_2$ are each independently selected from the group consisting of:

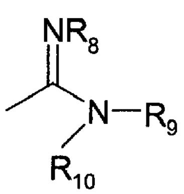 ; 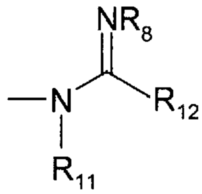 ; and 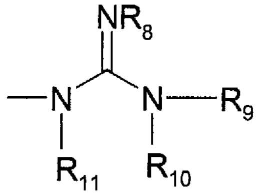 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or $R_8$ and $R_9$ or $R_8$ and $R_{12}$ together are:

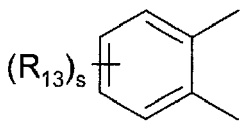 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof; and
b) contacting the compound of Formula (I) with telomeric DNA in the presence of telomerase, wherein the compound of Formula (I) is in an amount effective to stabilize and maintain the telomeric DNA in a quadruplex secondary structure, wherein the ability of telomerase to bind to the telomeric DNA is inhibited, thereby reducing telomeric extension.

**11.** The method of Claim 10, wherein the compound of Formula (I) comprises a compound having the following structure:

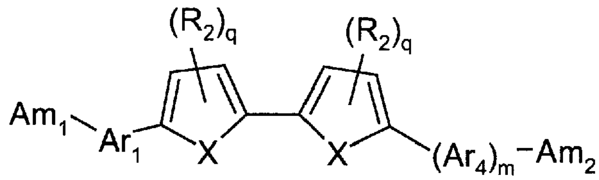

(II)

wherein:

m is an integer from 0 to 1;

$Ar_1$ and $Ar_4$ are independently selected from the group consisting of:

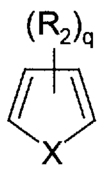

,

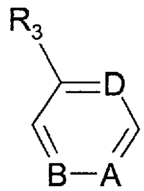

,

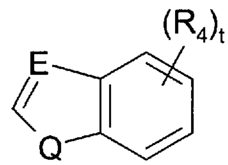

,

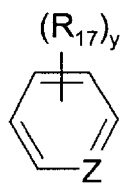

, and

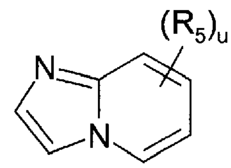

,

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl;

each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;

each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;

each E is independently selected from the group consisting of $CR_{18}$ and N; wherein $R_{18}$ is selected from the group consisting of H, halo, hydroxyl, alkyl, alkoxyl, substituted alkyl, cycloalkyl, aryl, aryloxyl, and substituted aryl;

each Z is independently selected from the group consisting of $CR_{19}$ and N; wherein $R_{19}$ is selected from the group consisting of H, halo, hydroxyl, alkyl, alkoxyl, substituted alkyl, cycloalkyl, aryl, aryloxyl, and substituted aryl;

each q is independently an integer from 0 to 2;

each y is independently an integer from 0 to 3;

each t is independently an integer from 0 to 3;

each u is independently an integer from 0 to 3;

each $R_2$, $R_3$, $R_4$, $R_5$ and $R_{17}$ is independently selected from the group consisting of H, alkyl, substituted alkyl, aryl, substituted aryl, halo, hydroxyl, alkoxyl, aryloxyl, and aralkyloxyl;

$Am_1$ and $Am_2$ are each independently selected from the group consisting of:

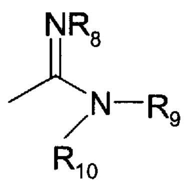 ; 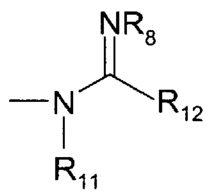 ; and 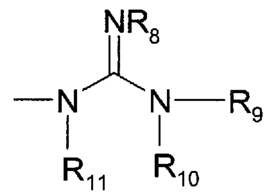 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or $R_8$ and $R_9$ or $R_8$ and $R_{12}$ together are:

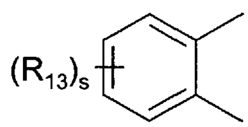 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof.

**12.** The method of Claim 10, wherein the compound of Formula (I) comprises a compound having the following structure:

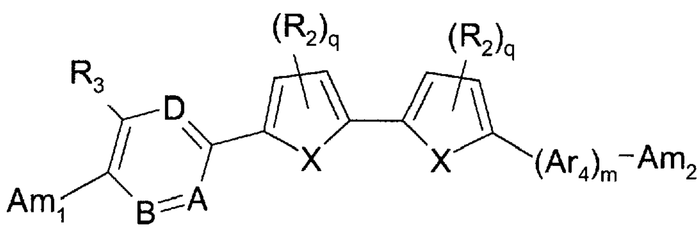 (III)

wherein:

m is selected from 0 and 1;
$Ar_4$ is selected from the group consisting of:

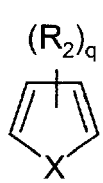 and 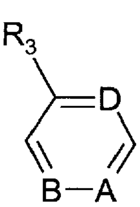 ;

wherein:

each X is independently selected from the group consisting of O, S, CH, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;
each q is independently an integer from 0 to 2;
each $R_2$ and $R_3$ is independently selected from the group consisting of H, alkyl, substituted alkyl, cycloalkyl, aryl, substituted aryl, halo, hydroxyl, alkoxyl, aryloxyl, and aralkyloxyl;
$Am_1$ and $Am_2$ are each independently selected from the group consisting of:

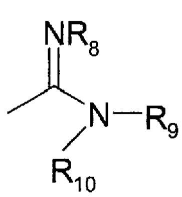 ; 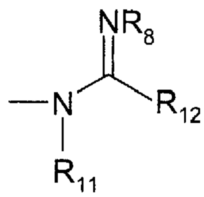 ; and 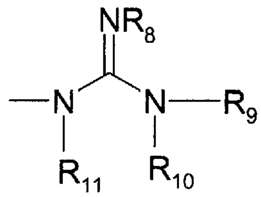 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

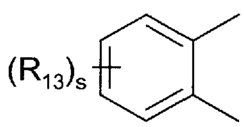 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof.

**13.** The method of Claim 10, wherein the compound of Formula (I) is selected from the group consisting of:

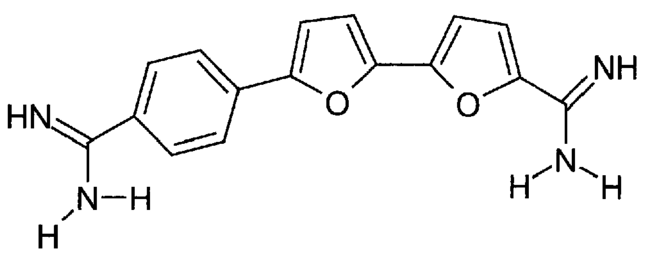 ,

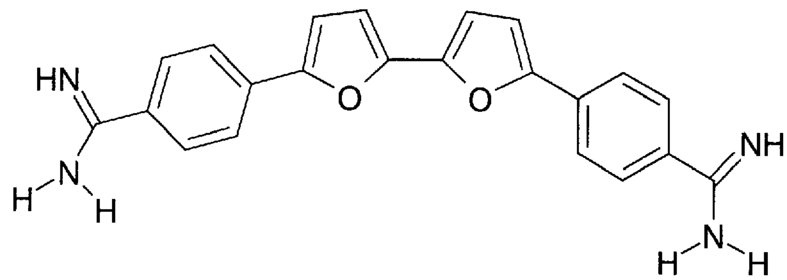

,

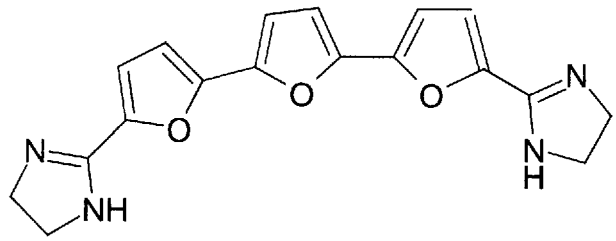

,

and

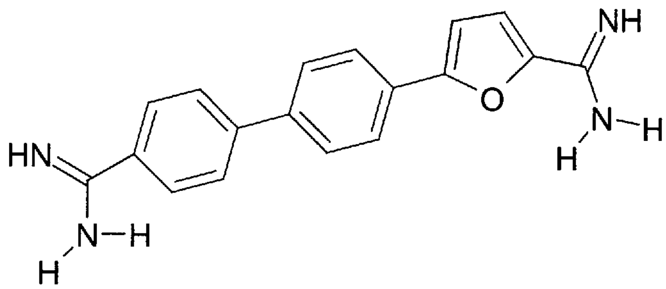

.

**14.** A method of reducing the proliferative capacity in a cell comprising contacting the cell with an effective amount of a compound of Formula (I):

$$Am_1\text{-}Ar_1\text{-}Ar_2\text{-}Ar_3\text{-}(Ar_4)_m\text{-}Am_2 \qquad (I)$$

wherein:

m is an integer from 0 to 1;
$Ar_1$, $Ar_2$, $Ar_3$, and $Ar_4$ are independently selected from the group consisting of:

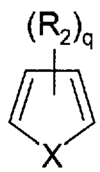

,

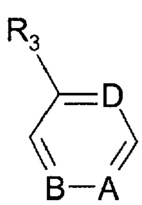

,

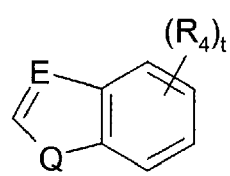

,

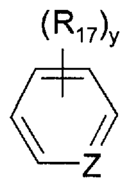
, and
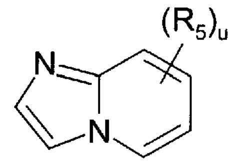
,

wherein:

each X is independently selected from the group consisting of O, S, CH, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, aryloxyl, and substituted aryl;
each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;
each E is independently selected from the group consisting of $CR_{18}$ and N, wherein $R_{18}$ is selected from the group consisting of H, halo, hydroxyl, alkyl, alkoxyl, substituted alkyl, cycloalkyl, aryl, aryloxyl, and substituted aryl;
each Z is independently selected from the group consisting of $CR_{19}$ and N, wherein $R_{19}$ is selected from the group consisting of H, halo, hydroxyl, alkyl, alkoxyl, substituted alkyl, cycloalkyl, aryl, aryloxyl, and substituted aryl;
each q is independently an integer from 0 to 2;
each y is independently an integer from 0 to 3;
each t is independently an integer from 0 to 3;
each u is independently an integer from 0 to 3;
each $R_2$, $R_3$, $R_4$, $R_5$, and $R_{17}$ is independently selected from the group consisting of H, alkyl, substituted alkyl, cycloalkyl, halo, hydroxyl, alkoxyl, aryl, aryloxyl, substituted aryl, and aralkyloxyl;
$Am_1$ and $Am_2$ are each independently selected from the group consisting of:

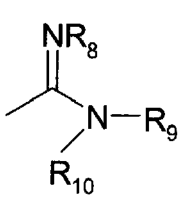
;
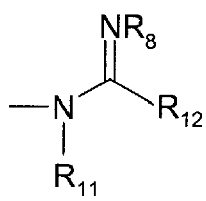
; and
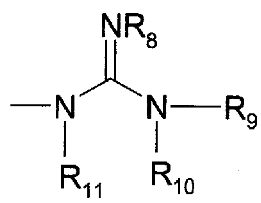
;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

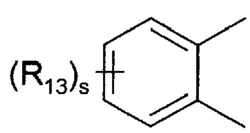
;

wherein s is an integer from 1 to 4, and $R_{13}$ is H or $-CONHR_{14}NR_{15}R_{16}$, wherein $R_{14}$ is alkyl, and $R_{15}$ and

$R_{16}$ are each independently selected from the group consisting of H and alkyl;

or a pharmaceutically acceptable salt thereof.

**15.** The method of Claim 14, wherein the compound of Formula (I) comprises a compound having the following structure:

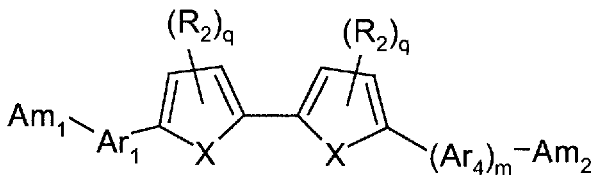

(II)

wherein:

m is an integer from 0 to 1;
$Ar_1$ and $Ar_4$ are independently selected from the group consisting of:

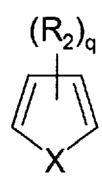
,
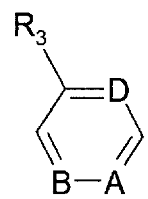
,
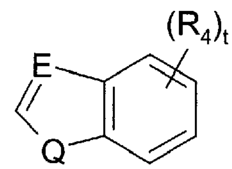
,

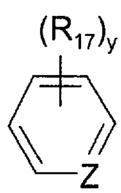
, and
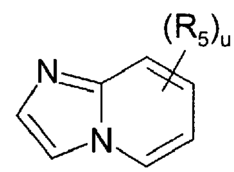
,

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;
each Q is independently selected from the group consisting of 0, S, Se, Te, and $NR_7$, wherein $R_7$ is selected from selected from the group consisting of H, alkyl, substituted alkyl, cycloalkyl, aryl, and substituted aryl;
each E is independently selected from the group consisting of $CR_{18}$ and N; wherein $R_{18}$ is selected from the group consisting of H, halo, hydroxyl, alkyl, alkoxyl, substituted alkyl, cycloalkyl, aryl, aryloxyl, and substituted aryl;
each Z is independently selected from the group consisting of $CR_{19}$ and N; wherein $R_{19}$ is selected from the group consisting of H, halo, hydroxyl, alkyl, alkoxyl, substituted alkyl, cycloalkyl, aryl, aryloxyl, and substituted aryl;
each q is independently an integer from 0 to 2;
each y is independently an integer from 0 to 3;
each t is independently an integer from 0 to 3;
each u is independently an integer from 0 to 3;
each $R_2$, $R_3$, $R_4$, $R_5$ and $R_{17}$ is independently selected from the group consisting of H, alkyl, substituted

alkyl, aryl, substituted aryl, halo, hydroxyl, alkoxyl, aryloxyl, and aralkyloxyl;
$Am_1$ and $Am_2$ are each independently selected from the group consisting of:

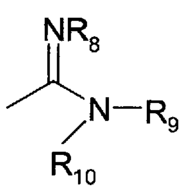 ; 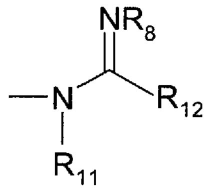 ; and 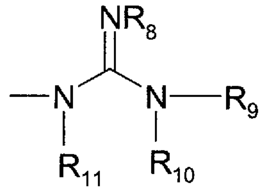 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or $R_8$ and $R_9$ or $R_8$ and $R_{12}$ together are:

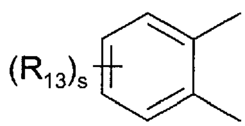 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof.

**16.** The method of Claim 14, wherein the compound of Formula (I) comprises a compound having the following structure:

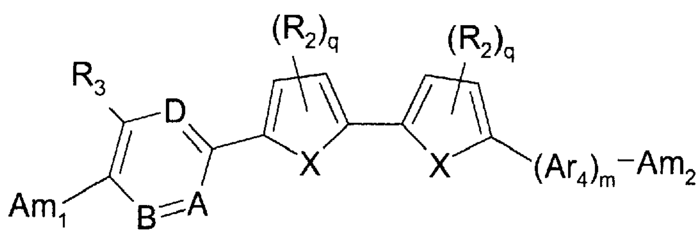

(III)

wherein:

m is selected from 0 and 1;
$Ar_4$ is selected from the group consisting of:

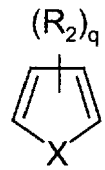 and 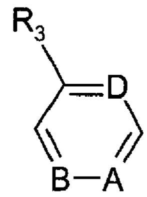 ;

wherein:

each X is independently selected from the group consisting of O, S, CH, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, and substituted aryl;
each q is independently an integer from 0 to 2;
each $R_2$ and $R_3$ is independently selected from the group consisting of H, alkyl, substituted alkyl, cycloalkyl, aryl, substituted aryl, halo, hydroxyl, alkoxyl, aryloxyl, and aralkyloxyl;
$Am_1$ and $Am_2$ are each independently selected from the group consisting of:

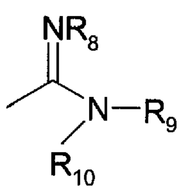 ; 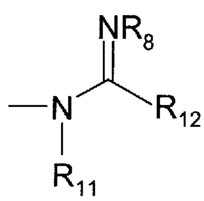 ; and 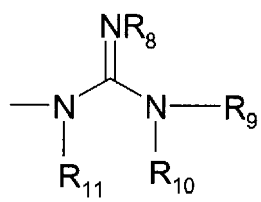 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

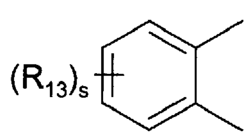 ;

wherein s is an integer from 1 to 4, and $R_{13}$ is H or- $CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof.

**17.** The method of Claim 14, wherein the compound of Formula (I) is selected from the group consisting of:

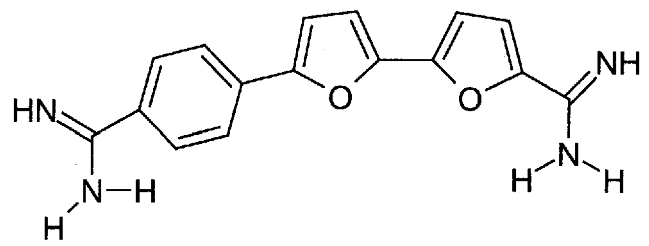

,

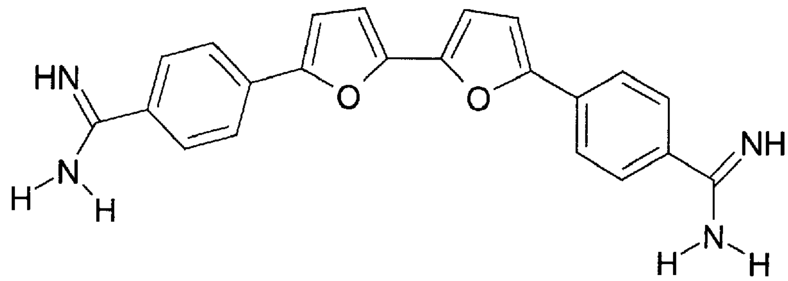

,

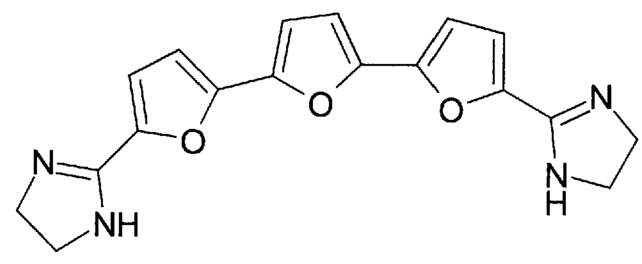

,

and

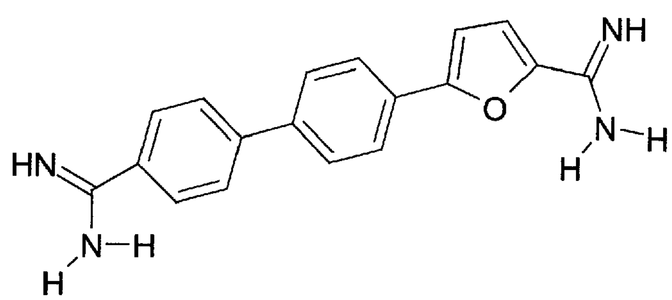

.

**18.** The method of Claim 14, wherein the cell comprises a mammalian cell.

**19.** The method of Claim 14, wherein the cell comprises a human cell.

**20.** The method of Claim 14, wherein the cell comprises a cancer cell.

**21.** The method of Claim 20, wherein the cancer cell is selected from the group consisting of a breast cancer cell, a prostate cancer cell, a liver cancer cell, a pancreatic cancer cell, a lung cancer cell, a brain cancer cell, an ovarian cancer cell, a uterine cancer cell, a testicular cancer cell, a skin cancer cell, a leukemia cell, a head and neck cancer cell, a colon cancer cell, a retinal cancer cell, a bladder cancer cell, an anal cancer cell, and a rectal cancer cell.

**22.** The method of Claim 14, wherein the compound promotes apoptosis.

**23.** The use of an effective amount of a compound of Formula (I):

$$Am_1\text{-}Ar_1\text{-}Ar_2\text{-}Ar_3\text{-}(Ar_4)_m\text{-}Am_2 \qquad (I)$$

wherein:

m is an integer from 0 to 1;
$Ar_1$, $Ar_2$, $Ar_3$, and $Ar_4$ are independently selected from the group consisting of:

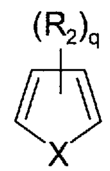
,
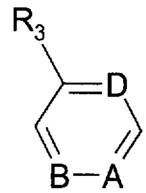
,
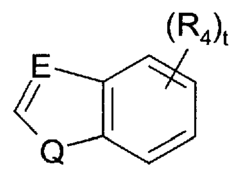
,

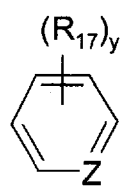
, and
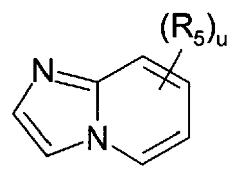
,

wherein:

each X is independently selected from the group consisting of O, S, CH, Se, Te, and $NR_1$, wherein $R_1$ is selected from the group consisting of H, alkyl, substituted alkyl, cycloalkyl, aryl, and substituted aryl;
each A, B, and D is independently selected from the group consisting of $CR_6$ and N, wherein $R_6$ is selected from the group consisting of H, halo, hydroxyl, alkyl, alkoxyl, substituted alkyl, cycloalkyl, aryl, aryloxyl, and substituted aryl;
each Q is independently selected from the group consisting of O, S, Se, Te, and $NR_7$, wherein $R_7$ is selected from selected from the group consisting of H, alkyl, substituted alkyl, cycloalkyl, aryl, and substituted aryl;
each E is independently selected from the group consisting of $CR_{18}$ and N, wherein $R_{18}$ is selected from the group consisting of H, halo, hydroxyl, alkyl, alkoxyl, substituted alkyl, cycloalkyl, aryl, aryloxyl, and substituted aryl;
each Z is independently selected from the group consisting of $CR_{19}$ and N, wherein $R_{19}$ is selected from the group consisting of H, halo, hydroxyl, alkyl, alkoxyl, substituted alkyl, cycloalkyl, aryl, aryloxyl, and substituted aryl;
each q is independently an integer from 0 to 2;
each y is independently an integer from 0 to 3;
each t is independently an integer from 0 to 3;
each u is independently an integer from 0 to 3;
each $R_2$, $R_3$, $R_4$, $R_5$, and $R_{17}$ is independently selected from the group consisting of H, alkyl, substituted alkyl, cycloalkyl, halo, hydroxyl, alkoxyl, aryl, aryloxyl, substituted aryl, and aralkyloxyl;
$Am_1$ and $Am_2$ are each independently selected from the group consisting of:

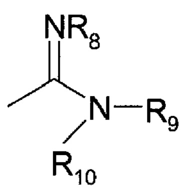 ; 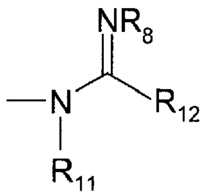 ; and 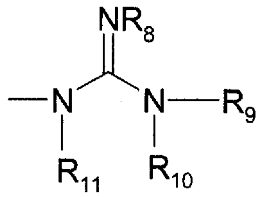 ;

wherein:

each $R_8$ is independently selected from the group consisting of H, hydroxyl, alkyl, substituted alkyl, aryl, substituted aryl, acyloxyl, and alkoxyl;

each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or

$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or $R_8$ and $R_9$ or $R_8$ and $R_{12}$ together are:

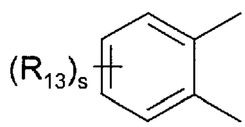 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof, for treating a cancer in a subject in need of treatment thereof.

**24.** The use of Claim 23, comprising administering to the subject one or more additional therapeutic compounds.

**25.** A compound of the following structure:

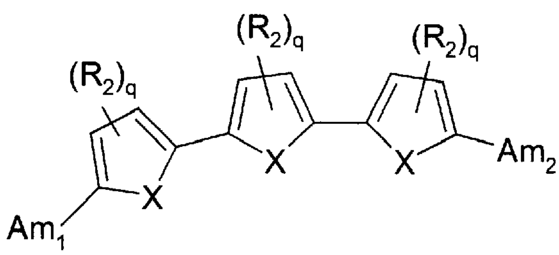

(IV)

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl, provided that at least one X is selected from Se, Te, and $NR_1$, wherein $R_1$ is selected from aryl and substituted aryl;

each q is independently an integer from 0 to 2;

each $R_2$ is independently selected from the group consisting of H, alkyl, substituted alkyl, cycloalkyl, aryl, substituted aryl, halo, hydroxyl, alkoxyl, aryloxyl, and aralkyloxyl;

$Am_1$ and $Am_2$ are each independently selected from the group consisting of:

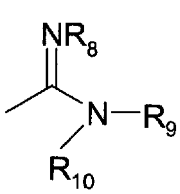 ; 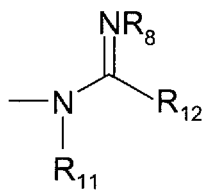 ; and 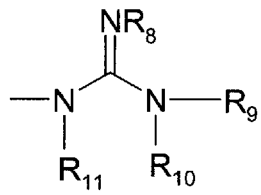 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

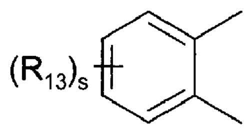 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof.

**26.** A pharmaceutical formulation comprising a compound of the following Formula:

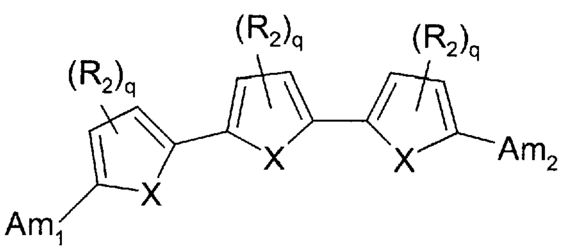 (IV)

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl, provided that at least one X is selected from Se, Te, and $NR_1$, wherein $R_1$ is selected from aryl and substituted aryl;
each q is independently an integer from 0 to 2;
each $R_2$ is independently selected from the group consisting of H, alkyl, substituted alkyl, cycloalkyl, aryl, substituted aryl, halo, hydroxyl, alkoxyl, aryloxyl, and aralkyloxyl;
$Am_1$ and $Am_2$ are each independently selected from the group consisting of:

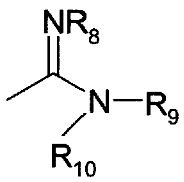 ; 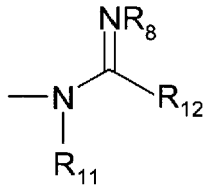 ; and 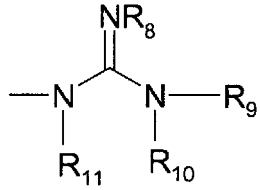 ;

wherein:

each $R_8$ is independently selected from the group consisting of H, hydroxyl, alkyl, substituted alkyl, aryl, substituted aryl, acyloxyl, and alkoxyl;
each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or
$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or
$R_8$ and $R_9$ or $R_8$ and $R_{12}$ together are:

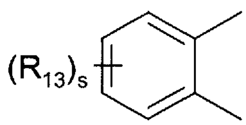 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof, in a pharmaceutically acceptable carrier.

**27.** The Use of an effective amount of a compound of Formula (IV):

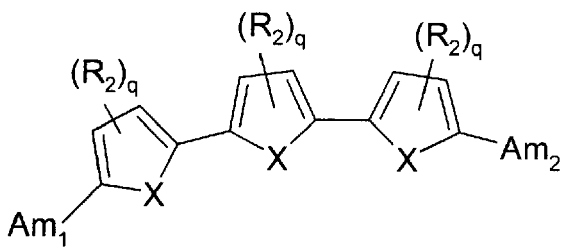 (IV)

wherein:

each X is independently selected from the group consisting of O, S, Se, Te, and $NR_1$, wherein $R_1$ is selected from the group consisting of H, alkyl, substituted alkyl, cycloalkyl, aryl, and substituted aryl, provided that at least one X is selected from Se, Te, and $NR_1$, wherein $R_1$ is selected from aryl and substituted aryl;
each q is independently an integer from 0 to 2;
each $R_2$ is independently selected from the group consisting of H, alkyl, substituted alkyl, cycloalkyl, aryl, substituted aryl, halo, hydroxyl, alkoxyl, aryloxyl, and aralkyloxyl;
$Am_1$ and $Am_2$ are each independently selected from the group consisting of:

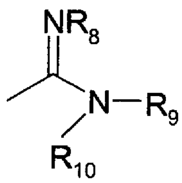 ; 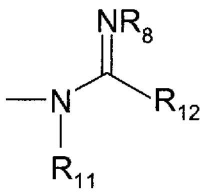 ; and 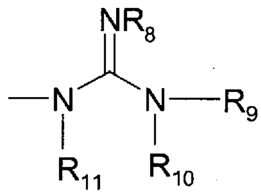 ;

wherein:

each $R_8$ is independently selected from the group consisting of H, hydroxyl, alkyl, substituted alkyl, aryl, substituted aryl, acyloxyl, and alkoxyl;

each $R_9$, $R_{10}$, $R_{11}$, and $R_{12}$ is independently selected from the group consisting of H, alkyl, substituted alkyl, cycloalkyl, aryl, substituted aryl, aralkyl, hydroxyl, alkoxyl, hydroxyalkyl, hydroxycycloalkyl, alkoxycycloalkyl, aminoalkyl, acyloxyl, alkylaminoalkyl, and alkoxycarbonyl; or

$R_8$ and $R_9$ or $R_8$ and $R_{12}$ together represent a $C_2$ to $C_{10}$ alkyl, $C_2$ to $C_{10}$ hydroxyalkyl, or $C_2$ to $C_{10}$ alkylene; or $R_8$ and $R_9$ or $R_8$ and $R_{12}$ together are:

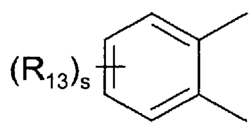 ;

wherein s is an integer from 1 to 4, and $R_{13}$ is H or $-CONHR_{14}NR_{15}R_{16}$, wherein $R_{14}$ is alkyl, and $R_{15}$ and $R_{16}$ are each independently selected from the group consisting of H and alkyl;

or a pharmaceutically acceptable salt thereof, for treating a microbial infection in a subject in need of treatment thereof.

**28.** The use of Claim 27, wherein the microbial infection is a protozoal infection.

**29.** The use of Claim 28, wherein the protozoal infection is caused by a species selected from the group consisting of *Trypanosoma* spp., *Plasmodium* spp., and *Leishmania* spp.

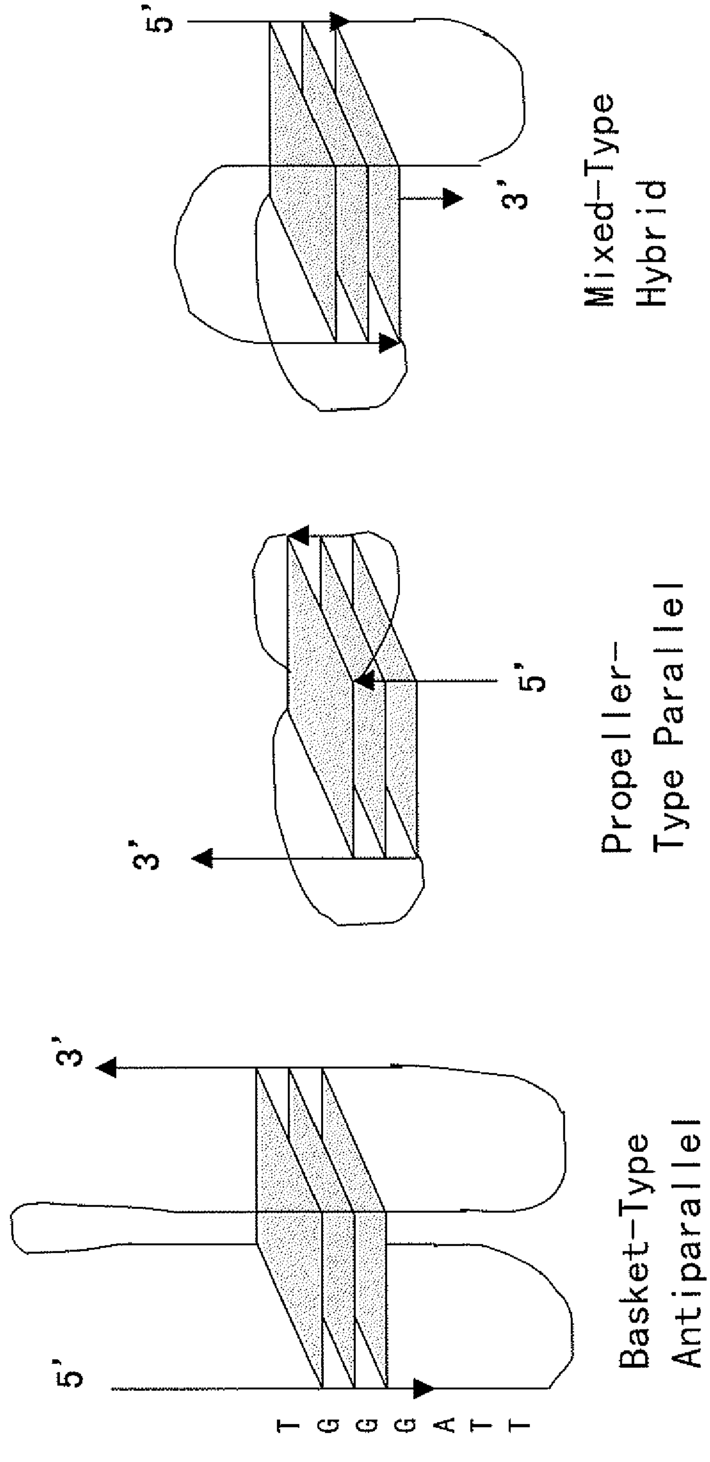
5'
3'
T G G G A T T
Basket-Type
Antiparallel
3'
5'
Propeller-
Type Parallel
5'
3'
Mixed-Type
Hybrid

***Fig 1***

**2A**

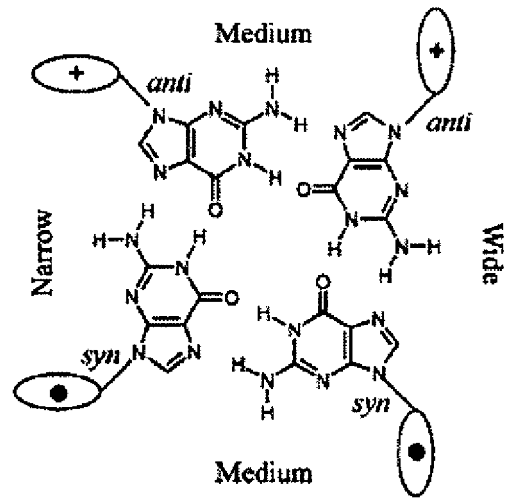


**2B**

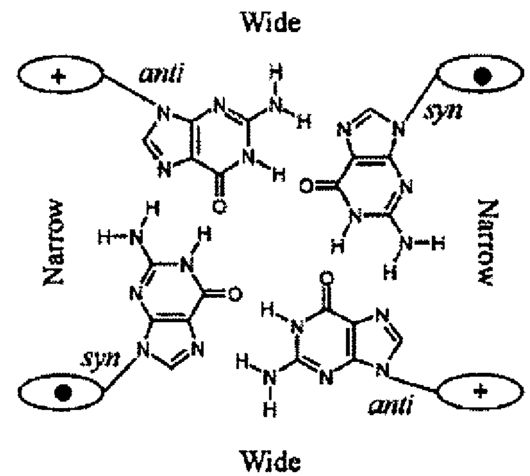


Reproduced from Simonsson, T., *Biol. Chem.*, 382, 621-628 (2001)

**Fig 2**

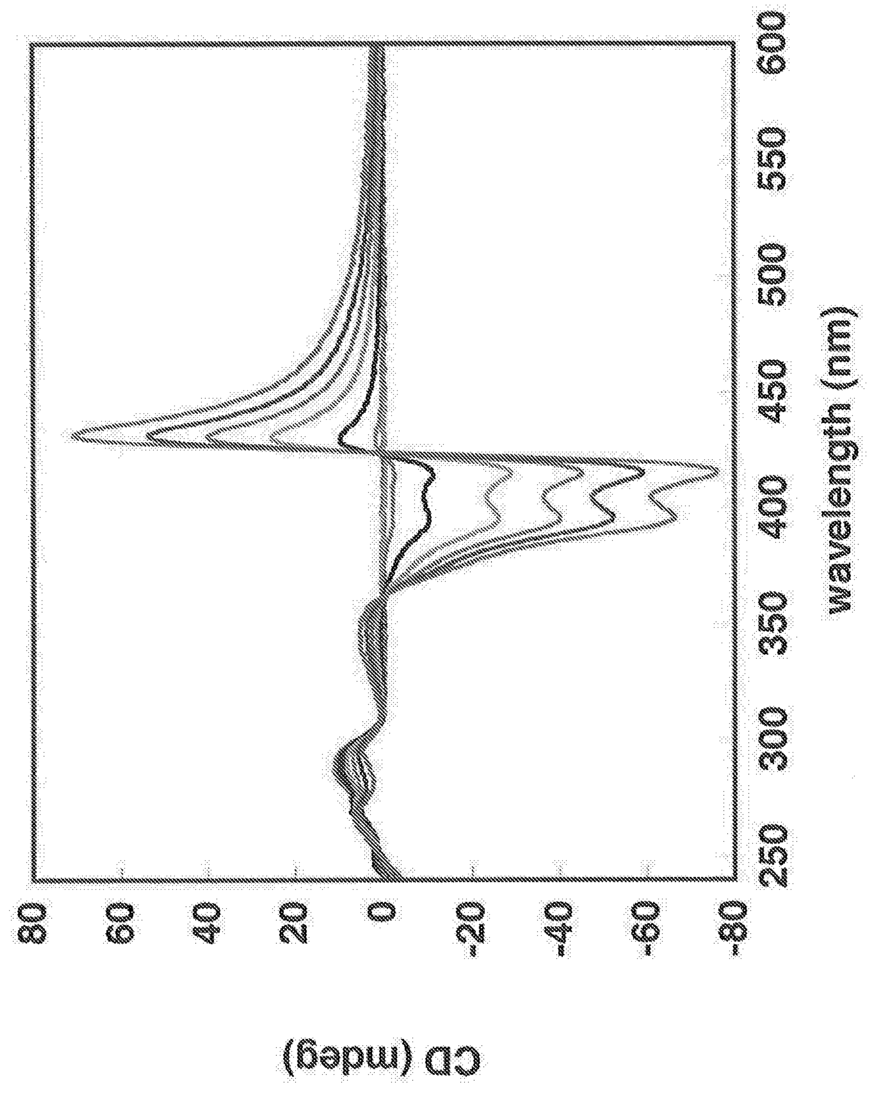
CD (mdeg)
80
60
40
20
0
-20
-40
-60
-80
250
300
350
400
450
500
550
600
wavelength (nm)

*Fig 3*

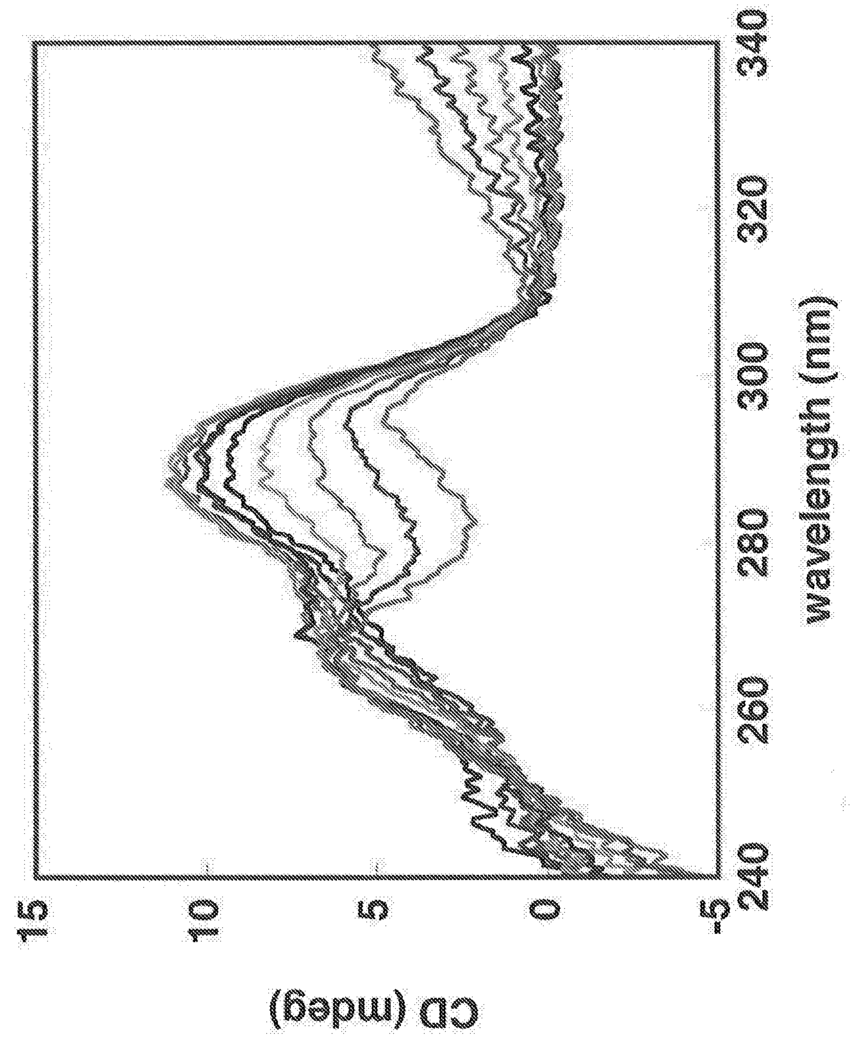


Fig 4

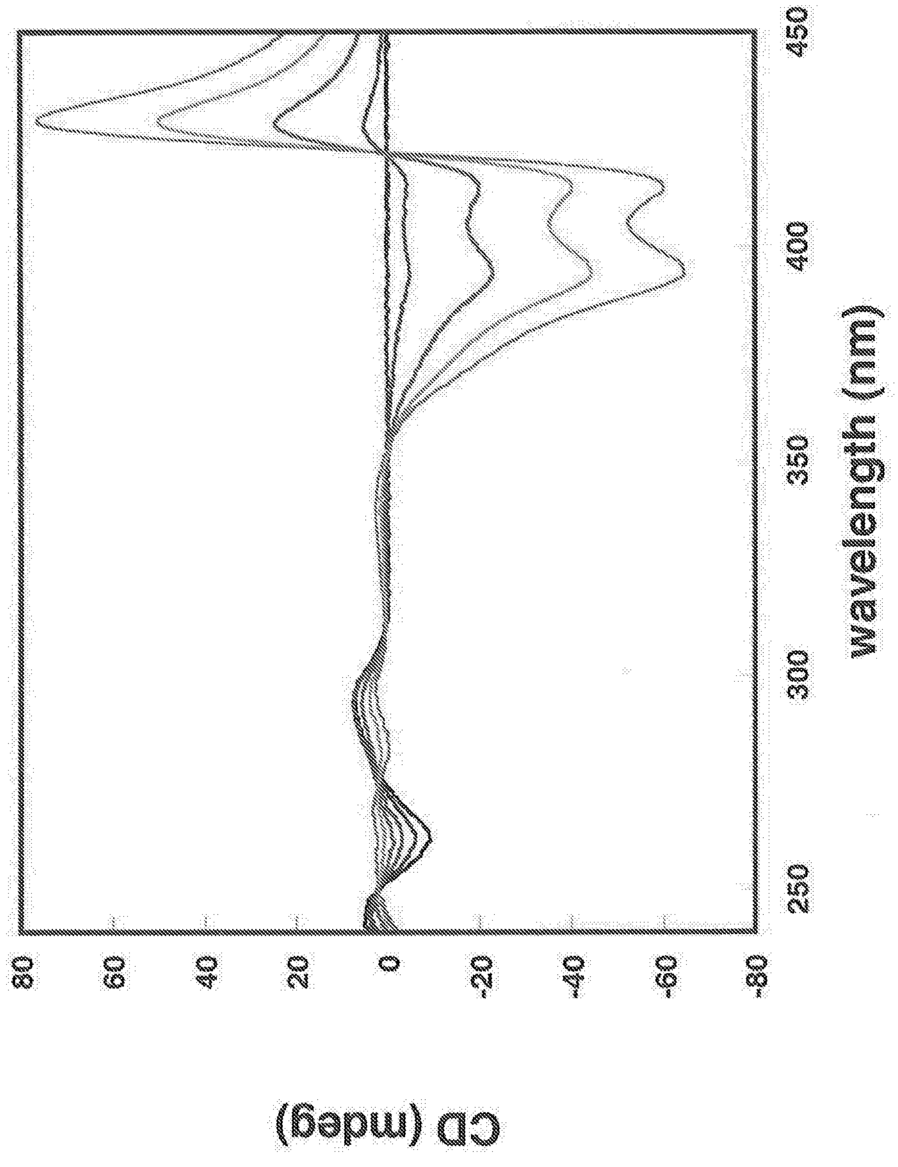
CD (mdeg)
80
60
40
20
0
-20
-40
-60
-80
250
300
350
400
450
wavelength (nm)

Fig 5A

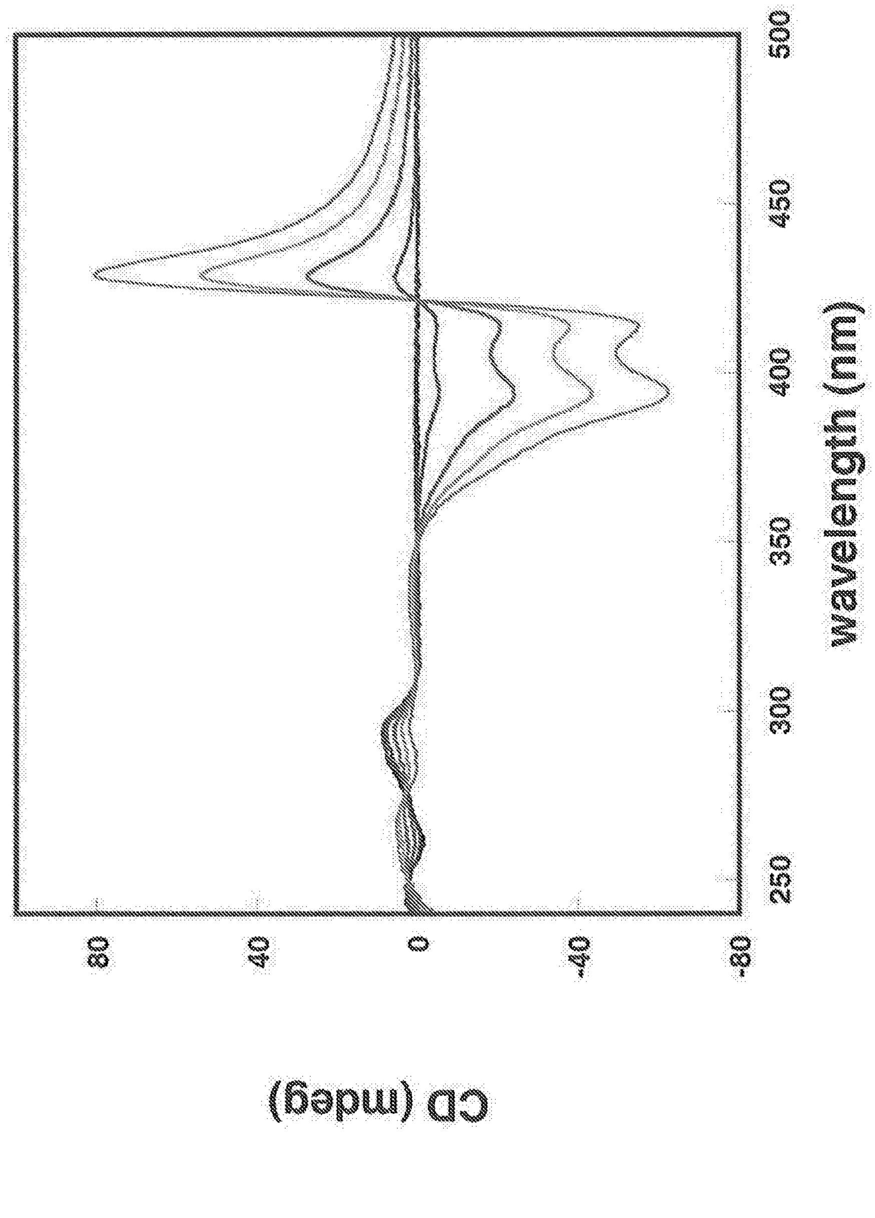


Fig 5B

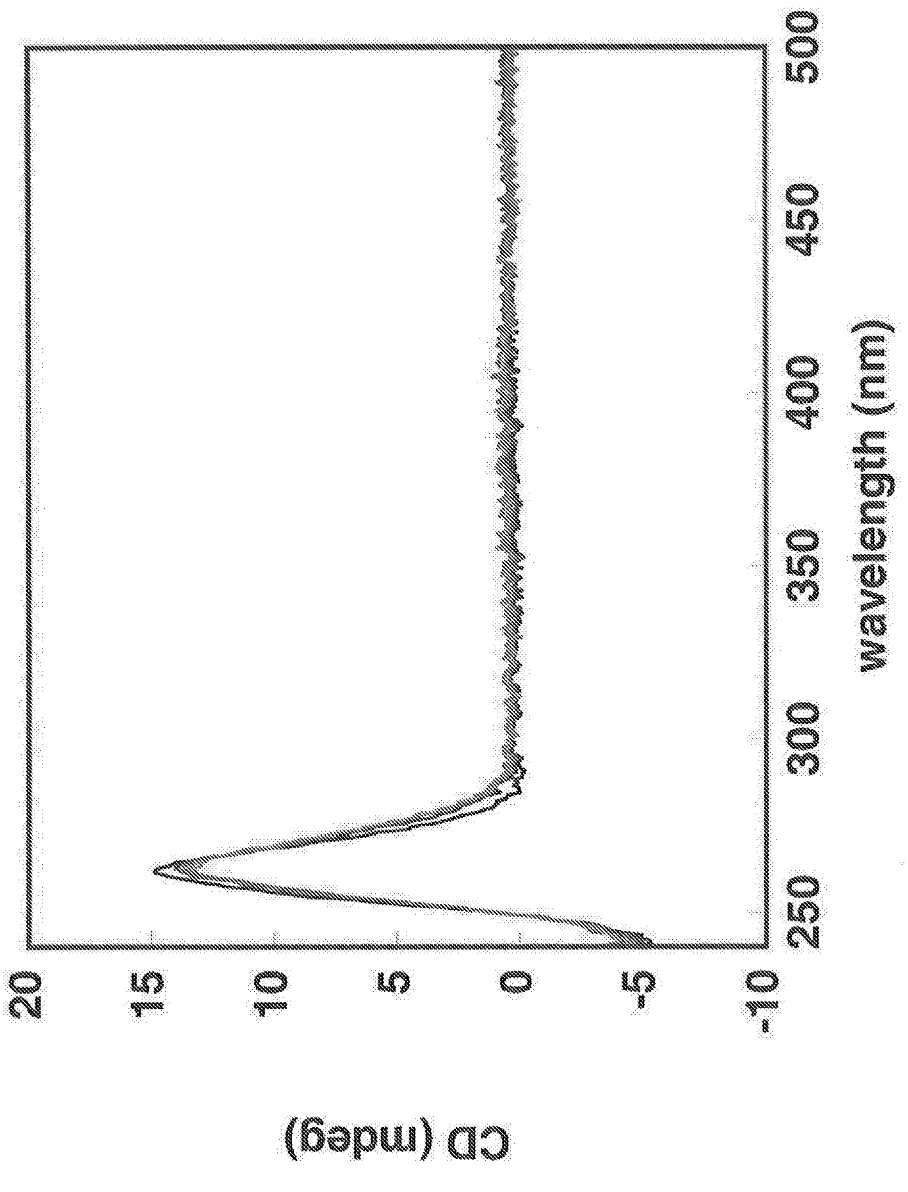


Fig 6

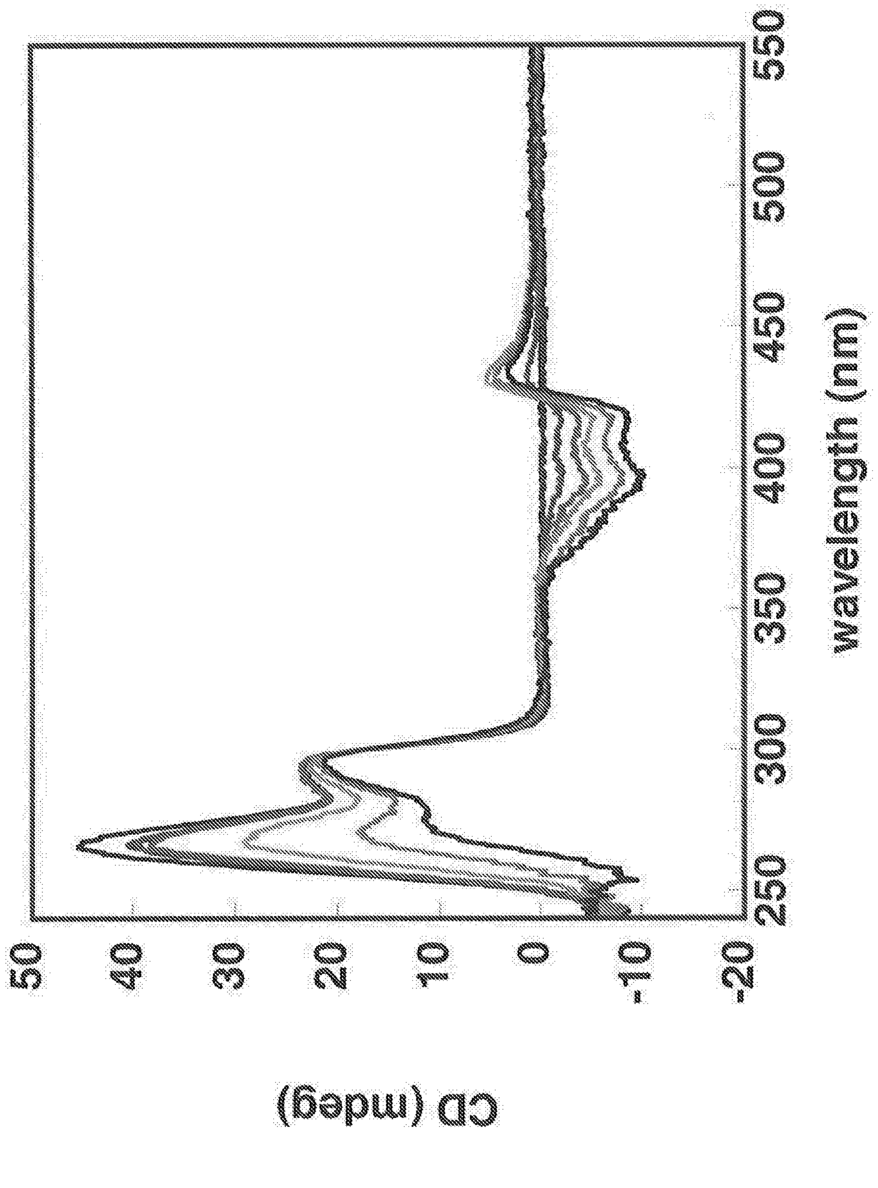
CD (mdeg)
50
40
30
20
10
0
-10
-20
250
300
350
400
450
500
550
wavelength (nm)

Fig 7

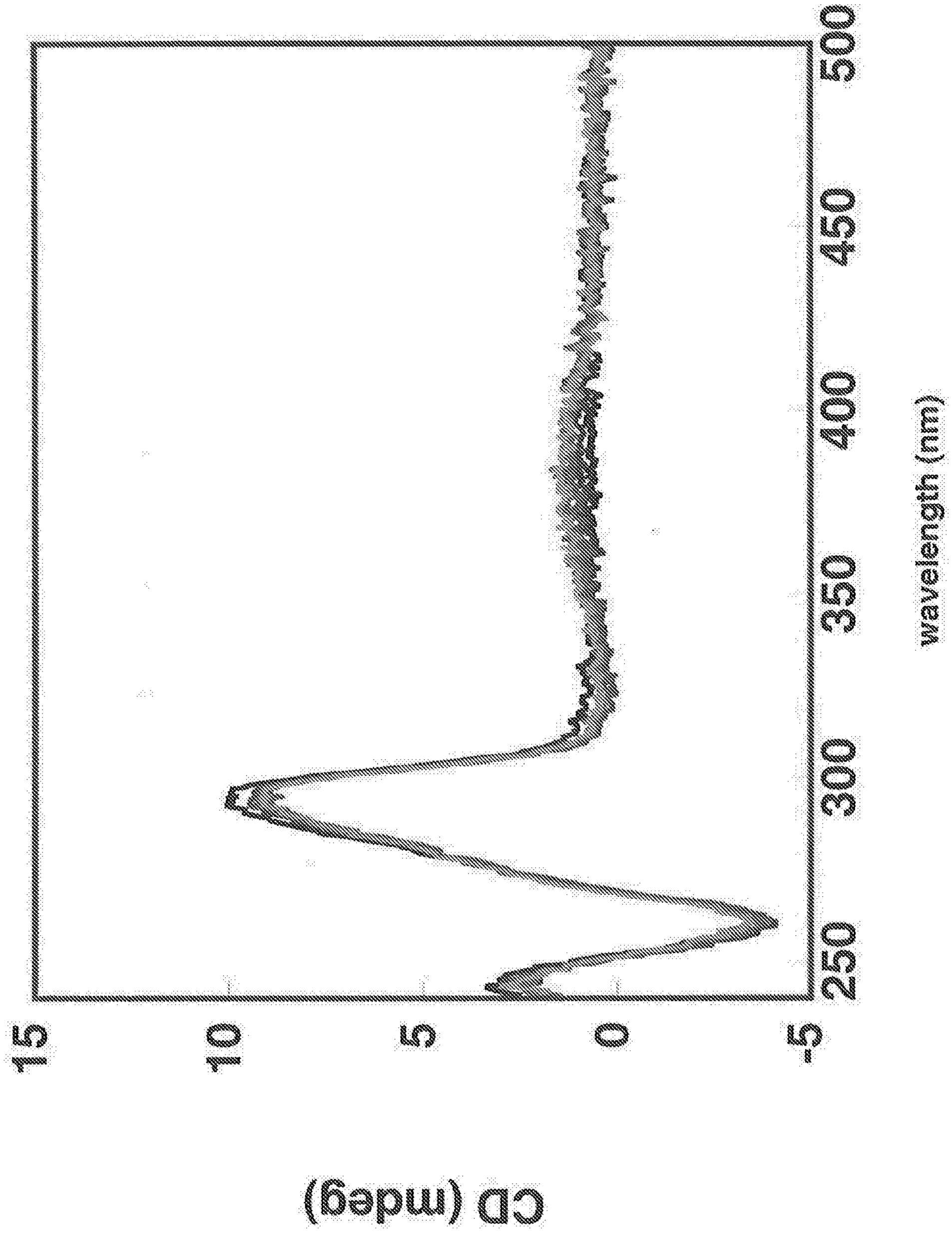


Fig 8

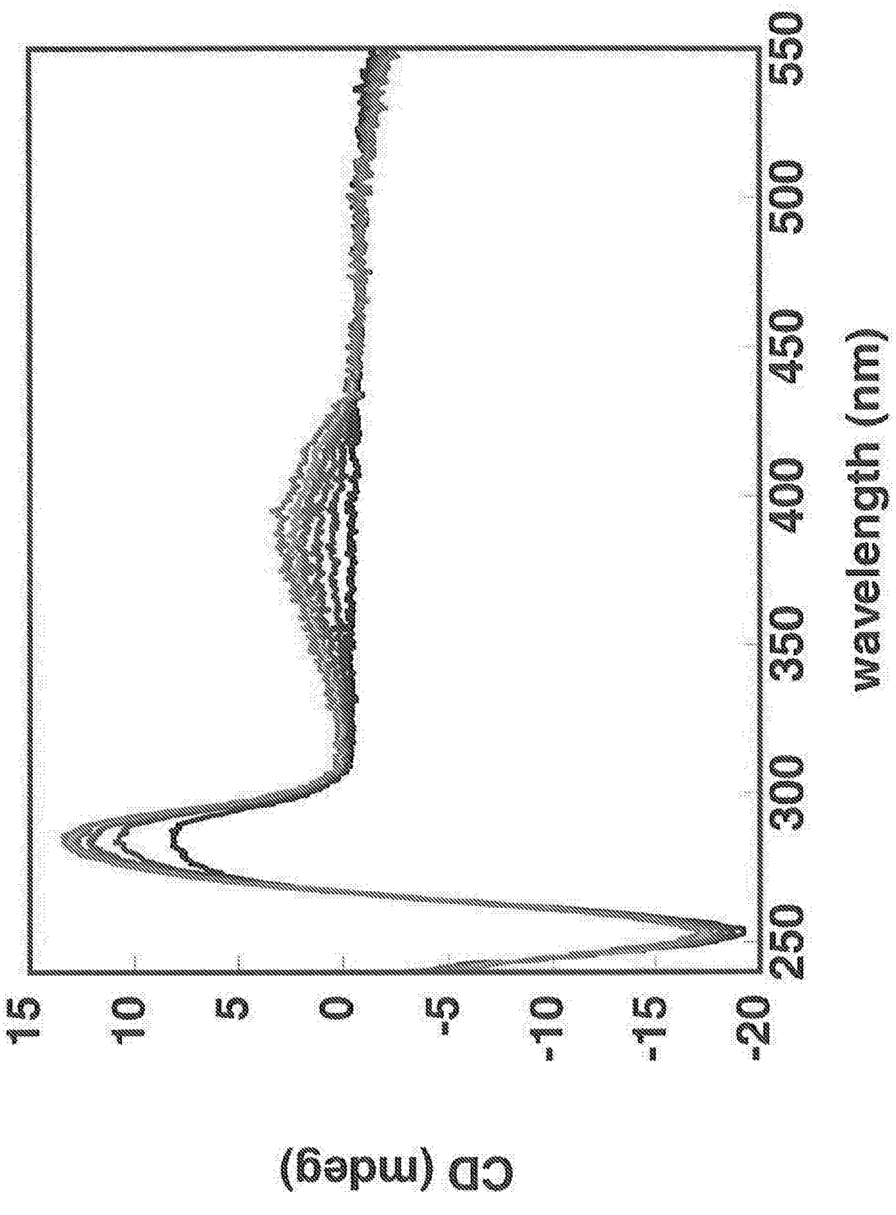
CD (mdeg)
15
10
5
0
-5
-10
-15
-20
250
300
350
400
450
500
550
wavelength (nm)

*Fig 9*

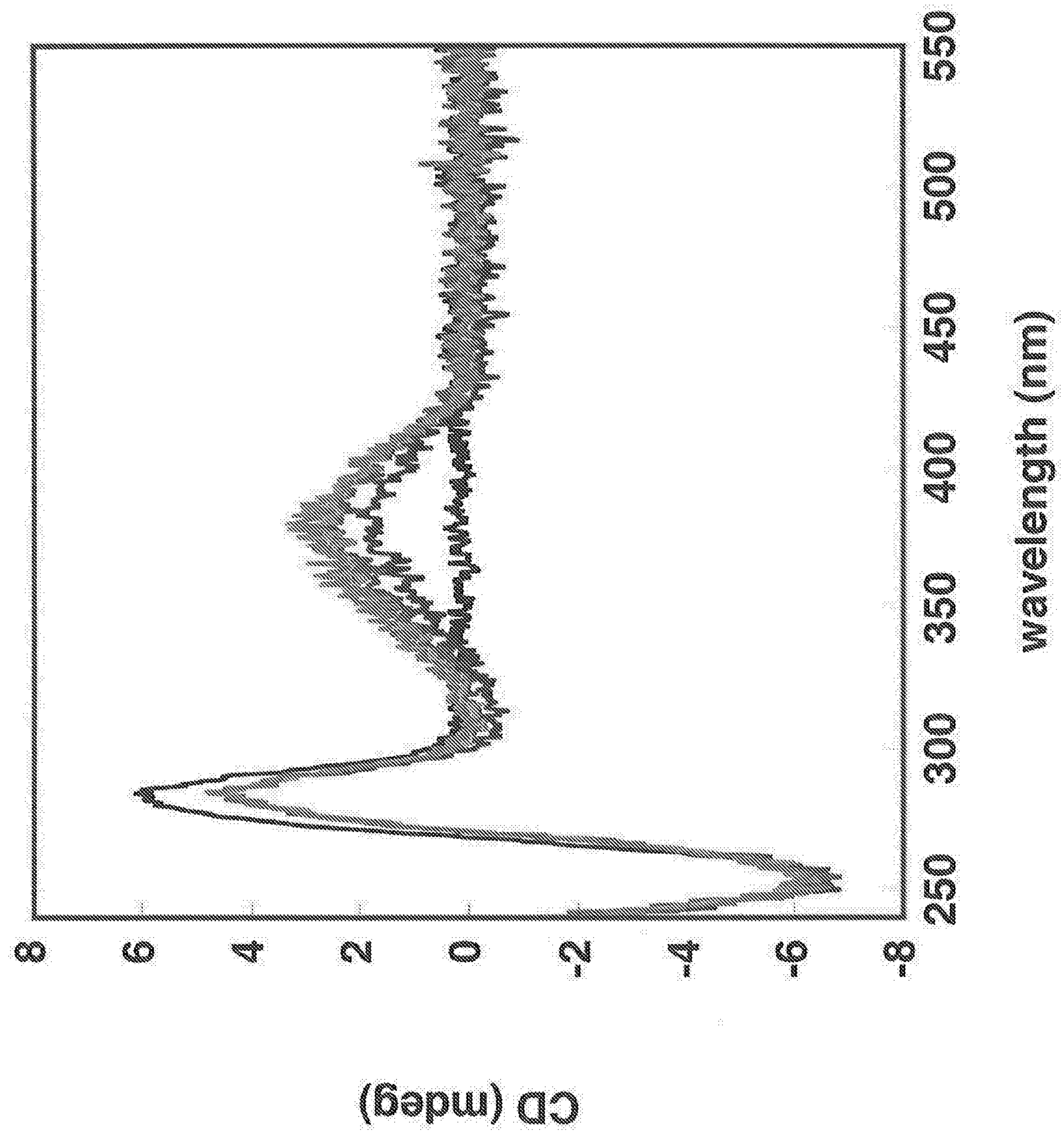


Fig 10

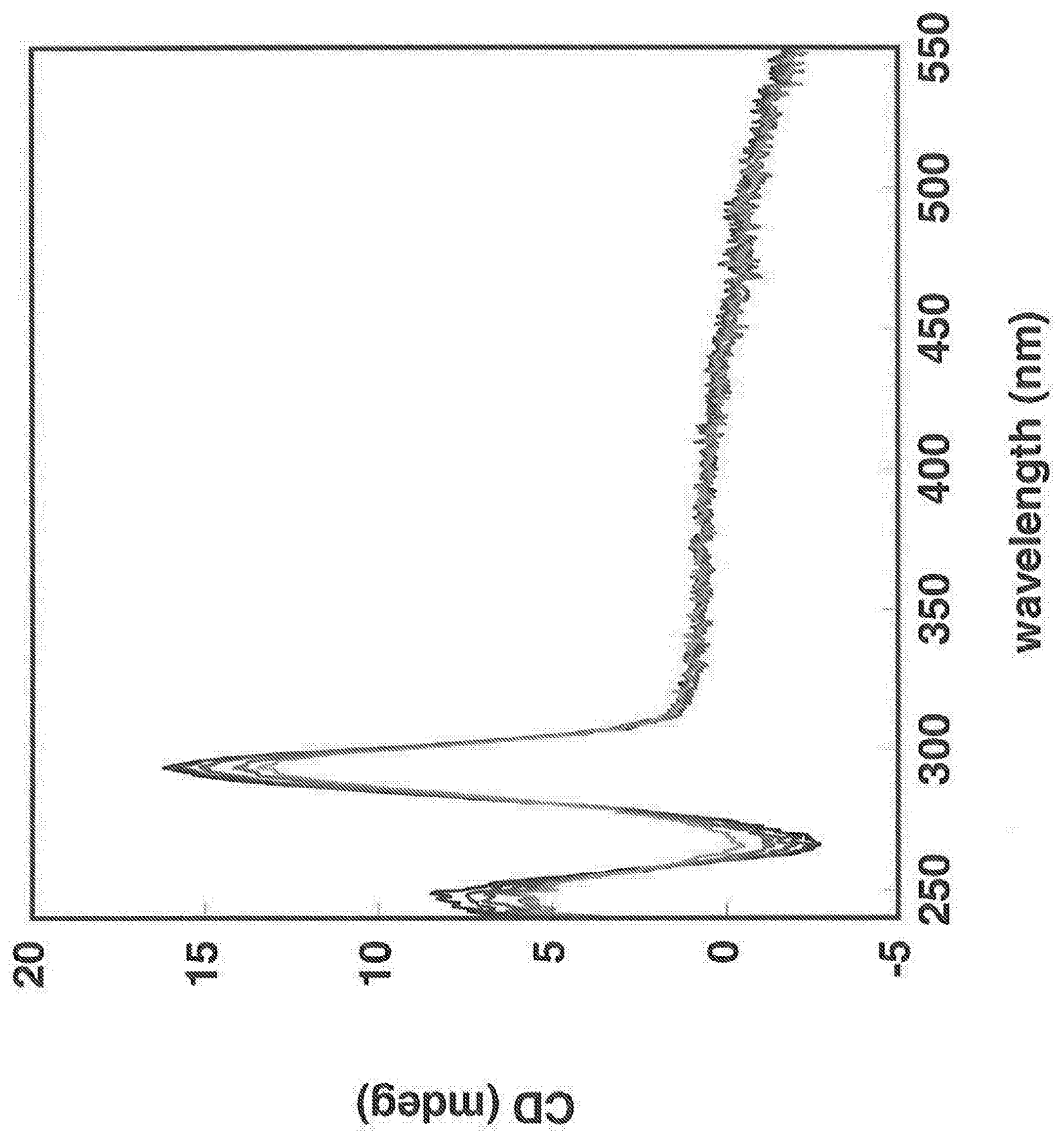


Fig 11

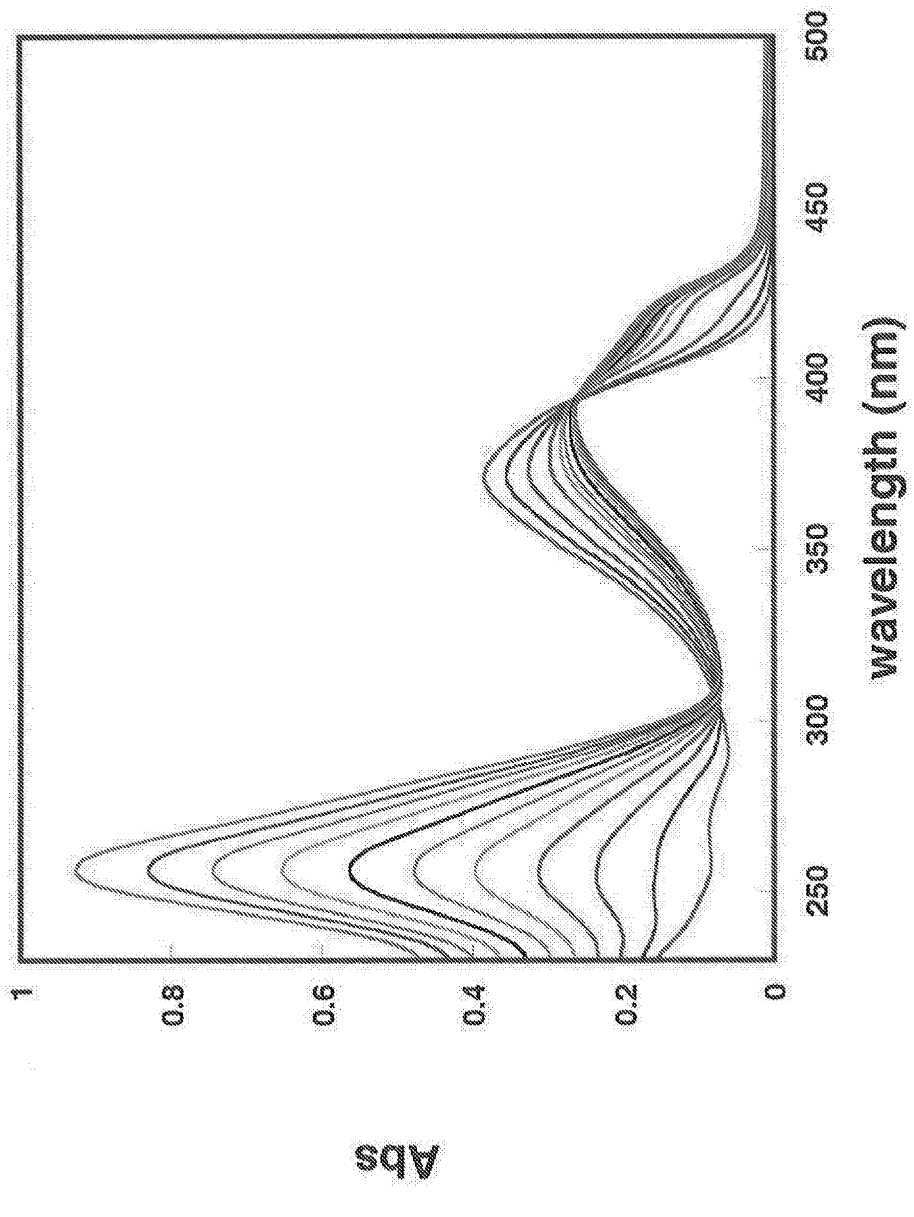


*Fig 12*

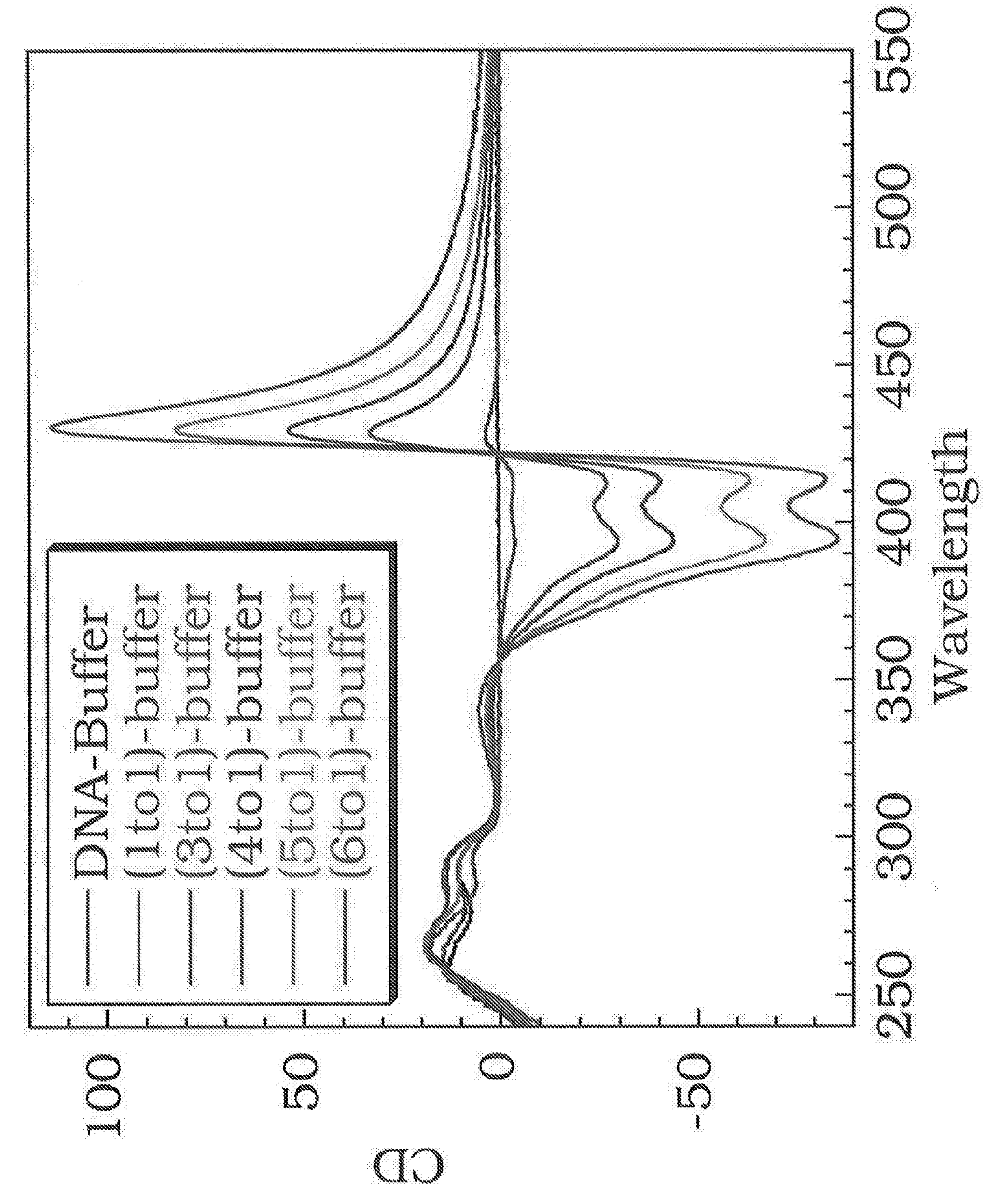
DNA-Buffer
(1to1)-buffer
(3to1)-buffer
(4to1)-buffer
(5to1)-buffer
(6to1)-buffer
CD
100
50
0
-50
Wavelength
250
300
350
400
450
500
550

Fig 13

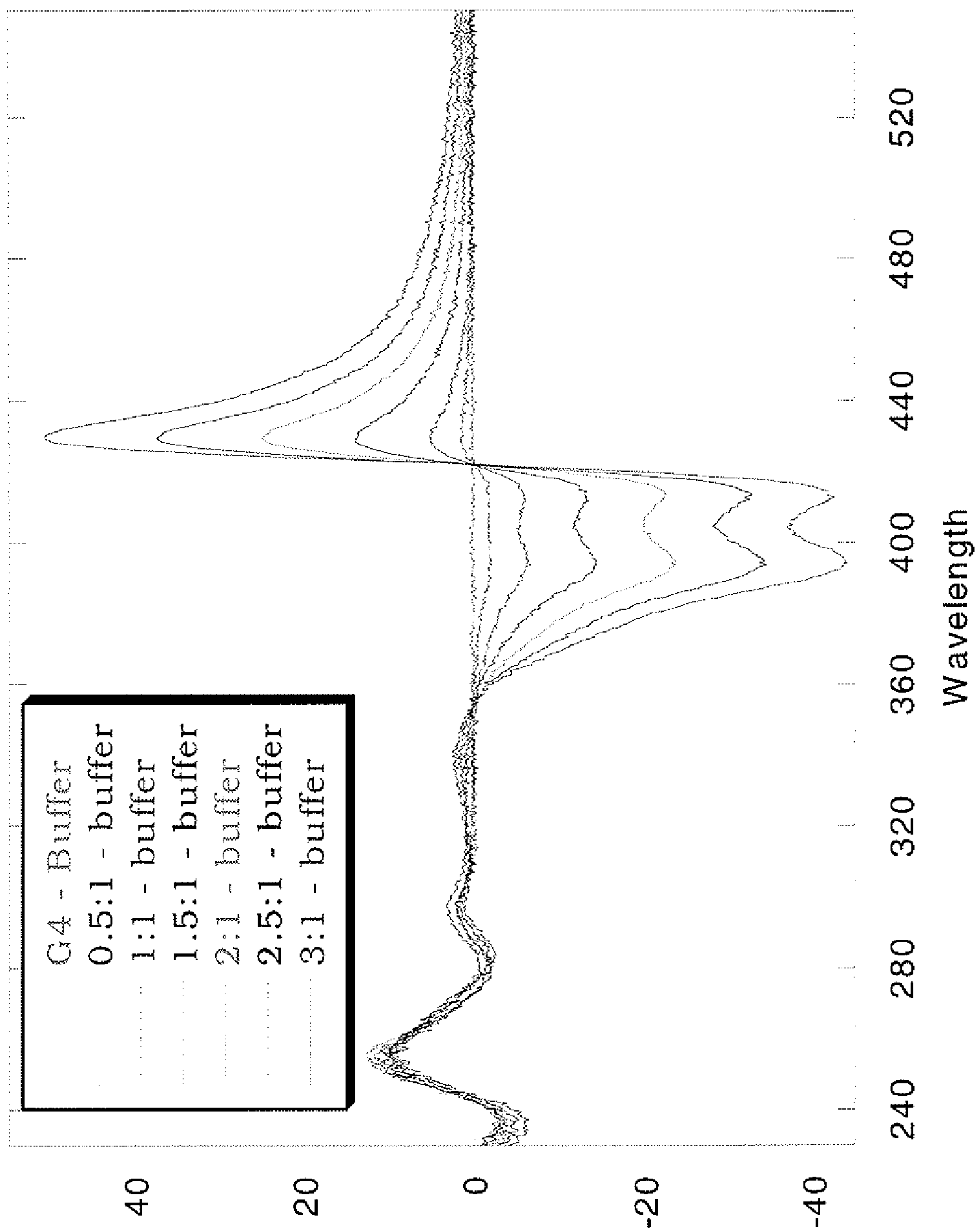
G4 - Buffer
0.5:1 - buffer
1:1 - buffer
1.5:1 - buffer
2:1 - buffer
2.5:1 - buffer
3:1 - buffer
40
20
0
-20
-40
240
280
320
360
400
440
480
520
Wavelength

*Fig 14*

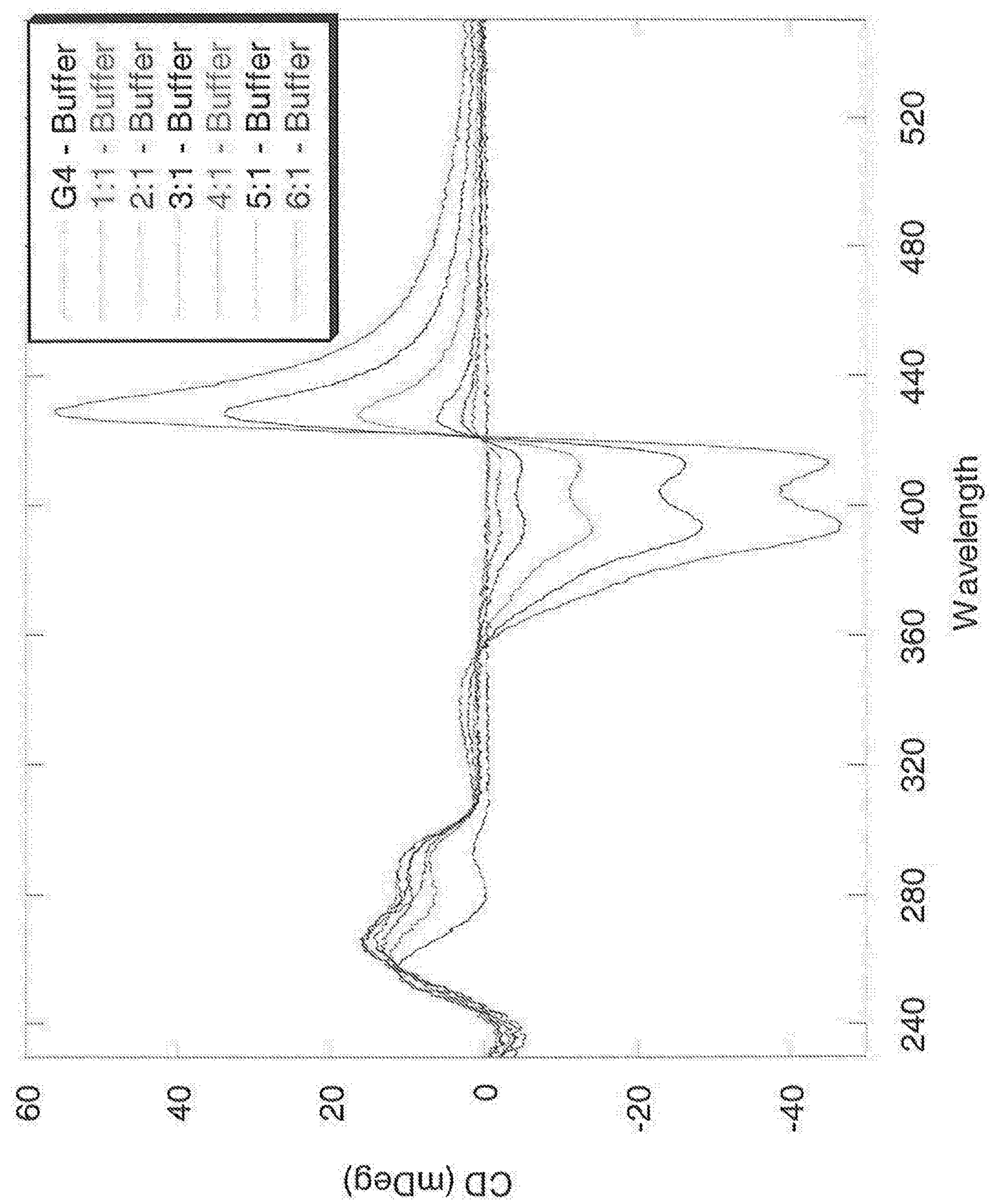
G4 - Buffer
1:1 - Buffer
2:1 - Buffer
3:1 - Buffer
4:1 - Buffer
5:1 - Buffer
6:1 - Buffer
CD (mDeg)
60
40
20
0
-20
-40
240
280
320
360
400
440
480
520
Wavelength

**Fig 15**

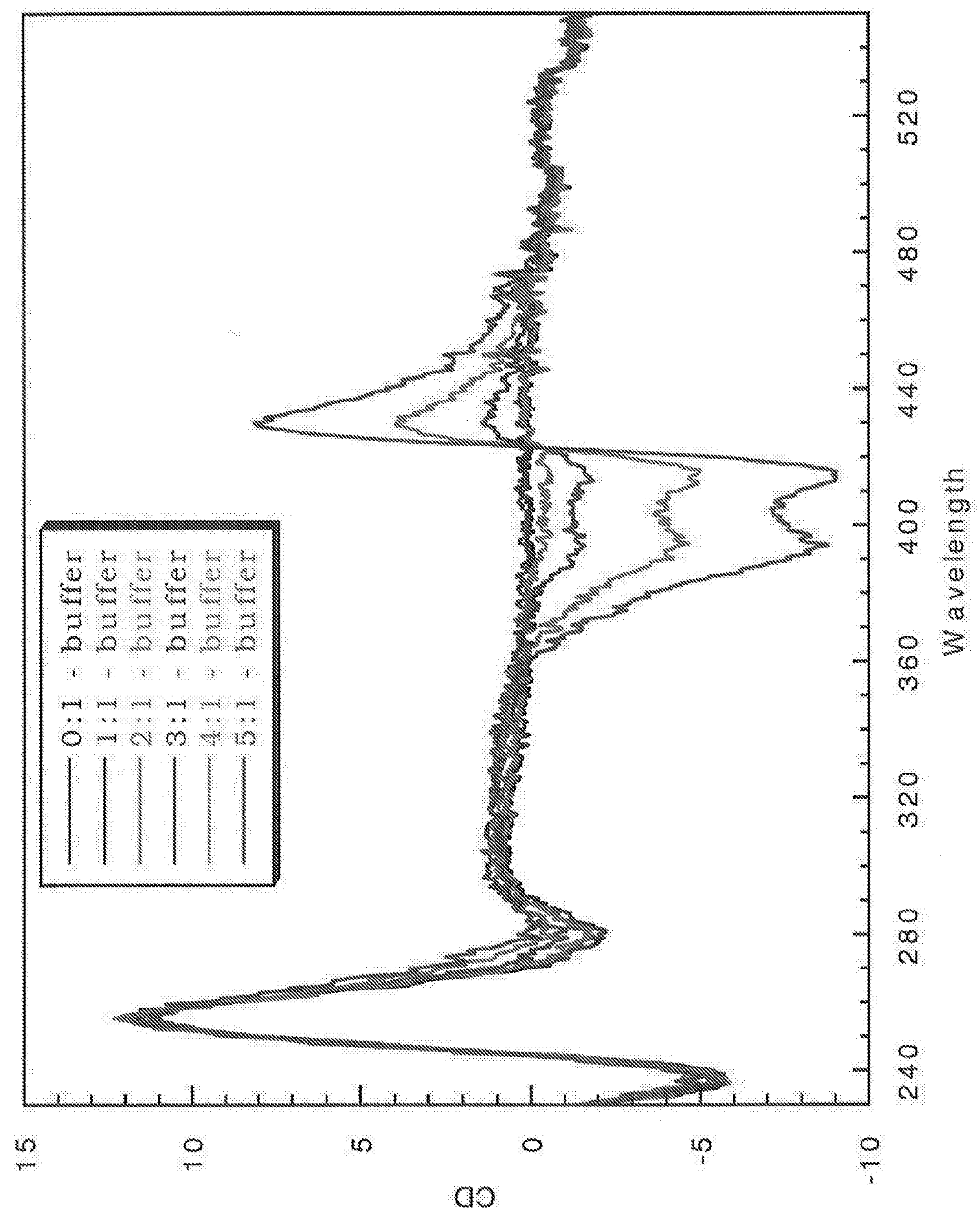
0:1 - buffer
1:1 - buffer
2:1 - buffer
3:1 - buffer
4:1 - buffer
5:1 - buffer
CD
15
10
5
0
-5
-10
Wavelength
240
280
320
360
400
440
480
520

**Fig 16**

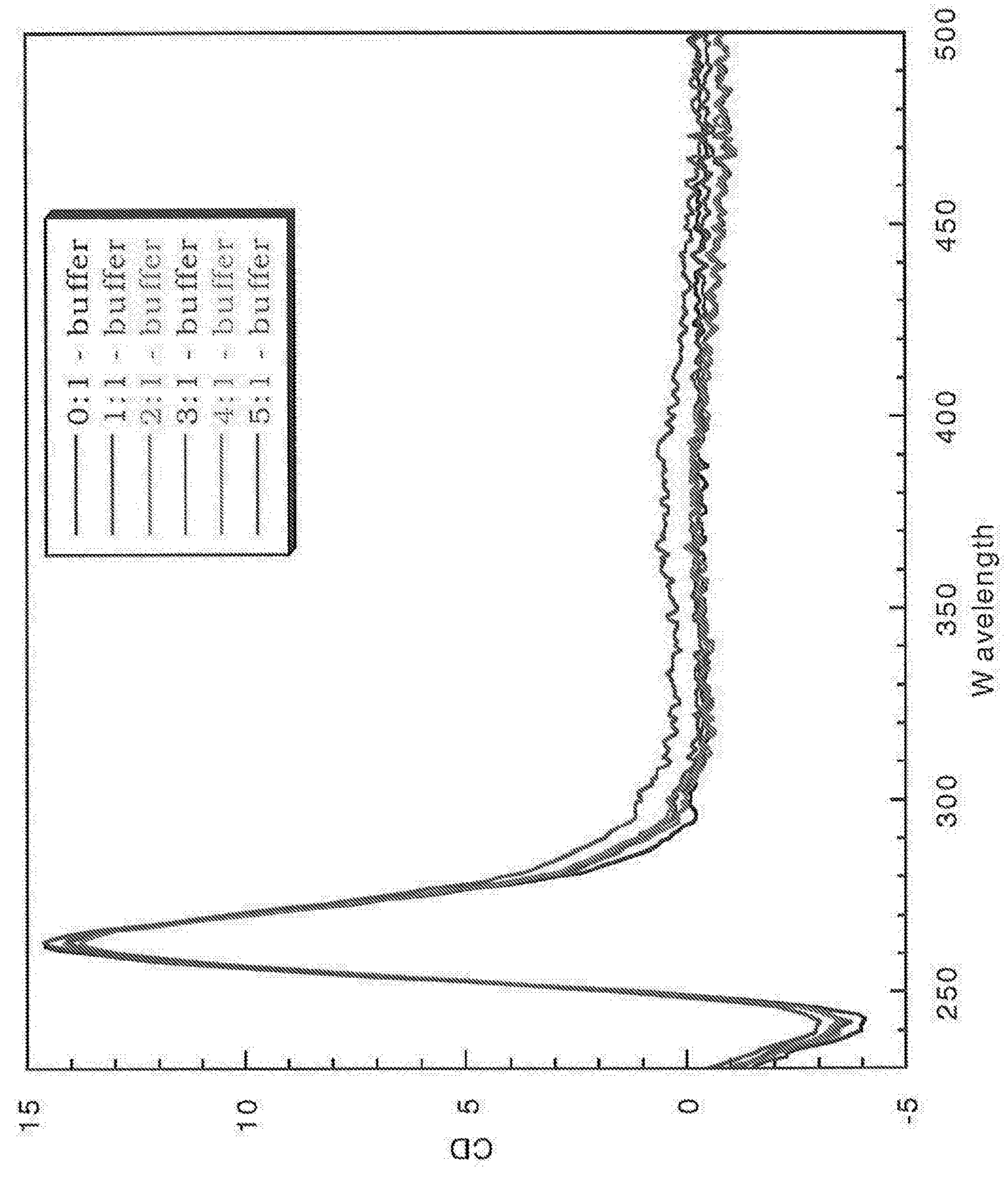

CD
15
10
5
0
-5
250
300
350
400
450
500
Wavelength
0:1 - buffer
1:1 - buffer
2:1 - buffer
3:1 - buffer
4:1 - buffer
5:1 - buffer


*Fig 17*

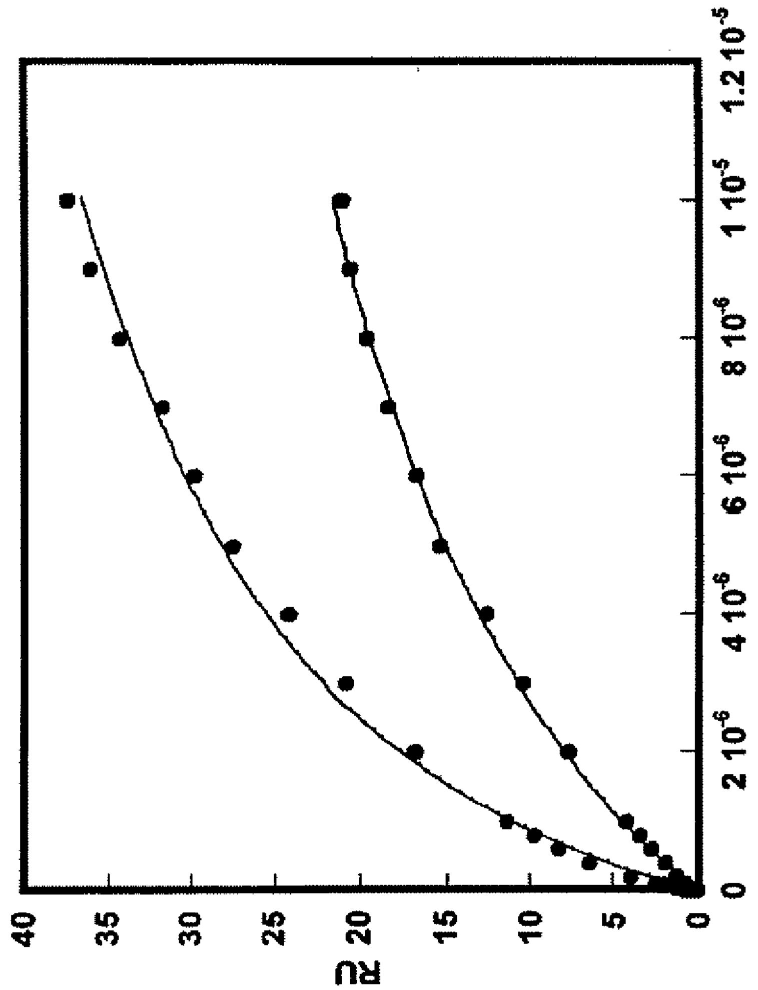


*Fig 18*

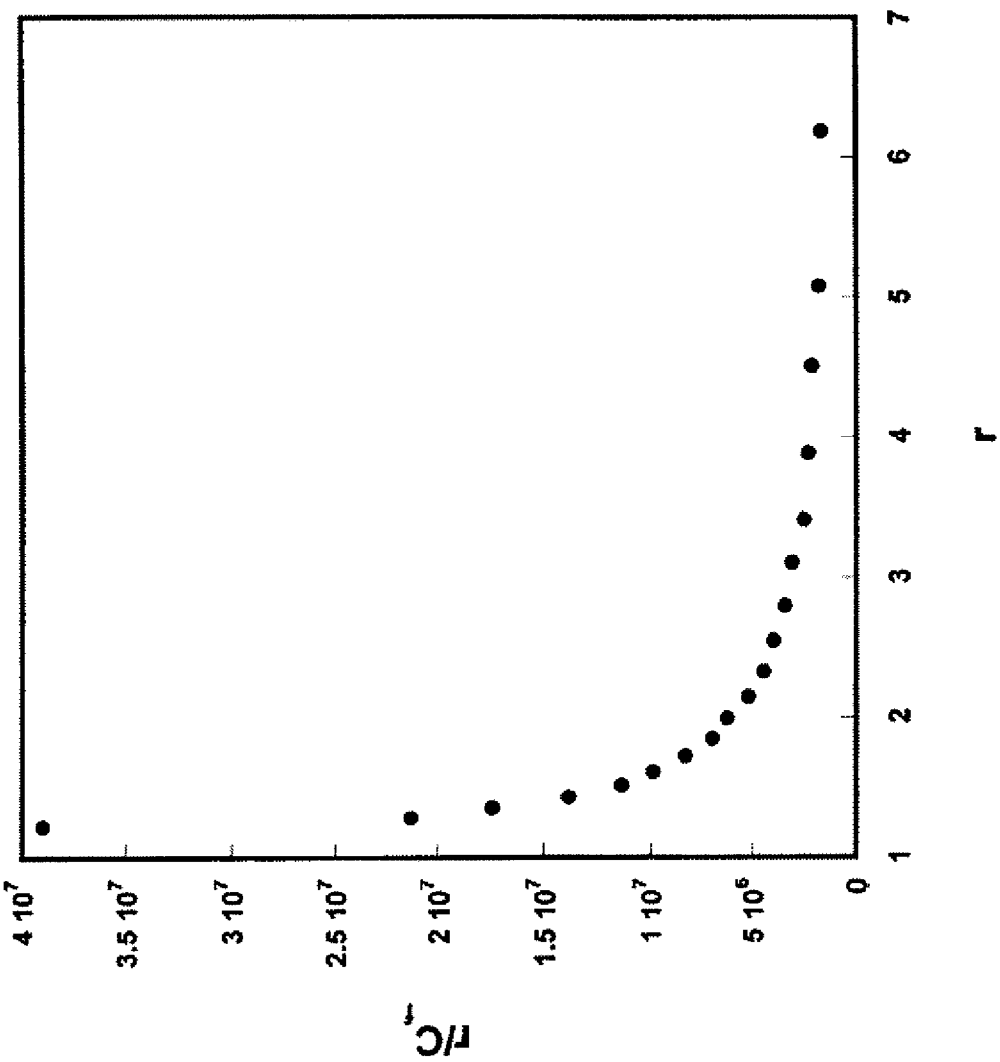


*Fig 19*

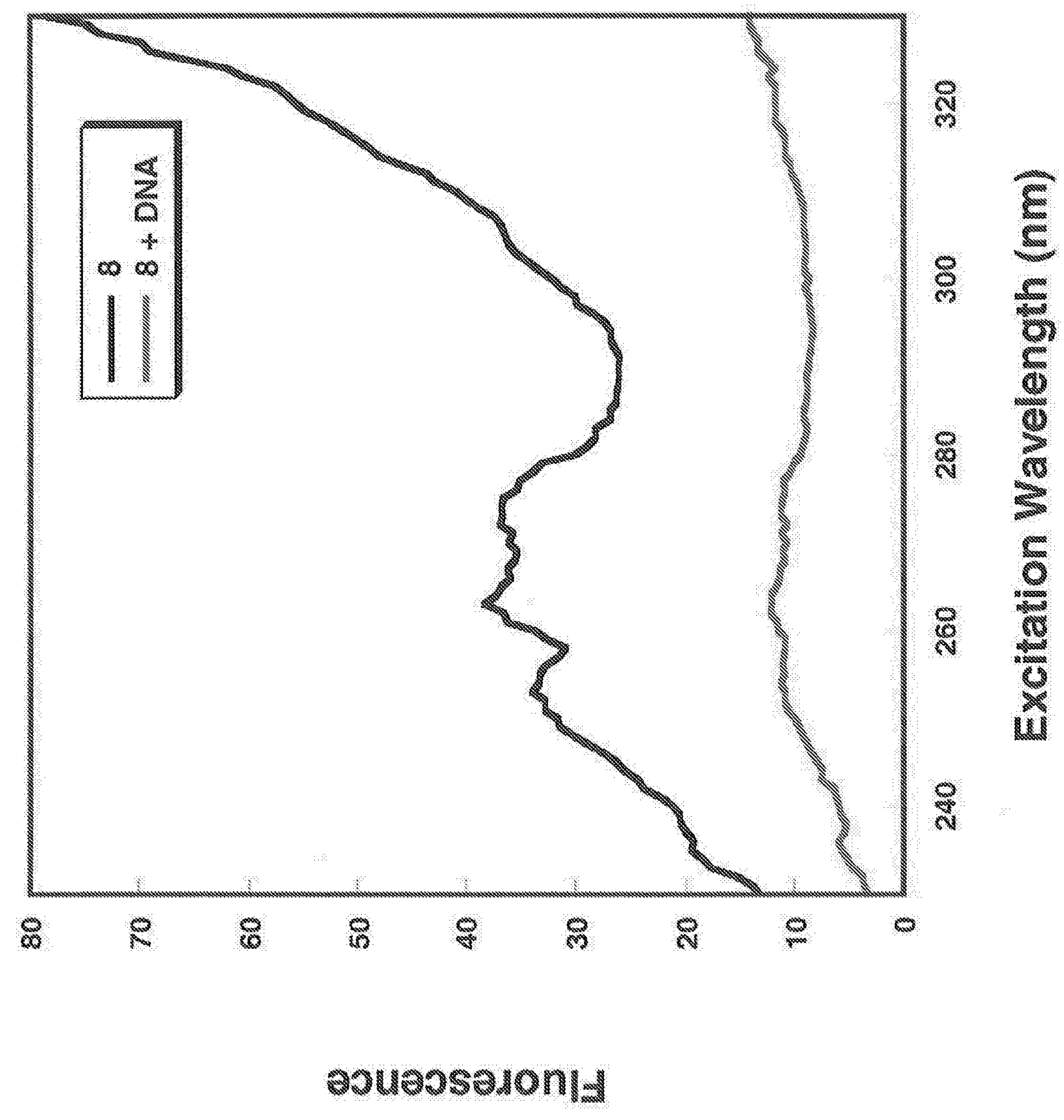

Fluorescence
80
70
60
50
40
30
20
10
0
240
260
280
300
320
Excitation Wavelength (nm)
8
8 + DNA


*Fig 20*

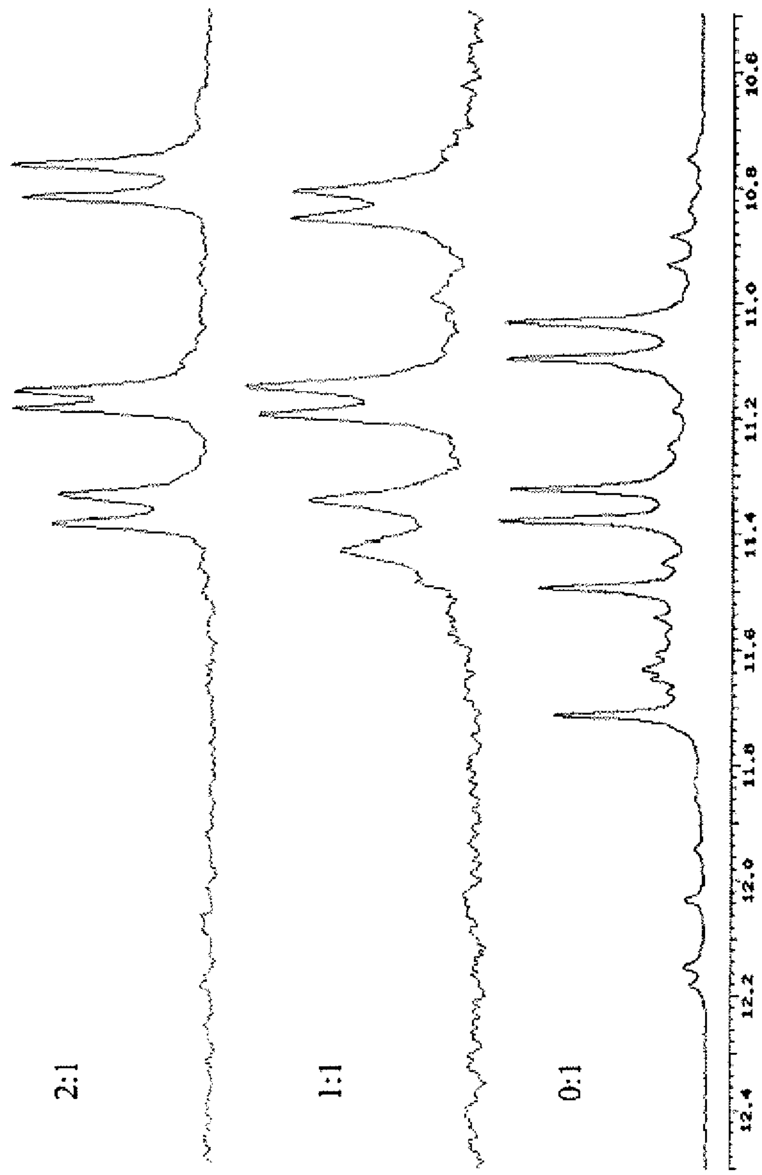


*Fig 21*

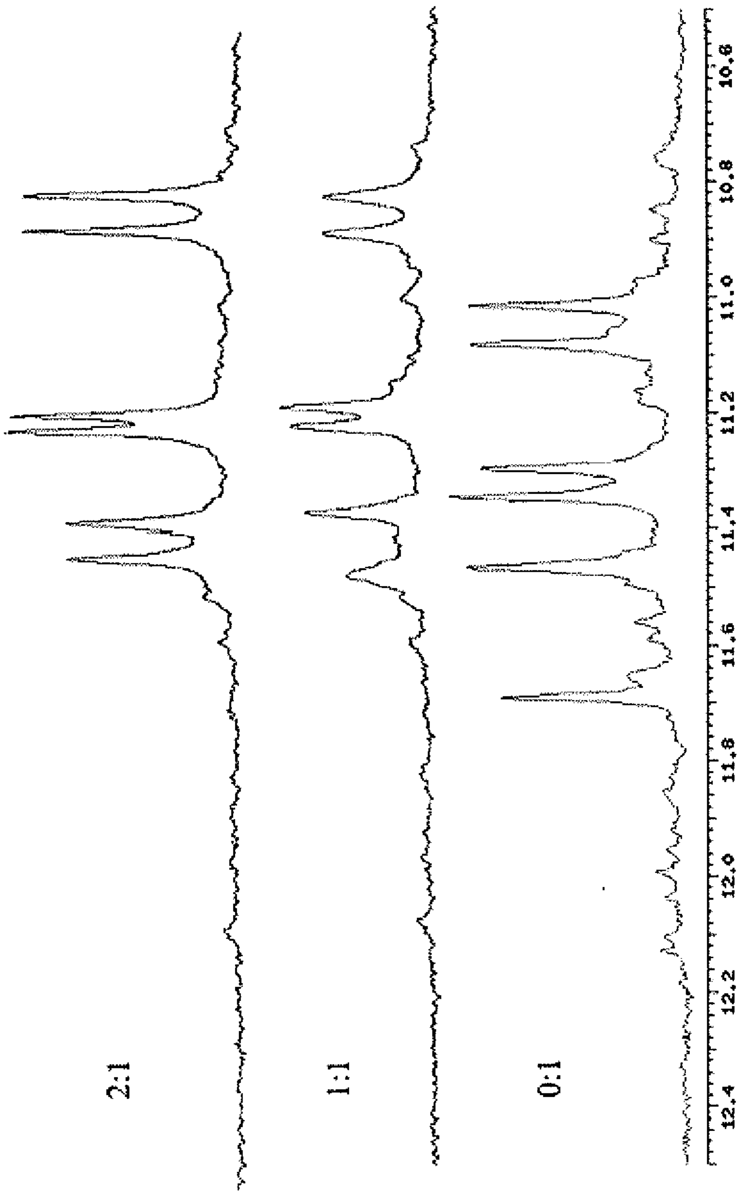


*Fig 22*

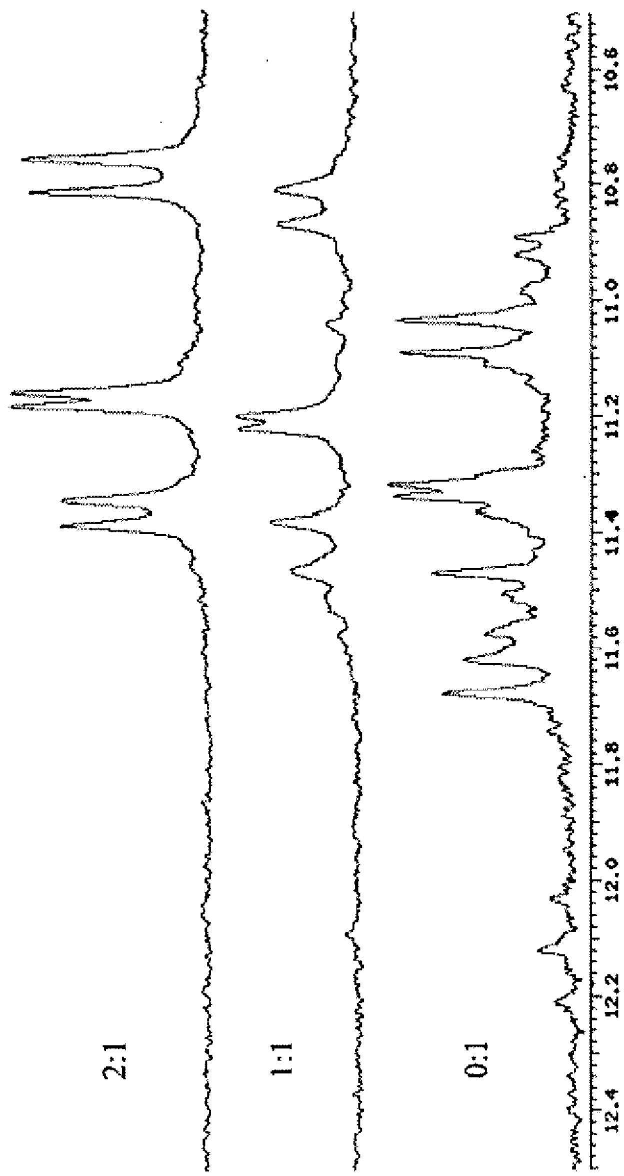


Fig 23

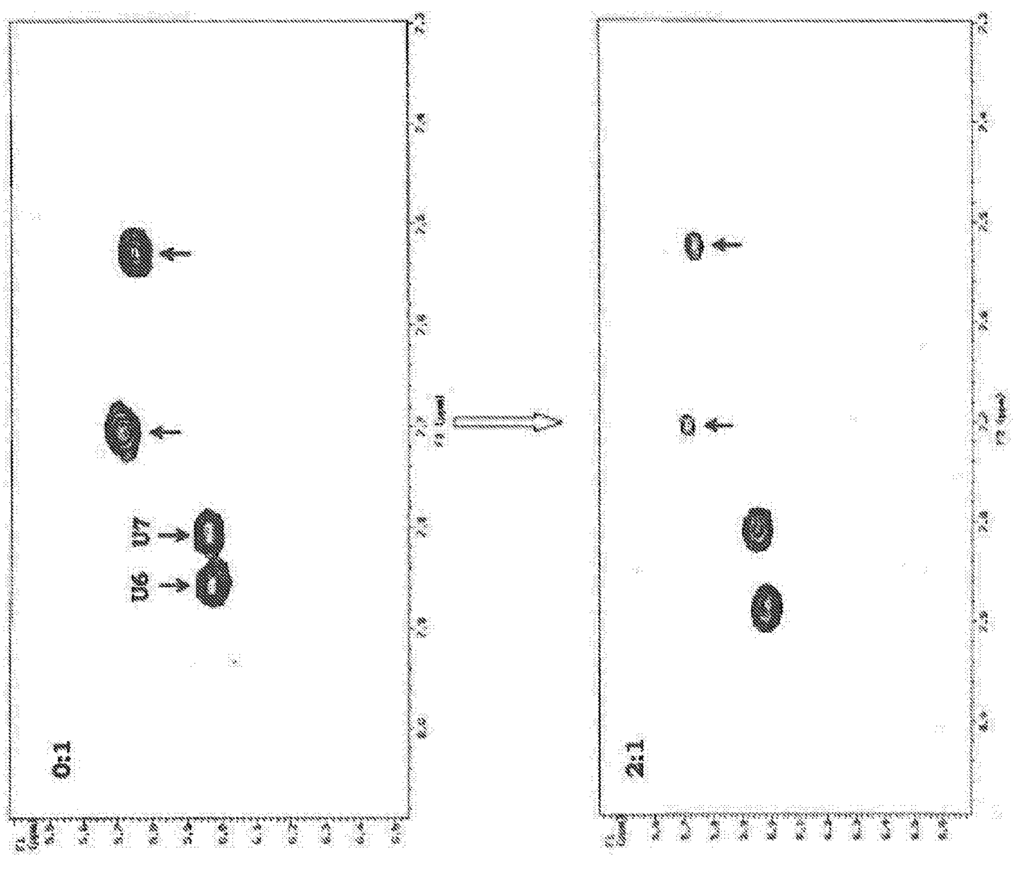


Fig 24

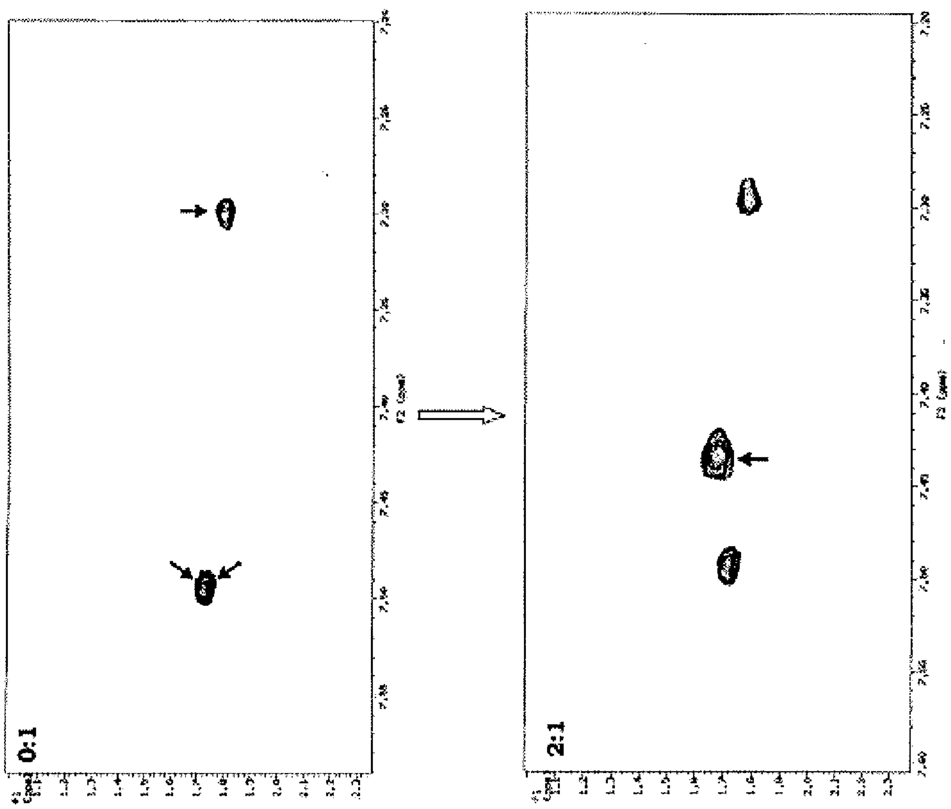


Fig 25

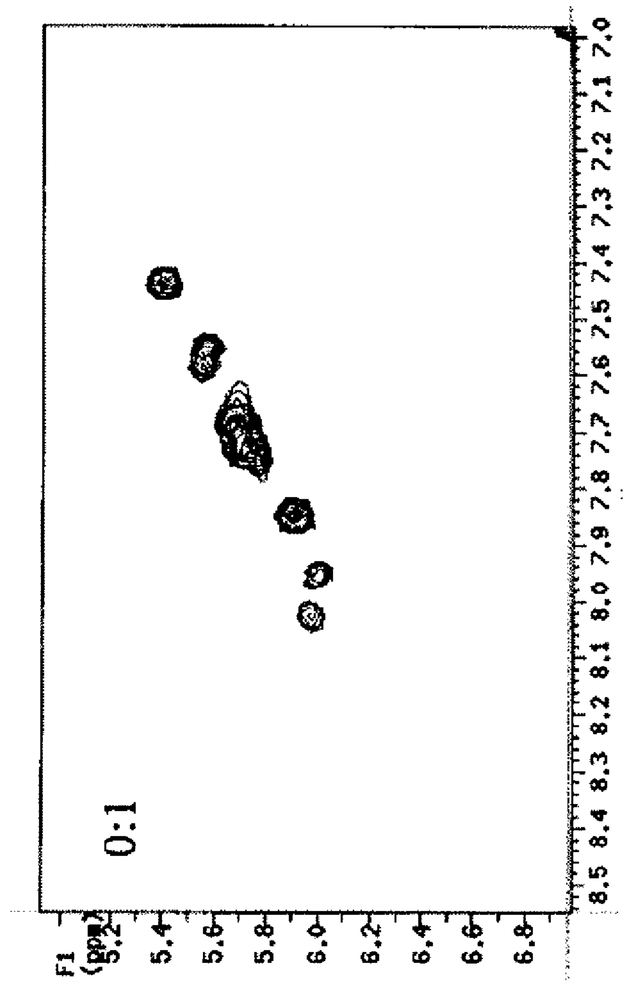
0:1
F1 (ppm)
5.2
5.4
5.6
5.8
6.0
6.2
6.4
6.6
6.8
7.0
7.1
7.2
7.3
7.4
7.5
7.6
7.7
7.8
7.9
8.0
8.1
8.2
8.3
8.4
8.5

Fig 26A

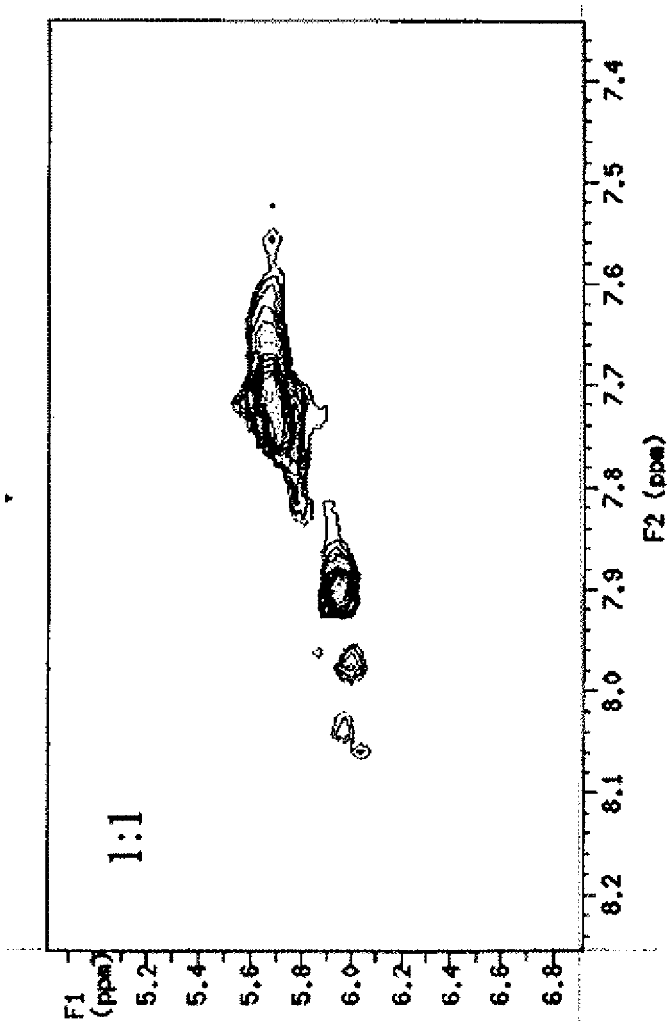


*Fig 26B*

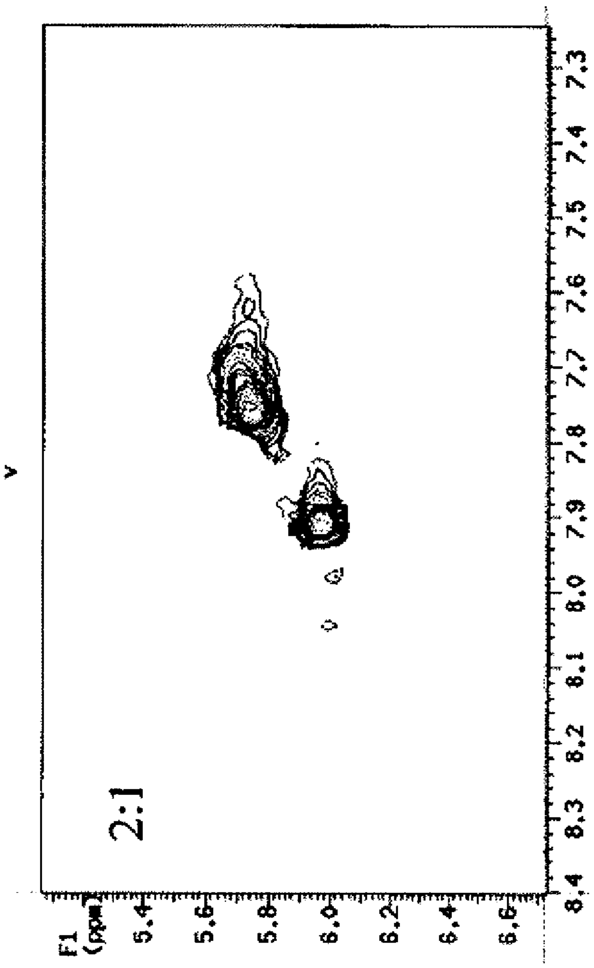


*Fig 26C*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6689887 B, Kerwin, S. M.
- US 5628984 A
- US 20050148646 A, Boykin, D. W.
- WO 2005086808 A, Boykin, D. W.


### Non-patent literature cited in the description


- **CECH, T.R.** *Nature,* 1988, vol. 332, 777-778
- **SEN, D. ; GILBERT, W.** *Nature,* 1988, vol. 334, 364-366
- **CASTASI, P. et al.** *J. Mol. Biol.,* 1996, vol. 264, 534-545
- **SIDDIQUI-JAIN, A. et al.** *Proc. Natl. Acad. Sci. USA,* 2002, vol. 99, 11593-11598
- **SIMONSSON, T. et al.** *Nuc. Acids Res.,* 1998, vol. 26, 1167-1172
- **WANG, Y. ; PATEL, D. J.** *Structure,* 1994, vol. 2, 1141-1156
- **WANG, Y. ; PATEL, D. J.** *J. Mol. Biol.,* 1995, vol. 251, 76-94
- **SMITH, F. W. et al.** *Structure,* 1995, vol. 3, 997-1008
- **PARKINSON, G. N. et al.** *Nature,* 2002, vol. 417, 876-880
- **WANG, Y. ; PATEL, D.** *Structure,* 1993, vol. 1, 263-282
- **GIRALDO, T. et al.** *Proc. Natl. Acad. Sci. USA,* 1994, vol. 91, 7658-7662
- **FANG, G. ; CECH, T. R.** *Biochemistry,* 1993, vol. 32, 11646-11657
- **FANG, G. ; CECH, T, R.** *Cell,* 1993, vol. 74, 875-885
- **GRANOTIER, C. et al.** *Nuc. Acids. Res.,* 2005, vol. 33, 4182-4190
- **HARLEY, C. B. et al.** *Nature,* 1990, vol. 345, 458-460
- **KIM, N. W. et al.** *Science,* 1994, vol. 266, 2011-2015
- **ZHANG, X. et al.** *Gene Dev.,* 1999, vol. 13, 2388-2399
- **HAHN, W. C. et al.** *Nat. Med.,* 1999, vol. 5, 1164-1170
- **STRAHL, C. ; BLACKBURN, E. H.** *Mol. Cell. Biol.,* 1996, vol. 16, 53-56
- **MELANA, S. M. et al.** *Clin. Cancer Res.,* 1997, vol. 4, 693-696
- **MURAKAMI, J. et al.** *Eur. J. Cancer,* 1998, vol. 35, 1027-1034
- **GOMEZ, D. E. et al.** *Biochem. Biophys. Res. Commun.,* 1998, vol. 246, 107-110
- **SCHINDLER, A. et al.** *Int J. Oncol.,* 2001, vol. 19, 25-30
- **FU, W. M. et al.** *J. Mol. Neurosci.,* 2000, vol. 14, 3-15
- **YOKOYAMA, Y. et al.** *Biochem. Biophys. Res. Commun.,* 2000, vol. 273, 316-321
- **LUDWIG, A. et al.** *Cancer Res.,* 2001, vol. 61, 3053-3061
- **ZAHLER, A. M. et al.** *Nature,* 1991, vol. 350, 718-720
- **BOTTIUS, E. N. et al.** *Mol. Cell. Biol.,* 1998, vol. 18, 919-925
- **CANO, M. I. N. et al.** *Proc. Natl. Acad. Sci. USA,* 1999, vol. 96, 3616-3121
- **HAN, F. X. et al.** *J. Am. Chem. Soc,* 1999, vol. 121, 3561-3570
- **TEULADE-FICHOU, M-P. et al.** *J. Am. Chem. Soc.,* 2003, vol. 125, 4732-4740
- **FEDOROFF O. Y. et al.** *Biochemistry,* 1998, vol. 37, 12367-12374
- **READ, M. et al.** *Proc. Natl. Acad. Sci. USA,* 2001, vol. 98, 4844-4849
- **CLARK, G. R. et al.** *J. Am. Chem. Soc.,* 2003, vol. 125, 4066-4067
- **WANG, Y. ; PATEL, D. J.** *Structure,* 1993, vol. 1, 263-282
- **PARKINSON, G. N. et al.** *Nature,* 2002, 876-880
- **SIMONSSON, T.** *Biol. Chem.,* 2001, vol. 382, 621-628
- **REZLER, E. M. et al.** *J. Am. Chem. Soc.,* 2005, vol. 127, 9439-9447
- **HAN, H. et al.** *Biochemistry,* 1999, vol. 38, 6981-6986
- **GUO, Q. et al.** *Biochemistry,* 1992, vol. 31, 2451-2455
- **HAN, H. et al.** *J. Am. Chem. Soc.,* 2001, vol. 123, 8902-8913
- **MERGNY, J.-L. et al.** *Anti-Cancer Drug Design,* 1999, vol. 14, 327-339
- **RANDAZZO, A. et al.** *Nucleosides, Nucleotides, and Nucleic Acids,* 2002, vol. 21, 535-545

- **CHEN, Q. et al.** *Proc. Natl. Acad. Sci. USA,* 1996, vol. 93, 2635-2639
- **CHENG, J.-Y. et al.** *J. Phys. Chem.,* 1998, vol. 102, 5542-5546
- **CHIANG, C.-C. et al.** *Proceedings of the National Science Council ROC(A),* 1999, vol. 23, 679-694
- **DAVID, W. M. et al.** *Anal. Chem.,* 2002, vol. 74, 2029-2033
- **KERWIN, S. M. et al.** *Bioorg. Med. Chem. Lett.,* 2001, vol. 11, 2411-2414
- **COCCO, M. J. et al.** *Nuc. Acids Res.,* 2003, vol. 31, 2944-2951
- **DAS, B. P. ; BOYKIN, D. W.** *J. Med. Chem.,* 1977, vol. 20, 531-536
- **CZARNY, A. et al.** *J. Heterocycl. Chem.,* 1996, vol. 33, 1393-1397
- **HOPKINS, K. T. et al.** *J. Med. Chem.,* 1998, vol. 41, 3872-3878
- **LANSIAUX, A. et al.** *J. Med. Chem.,* 2002, vol. 45, 1994-2002
- **BILIK, P. et al.** *CHEMBIOCHEM,* 2001, vol. 2, 559-569
- **DAS, B. P. ; D. W. BOYKIN.** *J. Med. Chem.,* 1977, vol. 20, 531-536
- **MCFARLAND, J. W. ; H. L. HOWES, JR.** *J. Med. Chem.,* 1972, vol. 15, 365-368
- **LACY, E.R. et al.** *J. Amer. Chem. Soc.,* 2002, vol. 124, 2153-2163
- **MAZUR, S. et al.** *J. Mol. Biol.,* 2000, vol. 300, 321-337
- **NGUYEN, B. et al.** *Biopolymers,* 2002, vol. 63, 281-297
- **WANG, L. et al.** *Proc. Natl. Acad. Sci. USA,* 2000, vol. 97, 12-16
- **WANG, L. et al.** *Biochemistry,* 2001, vol. 40, 2511-2521
- **ALLENMARK, S.** *Chirality,* 2003, vol. 15, 409-422
- **BALAGURUMOORTHY, P. et al.** *J. Biomol. Struc. Dyn.,* 1992, vol. 8, 15
- **BALAGURUMOORTHY, P. et al.** *Nuc. Acids. Res.,* 1992, vol. 20, 4061-4067
- **HE. Y. et al.** *Nuc. Acids Res.,* 2004, vol. 32, 5359-5367
- **LI, J. et al.** *Nuc. Acids Res.,* 2005, vol. 33, 4649-4659
- **QI, J. ; SHAFER, R.** *Nuc. Acids Res.,* 2005, vol. 33, 3185-3192
- **SEN. D. ; GILBERT, W.** *Methods Enzymol,* 1992, vol. 211, 191-199
- **PHAN, A. T. et al.** *Nature Chem. Biol.,* 2005, vol. 1, 167-234
- **JELLEY, E. E.** *Nature,* 1936, vol. 138, 1009
- **SCHIEBE, G.** *Angew. Chem.,* 1937, vol. 50, 212
- **PHAN, A. T. ; PATEL, D.** *J. Am. Chem. Soc.,* 2003, vol. 125, 15021-15027
- **ISMAIL, M. A. et al.** *J. Med. Chem.,* 2003, vol. 46, 4761-4769
- **STEPHENS, C. E. et al.** *Bioorg. Med. Chem. Lett.,* 2003, vol. 13, 2065-2069

SEQUENCE LISTING

<110> Wilson, W. David
Boykin, David
White, Elizabeth
Ismail, Mohamed
Kumar, Arvind
Nanjunda, Rupesh
Tidwell, Richard

<120> DICATIONIC COMPOUNDS WHICH SELECTIVELY RECOGNIZE G-QUADRUPL
EX DNA

<130> 421-60-32

<160> 13

<170> PatentIn version 3.0

<210> 1
<211> 22
<212> DNA
<213> Homo sapiens

<400> 1
agggttaggg ttagggttag gg
22

<210> 2
<211> 19
<212> DNA
<213> Homo sapiens

<400> 2
agggtgggga gggtgggga
19

<210> 3
<211> 28
<212> DNA
<213> Oxytricha

<400> 3
ggggttttgg ggttttgggg ttttgggg
28

<210> 4
<211> 24
<212> DNA

<213> Tetrahymena sp.

<400> 4
ttggggttgg ggttggggtt gggg
24

<210> 5
<211> 14
<212> DNA
<213> artificial sequence

<400> 5
gcgcgcgcgc gcgc
14

<210> 6
<211> 10
<212> DNA
<213> artificial sequence

<400> 6
gcgaattcgc
10

<210> 7
<211> 15
<212> DNA
<213> artificial sequence

<400> 7
ggttggtgtg gttgg
15

<210> 8
<211> 20
<212> DNA
<213> artificial sequence

<400> 8
cgaattcgtc tccgaattcg
20

<210> 9
<211> 12
<212> DNA
<213> artificial sequence

```
<400>  9
tagggutagg gt
   12


<210>  10
<211>  12
<212>  DNA
<213>  artificial sequence

<400>  10
tagggwuagg gt
   12


<210>  11
<211>  12
<212>  DNA
<213>  artificial sequence

<400>  11
tagggutagg gu
   12


<210>  12
<211>  7
<212>  DNA
<213>  artificial sequence

<400>  12
ttagggt
    7


<210>  13
<211>  6
<212>  DNA
<213>  artificial sequence

<400>  13
tggggt
    6
```